# EUROPEAN PATENT APPLICATION

(11) **EP 1 719 761 A1**
(43) Date of publication of application: **08.11.2006**
(21) Application number: 05710695.7
(22) Date of filing: 18.02.2005
(51) Int. Cl.: C07D 209/34, C07D 235/26, C07D 263/58, C07D 277/68, C07D 401/02, C07D 403/02, C07D 405/02, C07D 413/02, C07D 417/02, C07D 471/06, C07D 487/06, C07D 498/06, A61K 31/40, A61K 31/4184, A61K 31/423, A61K 31/428, A61K 31/4745, A61K 31/536, A61K 31/551, A61P 25/22, A61P 25/24

(54) **NOVEL HETEROCYCLIC COMPOUND**

(30) Priority: 23.02.2004 JP 2004045979
(71) Applicant: Dainippon Sumitomo Pharma Co., Ltd., Osaka 541-8524 (JP)
(72) Inventor: KODO, Toru, c/o Dainippon Sumitomo Pharma. Co.,Ltd, Osaka 564-0053 (JP); FUKAYA, Takayuki, Dainippon Sumitomo Ph. Co, Ltd, Osaka 564-0053 (JP); KOYAMA, Koji, c/o Dainippon Sumitomo Ph. Co., Ltd., Osaka 564-0053 (JP); MASUMOTO, Shuji, c/oDainippon Sumitomo Ph. Co.,Ltd, Osaka 564-0053 (JP); FUJIBAYASHI, Nao, Dainippon Sumitomo Pha. Co, Ltd, Osaka-shi Osaka 541-8524 (JP)
(74) Representative: Coleiro, Raymond
(86) International application number: PCT/JP2005/003095
(87) International publication number: WO 2005/080334

(57) **Abstract**

A drug having a high affinity for benzodiazepine ω₃ receptors and showing curative and preventive effects for anxiety and depression, which comprises as the active ingredient, for example, a compound of the formula (1): 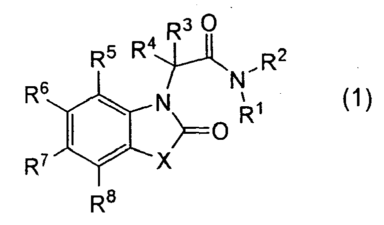 wherein R¹ and R² are independently a hydrogen atom, an optionally substituted alkyl group, an optionally substituted aryl group, etc.,
R³ and R⁴ are independently a hydrogen atom, an optionally substituted alkyl group, etc.,
R⁵, R⁶, R⁷ and R⁸ are independently a hydrogen atom, an optionally substituted alkyl group, an optionally substituted aryl group, etc.,
X is an oxygen atom, a sulfur atom, NR¹⁰, etc. (in which R¹⁰ is a hydrogen atom, an optionally substituted alkyl group, etc.)

## Description

### TECHNICAL FIELD

The present invention relates to a medicament comprising a novel heterocyclic compound selectively acting on benzodiazepine ω₃₋receptor.

### BACKGROUND ART

The existing anti-anxiety agents are largely classified into benzodiazepine drugs and serotonin drugs represented by serotonin 5-HT_{1A} receptor agonists and selective serotonin reuptake inhibitors (SSRI). Benzodiazepine drugs exhibit fast-acting and potent anti-anxiety effects, while they sometimes show side effects such as drug-dependent formation, excessive suppression and cognitive deficiency, and these side effects become problems. Further, it has been known that anxiety disorder is associated with a high incidence of depression, but usually benzodiazepine drugs hardly exhibit therapeutic effects on depression, and hence, the therapeutic effects of benzodiazepine drugs on such cases are limited. On the other hand, serotonin antianxiety agents have problems, for example, a long duration of drug exposure, inherent side effects such as sexual dysfunction with respect to SSRI, exaggerated anxiety in the initial stage of the medication thereby, and resistance to therapy. Under these circumstances, it has been desired to develop a novel antianxiety agent exhibiting therapeutic effects on depression with fewer side effects.

There are three subtypes of benzodiazepine receptors such as two types of central-type benzodiazepine receptors (benzodiazepine ω₁ and benzodiazepine ω₂ receptor) being present on the GABA_{A} receptor complexes, and a peripheral-type benzodiazepine receptor (benzodiazepine ω₃ receptor) being present on the mitochondrial outer membrane. It has been reported that the benzodiazepine ω₃ receptor agonists exhibit its anti-anxiety effects by indirectly controlling GABA_{A} receptor function via neurosteroidogenesis in the brain. Moreover, it has been reported that the benzodiazepine ω₃ receptor agonists show no side effect which is observed in benzodiazepine drugs, and further the benzodiazepine ω₃ receptor agonists have been known to exhibit antidepressant effects. Accordingly, benzodiazepine ω₃ receptor agonists can be expected to be a therapeutic agent having fewer side effects and wide action spectra on psychiatric disorders including anxiety disorder and depression.

On the other hand, in addition to the above-mentioned possibility as anti-anxiety agent and anti-depressive agent, it has been pointed out that benzodiazepine ω₃ receptor agonists may possibly be useful in sleep disorder, convulsion, epilepsy, cognitive dysfunction, Alzheimer's disease, Parkinson's disease, Huntington's chorea, schizophrenia, neuropathy, multiple sclerosis, cerebral infarction, cancer, or circulatory system diseases such as hypertension, coronary infarction, and immunological diseases such as rheumatic arthritis
2-Aryl-8-oxodihydropurine derivatives are disclosed in Patent Literature 1 and Patent Literature 2 as a therapeutic agent for central nervous diseases such as anxiety-related diseases, depression and epilepsy, and also disclosed in Patent Literature 3 as a therapeutic agent for dementia. Further, Patent Literature 4 discloses acetamide derivatives having a 2-phenyl-4-pyrimidinylamino moiety or 2-phenyl-4-pyrimidinyloxy moiety as a therapeutic agent for anxiety-related diseases and immunological diseases.

In addition, Patent Literature 5 discloses 4-amino-3-carboxy-quinolines and naphthyridines as an agent for prevention or treatment of cardiovascular diseases, allergy and infections, or as a therapeutic agent for anxiety-related diseases.

Furthermore, Patent Literature 6 discloses benzothiazoline derivatives as a neuropeptide Y receptors antagonist.
Patent Literature 1: WO 99/28320
Patent Literature 2: JP-A-2001-48882
Patent Literature 3: WO 02/10167
Patent Literature 4: WO 96/32383
Patent Literature 5: JP-A-2-32058
Patent Literature 6: JP-A-2001-139574

### DISCLOSURE OF INVENTION

An object of the present invention is to provide a drug having a high affinity for benzodiazepine ω₃ receptor and being effective in the symptoms (obsessive-compulsive disorder, panic disorder), which are not sufficiently treated by the existing benzodiazepine drugs, and further exhibiting therapeutic and preventive effects on central nervous diseases such as anxiety and related diseases thereof, depression, cognitive dysfunction, convulsion, etc., without showing any side effects that are observed in the exiting benzodiazepine drugs such as excessive suppression or mental dependency.

The present inventors have intensively studied, and have found that the compounds as described below exhibit a selective and high affinity for benzodiazepine ω₃ receptor, and have accomplished the present invention.

Namely, the present invention relates to the following:
[1] An antianxiety or antidepressant agent comprising a compound of the formula (1): 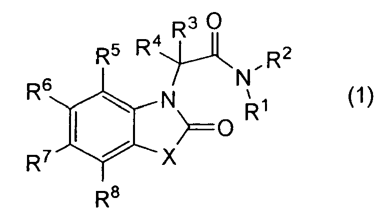 wherein R¹ and R² are independently a hydrogen atom, an optionally substituted alkyl group, an optionally substituted cycloalkyl group, an optionally substituted alkenyl group, an optionally substituted alkynyl group, an optionally substituted aryl group, an optionally substituted heteroaryl group, or an optionally substituted saturated heterocyclic group, or R¹ and R² combine together with the adjacent nitrogen atom to which they bond, and form an optionally substituted saturated heterocyclic group;
   R³ and R⁴ are independently a hydrogen atom, a halogen atom, an optionally substituted alkyl group, an optionally substituted cycloalkyl group, an optionally substituted alkenyl group, or an optionally substituted alkynyl group;
   R⁵, R⁶, R⁷ and R⁸ are independently a hydrogen atom, an optionally substituted alkyl group, an optionally substituted cycloalkyl group, an optionally substituted alkenyl group, an optionally substituted alkynyl group, a halogen atom, a cyano group, a nitro group, a hydroxy group, an optionally substituted amino group, an optionally substituted alkoxy group, an optionally substituted alkanoyl group, an optionally substituted alkoxycarbonyl group, an optionally substituted aryloxycarbonyl group, an optionally substituted heteroaryloxycarbonyl group, a carboxyl group, an optionally substituted carbamoyl group, an optionally substituted sulfamoyl group, an optionally substituted ureido group, an optionally substituted alkylthio group, an optionally substituted alkylsulfinyl group, an optionally substituted alkylsulfonyl group, or a group of the formula: -E-A (in which E is a single bond, an oxygen atom, a sulfur atom, -SO-, -SO₂-, -NR⁹- or -CO-, A is an optionally substituted aryl group or an optionally substituted heteroaryl group, and R⁹ is a hydrogen atom or an optionally substituted alkyl group);
   X is an oxygen atom, a sulfur atom, NR¹⁰, or CR¹¹R¹² (in which R¹⁰ is a hydrogen atom, an optionally substituted alkyl group, an optionally substituted cycloalkyl group, an optionally substituted alkenyl group, an optionally substituted alkynyl group, an optionally substituted alkanoyl group, or an optionally substituted alkoxycarbonyl group, R¹¹ and R¹² are independently a hydrogen atom, an optionally substituted alkyl group, an optionally substituted cycloalkyl group, an optionally substituted alkenyl group, an optionally substituted alkynyl group, an optionally substituted aryl group, an optionally substituted heteroaryl group, a halogen atom, a cyano group, a hydroxy group, an optionally substituted amino group, an optionally substituted alkoxy group, an optionally substituted aryloxy group, an optionally substituted alkanoyl group, an optionally substituted aroyl group, an optionally substituted heteroarylcarbonyl group, an optionally substituted alkoxycarbonyl group, a carboxyl group, or an optionally substituted carbamoyl group, or R¹¹ and R¹² combine each other and form an oxo group or =NOH);
   alternatively,
   (i) when X is NR¹⁰, then by combining R⁸ and R¹⁰, the formula (1) may be expressed by the formula (2): 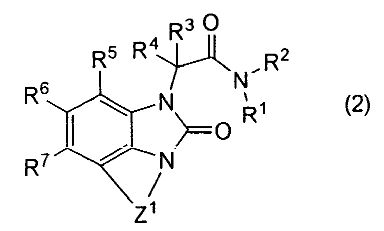 wherein R¹, R², R³, R⁴, R⁵, R⁶ and R⁷ are as defined above, and Z¹ is an optionally substituted alkylene group, and one of the carbon atoms of said alkylene group can be replaced by an oxygen atom, a sulfur atom or -NR¹³- (in which R¹³ is a hydrogen atom or an optionally substituted alkyl group), and a double bond may be formed between any adjacent atoms of said alkylene group;
   (ii) by combining R⁴ and R⁵, the formula (1) may be expressed by the formula (3): 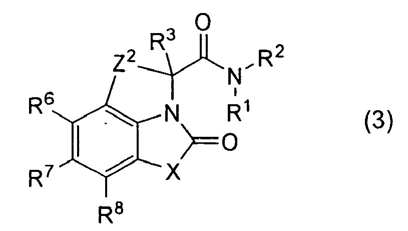
   wherein R¹, R², R³, R⁶, R⁷, R⁸ and X are as defined above, Z² is an optionally substituted alkylene group, and one of the carbon atoms of said alkylene group can be replaced by an oxygen atom, a sulfur atom or -NR¹³- (in which R¹³ is a hydrogen atom or an optionally substituted alkyl group), and a double bond may be formed between any adjacent atoms of said alkylene group;
   provided that
   (1) when X is an oxygen atom or a sulfur atom under the following conditions (a) or (b), then R¹ and R² never form an optionally substituted saturated heterocyclic group by combining together with the adjacent nitrogen atom to which they bond;
      (a) all of R⁵, R⁶, R⁷ and R⁸ are a hydrogen atom;
      (b) one or two groups of R⁵, R⁶, R⁷ and R⁸ are independently a halogen atom, and the remaining groups are a hydrogen atom;
   (2) when X is CR¹¹R¹², and R¹¹ and R¹² are independently an optionally substituted alkyl group, an optionally substituted cycloalkyl group, an optionally substituted alkenyl group, or an optionally substituted alkynyl group under the following conditions (a) or (b), then R¹ and R² are not a hydrogen atom nor an optionally substituted alkyl group, or R¹ and R² never form an optionally substituted saturated heterocyclic group by combining together with the adjacent nitrogen atom;
      (a) all of R⁵, R⁶, R⁷ and R⁸ are a hydrogen atom;
      (b) one or two groups of R⁵, R⁶, R⁷ and R⁸ are independently a halogen atom, an optionally substituted alkyl group or a nitro group, and the remaining groups are a hydrogen atom, or a prodrug thereof, or a pharmaceutically acceptable salt thereof.
   [2] A compound of the formula (1'): 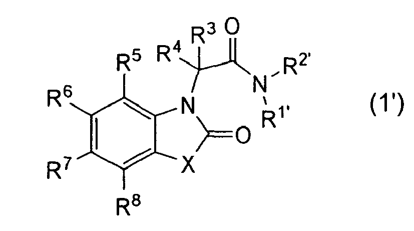 wherein R^{1'} and R^{2'} are independently a hydrogen atom, an optionally substituted alkyl group, an optionally substituted cycloalkyl group, an optionally substituted alkenyl group, an optionally substituted alkynyl group, an optionally substituted aryl group, an optionally substituted heteroaryl group, or an optionally substituted saturated heterocyclic group, or R^{1'} and R²' combine together with the adjacent nitrogen atom to which they bond, and form a group of the formula (4): 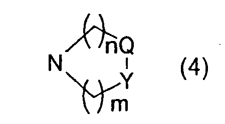 (in which n is 0 or 1, m is 1, 2 or 3, Y is a single bond, an oxygen atom or a sulfur atom, Q is methylene, ethylene, or an optionally substituted o-phenylene group);
      R³ and R⁴ are independently a hydrogen atom, a halogen atom, an optionally substituted alkyl group, an optionally substituted cycloalkyl group, an optionally substituted alkenyl group, or an optionally substituted alkynyl group;
      R⁵, R⁶, R⁷ and R⁸ are independently a hydrogen atom, an optionally substituted alkyl group, an optionally substituted cycloalkyl group, an optionally substituted alkenyl group, an optionally substituted alkynyl group, a halogen atom, a cyano group, a nitro group, a hydroxy group, an optionally substituted amino group, an optionally substituted alkoxy group, an optionally substituted alkanoyl group, an optionally substituted alkoxycarbonyl group, an optionally substituted aryloxycarbonyl group, an optionally substituted heteroaryloxycarbonyl group, a carboxyl group, an optionally substituted carbamoyl group, an optionally substituted sulfamoyl group, an optionally substituted ureido group, an optionally substituted alkylthio group, an optionally substituted alkylsulfinyl group, an optionally substituted alkylsulfonyl group, or a group of the formula: -E-A (in which E is a single bond, an oxygen atom, a sulfur atom, -SO-, -SO₂-, -NR⁹- or -CO-, A is an optionally substituted aryl group or an optionally substituted heteroaryl group, and R⁹ is a hydrogen atom or an optionally substituted alkyl group);
      X is an oxygen atom, a sulfur atom, NR¹⁰, or CR¹¹R¹² (in which R¹⁰ is a hydrogen atom, an optionally substituted alkyl group, an optionally substituted cycloalkyl group, an optionally substituted alkenyl group, an optionally substituted alkynyl group, an optionally substituted alkanoyl group, or an optionally substituted alkoxycarbonyl group, R¹¹ and R¹² are independently a hydrogen atom, an optionally substituted alkyl group, an optionally substituted cycloalkyl group, an optionally substituted alkenyl group, an optionally substituted alkynyl group, an optionally substituted aryl group, an optionally substituted heteroaryl group, a halogen atom, a cyano group, a hydroxy group, an optionally substituted amino group, an optionally substituted alkoxy group, an optionally substituted aryloxy group, an optionally substituted alkanoyl group, an optionally substituted aroyl group, an optionally substituted heteroarylcarbonyl group, an optionally substituted alkoxycarbonyl group, a carboxyl group, or an optionally substituted carbamoyl group, or R¹¹ and R¹² combine and form an oxo group or =NOH),
      alternatively,
      (i) when X is NR¹⁰, then by combining R⁸ and R¹⁰, the formula (1') may be expressed by the formula (2'): 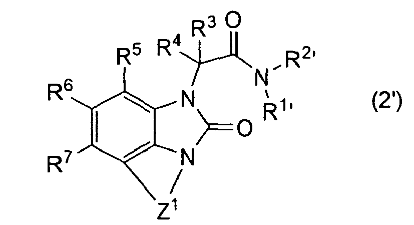 wherein R^{1'}, R^{2'}, R³, R⁴, R⁵, R⁶ and R⁷ are as defined above, Z¹ is an optionally substituted alkylene group, and one of the carbon atoms of said alkylene group can be replaced by an oxygen atom, a sulfur atom or -NR¹³- (in which R¹³ is a hydrogen atom or an optionally substituted alkyl group), and a double bond may be formed between any adjacent atoms of said alkylene group;
      (ii) by combining R⁴ and R⁵, the formula (1') may be expressed by the formula (3'): 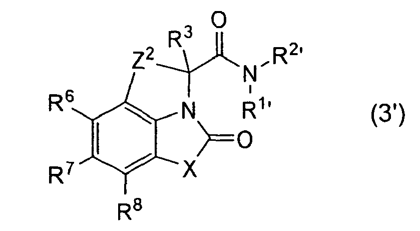
   wherein R^{1'}, R^{2'}, R³, R⁶, R⁷, R⁸ and X are as defined above, Z² is an optionally substituted alkylene group, and one of the carbon atoms of said alkylene group can be replaced by an oxygen atom, a sulfur atom or -NR¹³- (in which R¹³ is a hydrogen atom or an optionally substituted alkyl group), and a double bond may be formed between any adjacent atoms of said alkylene group,
   provided that in cases other than the above (i) or (ii),
   (1) R^{1'} and R^{2'} are not simultaneously a hydrogen atom,
   (2) R^{1'} or R^{2'} is not a saturated heterocyclic group,
   (3) when R^{1'} and R^{2'} combine together with the adjacent nitrogen atom to which they bond and form a group of the formula (4), then Q is an optionally substituted o-phenylene group,
   (4) R⁵, R⁶, R⁷ and R⁸ are not simultaneously a hydrogen atom,
   (5) when one or two groups of R⁵, R⁶, R⁷ and R⁸ are independently a halogen atom or an optionally substituted alkyl group, then the remaining groups are not a hydrogen atom,
   (6) when X is a sulfur atom, and one or two groups of R⁵, R⁶, R⁷ and R⁸ are independently a halogen atom, a nitro group, an alkyl group, a halogen-substituted alkyl group, an alkoxy group, or an optionally substituted amino group, then the remaining groups are not a hydrogen atom,
   (7) when X is an oxygen atom, and one or two groups of R⁵, R⁶, R⁷ and R⁸ are independently a halogen atom, an alkoxy group, or an optionally substituted arylcarbonyl group, and the remaining groups are a hydrogen atom, then R^{1'} or R^{2'} is not a hydrogen atom,
   (8) when X is an oxygen atom, R⁷ is a nitro group, and R⁵, R⁶ and R⁸ are a hydrogen atom, then R^{1'} and R^{2'} are not simultaneously an alkyl group,
   (9) when X is NR¹⁰, and one or two groups of R⁵, R⁶, R⁷ and R⁸ are independently an optionally substituted alkyl group, an optionally substituted alkoxy group, a halogen atom, or a cyano group, then the remaining groups are not a hydrogen atom,
   (10) when X is CR¹¹R¹², then R¹¹ and R¹² are independently a hydrogen atom, an alkyl group optionally substituted by a halogen atom, an optionally substituted cycloalkyl group, an optionally substituted alkenyl group, or an optionally substituted alkynyl group, or R¹¹ and R¹² combine each other and form an oxo group or =NOH, and R^{1'} or R^{2'} is not a hydrogen atom, or a prodrug thereof, or a pharmaceutically acceptable salt thereof.
[3] The compound according to the above [2], wherein in cases where the formula (1') in the above [2] is not expressed by the formula (2') or the formula (3'), and further
   (11) when one or two groups of R⁵, R⁶, R⁷ and R⁸ are independently a halogen atom, an optionally substituted alkyl group, an optionally substituted pyrimidylamino group or an optionally substituted thiazolyl group, then the remaining groups are not a hydrogen atom,
   (12) when X is a sulfur atom, and one or two groups of R⁵, R⁶, R⁷ and R⁸ are independently a halogen atom, a nitro group, an alkyl group, a haloalkyl group, an optionally substituted alkoxy group, or an optionally substituted amino group, then the remaining groups are not a hydrogen atom,
   (13) when X is an oxygen atom, one or two groups of R⁵, R⁶, R⁷ and R⁸ are independently a halogen atom, an optionally substituted alkoxy group, or an optionally substituted arylcarbonyl group, and the remaining groups are a hydrogen atom, then R^{1'} or R^{2'} is not a hydrogen atom,
   (14) when X is NR¹⁰, one or two groups of R⁵, R⁶, R⁷ and R⁸ are independently an optionally substituted heteroaryl group, and the remaining groups are a hydrogen atom, then R^{1'} or R^{2'} is not a hydrogen atom,
      or a prodrug thereof, or a pharmaceutically acceptable salt thereof.
[4] The compound according to the above [2], wherein X is NR¹⁰, and R⁸ and R¹⁰ combine each other, and thereby said compound is expressed by the formula (2"): 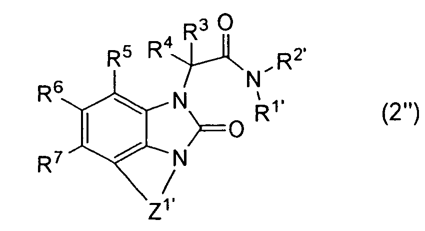 in which R^{1'}, R^{2'}, R³, R⁴, R⁵, R⁶ and R⁷ are as defined in the above [2], and Z^{1'} is an optionally substituted alkylene group, and one of the carbon atoms of said alkylene group can be replaced by an oxygen atom, a sulfur atom or -NR¹³- (in which R¹³ is a hydrogen atom or an optionally substituted alkyl group),
   or a prodrug thereof, or a pharmaceutically acceptable salt thereof.
[5] The compound according to the above [4], wherein at least one of R⁵, R⁶ and R⁷ is -E-A (in which E and A are as defined in the above [2]), or a prodrug thereof, or a pharmaceutically acceptable salt thereof.
[6] The compound according to the above [4] or [5], wherein Z^{1'} is an optionally substituted trimethylene or tetramethylene, and one of the carbon atoms of said trimethylene and tetramethylene can be replaced by an oxygen atom, or a prodrug thereof, or a pharmaceutically acceptable salt thereof.
[7] The compound according to the above [2], wherein R⁴ and R⁵ combine each other, and thereby said compound is expressed by the formula (3"): 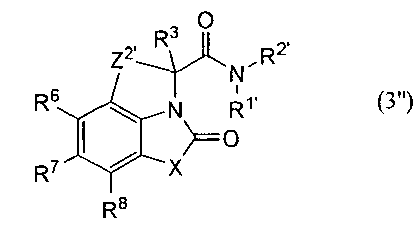 in which R^{1'}, R^{2'}, R³, R⁶, R⁷, R⁸ and X are as defined in the above [2], Z^{2'} is an optionally substituted alkylene group, and one of the carbon atoms of said alkylene group can be replaced by an oxygen atom, or a prodrug thereof, or a pharmaceutically acceptable salt thereof.
[8] The compound according to the above [7], wherein at least one of R⁶, R⁷ and R⁸ is -E-A (in which E and A are as defined in the above [2]), or a prodrug thereof, or a pharmaceutically acceptable salt thereof.
[9] The compound according to the above [7] or [8], wherein Z^{2'} is an optionally substituted ethylene group, or a prodrug thereof, or a pharmaceutically acceptable salt thereof.
[10] The compound according to the above [2] or [3], wherein R^{1'} is a hydrogen atom, an optionally substituted alkyl group, an optionally substituted cycloalkyl group, an optionally substituted alkenyl group, or an optionally substituted alkynyl group, R^{2'} is an optionally substituted alkyl group, an optionally substituted aryl group, or an optionally substituted heteroaryl group, or R^{1'} and R^{2'} combine together with the nitrogen atom to which they bond, and form a group of the formula (4'): 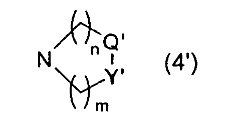 (in which n is 0 or 1, m is 1, 2 or 3, Y' is a single bond or an oxygen atom, and Q' is an optionally substituted o-phenylene group);
   R³ and R⁴ are independently a hydrogen atom, a halogen atom, or an optionally substituted alkyl group;
   at least one of R⁵, R⁶, R⁷ and R⁸ is a group of the formula: -E-A (in which E and A are as defined in the above [2]), or a prodrug thereof, or a pharmaceutically acceptable salt thereof.
[11] The compound according to the above [10], wherein X is an oxygen atom or a sulfur atom, or a prodrug thereof, or a pharmaceutically acceptable salt thereof.
[12] The compound according to the above [10], wherein X is NR¹⁰, and R¹⁰ is a hydrogen atom or an optionally substituted alkyl group, or a prodrug thereof, or a pharmaceutically acceptable salt thereof.
[13] The compound according to the above [10], wherein X is CR¹¹R¹², and R¹¹ and R¹² are independently a hydrogen atom, an alkyl group optionally substituted by a halogen atom, an optionally substituted cycloalkyl group, an optionally substituted alkenyl group, or an optionally substituted alkynyl group, or a prodrug thereof, or a pharmaceutically acceptable salt thereof.
[14] The compound according to the above [2] or [3], wherein R^{1'} and R^{2'} are a hydrogen atom or an optionally substituted alkyl group, R⁵, R⁶, R⁷ and R⁸ are independently an alkyl group substituted by a hydroxy group, a nitro group, a cyano group, an alkoxy group, a cycloalkyl group, an optionally substituted amino group, an alkylsulfonyl group, an arylsulfonyl group, or an optionally substituted heteroaryl group; an optionally substituted cycloalkyl group; an optionally substituted alkenyl group; an optionally substituted alkynyl group; a hydroxy group; a substituted amino group; a substituted alkoxy group; an optionally substituted alkanoyl group; an optionally substituted alkoxycarbonyl group; an optionally substituted aryloxycarbonyl group; an optionally substituted heteroaryloxycarbonyl group; a carboxyl group; an optionally substituted carbamoyl group; an aryl-substituted sulfamoyl group; an optionally substituted ureido group; an optionally substituted alkylthio group; an optionally substituted alkylsulfinyl group; an optionally substituted alkylsulfonyl group; or a group of the formula: -E-A' (in which E is a single bond, an oxygen atom, a sulfur atom, -SO-, -SO₂-, -NR⁹- or -CO-, A' is a phenyl group substituted by a hydroxy- or amino-substituted alkyl group, a halogen-substituted alkoxy group, an alkoxycarbonyl group, a carboxyl group, an amino group (said amino group may optionally be substituted by one or two groups selected from an alkyl group, an alkanoyl group and an alkoxycarbonyl group), a carbamoyl group, an aryl group, an aryloxy group, an alkylsulfonyl group or an arylsulfonyl group; an optionally substituted naphthyl group; or an optionally substituted heteroaryl group, R⁹ is a hydrogen atom or an optionally substituted alkyl group), or a prodrug thereof, or a pharmaceutically acceptable salt thereof.
[15] The compound according to the above [2] or [3], wherein at least one of R^{1'} and R^{2'} is an aryl group (said aryl group may optionally be substituted by a halogen atom, a hydroxy group, an alkoxy group, or an alkanoyl group), X is a sulfur atom, and R⁵, R⁶, R⁷ and R⁸ are independently a substituted alkyl group (the substituent thereof is selected from a hydroxy group, a nitro group, a cyano group, an alkoxy group, a cycloalkyl group, an amino group, an alkylamino group, a dialkylamino group, an alkanoylamino group, an alkoxycarbonylamino group, an alkylsulfonyl group, an arylsulfonyl group, an optionally substituted aryl group and an optionally substituted heteroaryl group); an optionally substituted cycloalkyl group; an optionally substituted alkenyl group; an optionally substituted alkynyl group; a halogen atom; a cyano group; a nitro group; a hydroxy group; an optionally substituted amino group; a substituted alkoxy group; an optionally substituted alkanoyl group; an optionally substituted alkoxycarbonyl group; an optionally substituted aryloxycarbonyl group; an optionally substituted heteroaryloxycarbonyl group; a carboxyl group; an optionally substituted carbamoyl group; an optionally substituted sulfamoyl group; an optionally substituted ureido group; an optionally substituted alkylthio group; an optionally substituted alkylsulfinyl group; an optionally substituted alkylsulfonyl group; or a group of the formula: -E-A (in which E is a single bond, an oxygen atom, a sulfur atom, -SO-, -SO₂-, -NR⁹- or -CO-, A is an optionally substituted aryl group or an optionally substituted heteroaryl group, R⁹ is a hydrogen atom or an optionally substituted alkyl group), or a prodrug thereof, or a pharmaceutically acceptable salt thereof.
[16] The compound according to the above [2] or [3], wherein at least one of R^{1'} and R^{2'} is an aryl group (said aryl group may optionally be substituted by a halogen atom, a hydroxy group, an alkoxy group, or an alkanoyl group) and X is an oxygen atom, NR¹⁰, or CR¹¹R¹², or a prodrug thereof, or a pharmaceutically acceptable salt thereof.
[17] A compound of the formula (5): 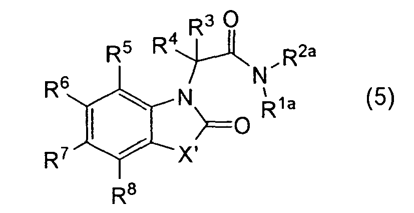 wherein R^{1a} is an optionally substituted alkyl group or an optionally substituted cycloalkyl group, R^{2a} is an optionally substituted aryl group or an optionally substituted heteroaryl group, or R^{1a} and R^{2a} combine together with the nitrogen atom to which they bond and form a group of the formula (4"): 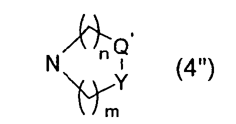 (in which n, m and Y are as defined in the above [2], and Q' is an optionally substituted o-phenylene group),
   R³ and R⁴ are independently a hydrogen atom, a halogen atom, an optionally substituted alkyl group, an optionally substituted cycloalkyl group, an optionally substituted alkenyl group, or an optionally substituted alkynyl group,
   R⁵, R⁶, R⁷ and R⁸ are independently a hydrogen atom, an optionally substituted alkyl group, an optionally substituted cycloalkyl group, an optionally substituted alkenyl group, an optionally substituted alkynyl group, a halogen atom, a cyano group, a nitro group, a hydroxy group, an optionally substituted amino group, an optionally substituted alkoxy group, an optionally substituted alkanoyl group, an optionally substituted alkoxycarbonyl group, an optionally substituted aryloxycarbonyl group, an optionally substituted heteroaryloxycarbonyl group, a carboxyl group, an optionally substituted carbamoyl group, an optionally substituted sulfamoyl group, an optionally substituted ureido group, an optionally substituted alkylthio group, an optionally substituted alkylsulfinyl group, an optionally substituted alkylsulfonyl group, or a group of the formula: -E-A (in which E is a single bond, an oxygen atom, a sulfur atom, -SO-, -SO₂-, -NR⁹- or -CO-, A is an optionally substituted aryl group or an optionally substituted heteroaryl group, and R⁹ is a hydrogen atom or an optionally substituted alkyl group), provided that R⁵, R⁶, R⁷ and R⁸ are not simultaneously a hydrogen atom,
   X' is an oxygen atom, a sulfur atom, NR¹⁰, or CR^{11a}R^{12a} (in which R¹⁰ is as defined in the above [2], R^{11a} and R^{12a} are independently a hydrogen atom, an alkyl group optionally substituted by a halogen atom, an optionally substituted cycloalkyl group, an optionally substituted alkenyl group, or an optionally substituted alkynyl group, or R^{11a} and R^{12a} combine and form an oxo group or =NOH),
   provided that
   (1) when X is a sulfur atom or NR¹⁰, and one or two groups of R⁵, R⁶, R⁷ and R⁸ are independently a halogen atom, an alkyl group, a trihalomethyl group, or an optionally substituted alkoxy group, then the remaining groups are not a hydrogen atom,
   (2) when X is an oxygen atom, then R⁷ is not a halogen atom,
   or a prodrug thereof, or a pharmaceutically acceptable salt thereof.
[18] The compound according to the above [17], wherein R^{1a} is an optionally substituted alkyl group or an optionally substituted cycloalkyl group, R^{2a} is an optionally substituted aryl group or an optionally substituted heteroaryl group, and at least one of R⁵, R⁶, R⁷ and R⁸ is a group of the formula: -E-A (in which E and A are as defined in the above [2]), or a prodrug thereof, or a pharmaceutically acceptable salt thereof.
   [18-2] The compound according to the above [17], wherein R⁶ is a group of the formula: -E-A (in which E and A are as defined in the above [2]), or a prodrug thereof, or a pharmaceutically acceptable salt thereof.
[19] The compound according to the above [18] and [18-2], wherein E is a single bond, or a prodrug thereof, or a pharmaceutically acceptable salt thereof.
[20] The compound according to the above [17], wherein R^{1a} is an optionally substituted alkyl group, R^{2a} is an optionally substituted aryl group or an optionally substituted heteroaryl group, and R⁶ and/or R⁸ are a halogen atom, or a prodrug thereof, or a pharmaceutically acceptable salt thereof.
[21] A compound of the formula (6): 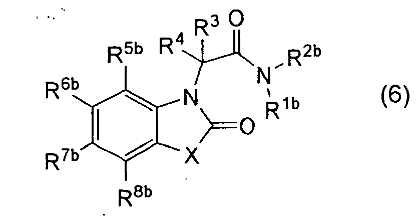 wherein R^{1b} and R^{2b} are independently a substituted alkyl group, an optionally substituted alkenyl group, or an optionally substituted alkynyl group,
   R³ and R⁴ are as defined in the above [2],
   R^{5b}, R^{6b}, R^{7b} and R^{8b} are independently a hydrogen atom, an optionally substituted alkyl group, an optionally substituted cycloalkyl group, an optionally substituted alkenyl group, an optionally substituted alkynyl group, a halogen atom, a cyano group, a nitro group, a hydroxy group, an optionally substituted amino group, an optionally substituted alkoxy group, an optionally substituted alkanoyl group, an optionally substituted alkoxycarbonyl group, an optionally substituted aryloxycarbonyl group, an optionally substituted heteroaryloxycarbonyl group, a carboxyl group, an optionally substituted carbamoyl group, an optionally substituted sulfamoyl group, an optionally substituted ureido group, an optionally substituted alkylthio group, an optionally substituted alkylsulfinyl group, an optionally substituted alkylsulfonyl group, or a group of the formula: -E-A^{b} (in which E is as defined in the above [2], A^{b} is a substituted phenyl group (the substituent thereof is selected from a halogen atom, an alkyl group substituted by a hydroxy group or an optionally substituted amino group, a halogen-substituted alkoxy group, an alkoxycarbonyl group, a carboxyl group, an amino group (said amino group being optionally substituted by one or two groups selected from an alkyl group, an alkanoyl group, an alkoxycarbonyl group, etc.), a carbamoyl group, an aryl group, an aryloxy group, an alkylsulfonyl group and an arylsulfonyl group); an optionally substituted naphthyl group; or an optionally substituted heteroaryl group), and at least one of R^{5b}, R^{6b}, R^{7b} and R^{8b} is a group of the formula: -E-A^{b},
   X is an oxygen atom, a sulfur atom, NR¹⁰, or CR^{11b}R^{12b} (in which R¹⁰ is as defined in the above [2], R^{11b} and R^{12b} are independently a hydrogen atom, an alkyl group optionally substituted by a halogen atom, an optionally substituted cycloalkyl group, an optionally substituted alkenyl group, or an optionally substituted alkynyl group, or R^{11b} and R^{12b} combine to form an oxo group or =NOH), or a prodrug thereof, or a pharmaceutically acceptable salt thereof.
   [21-2] The compound according to the above [21], wherein R^{6b} is a group of the formula: -E-A^{b} (in which E and A^{b} are as defined in the above [21]), or a prodrug thereof, or a pharmaceutically acceptable salt thereof.
[22] A drug comprising as the active ingredient the compound as set forth in any one of the above [2] to [21-2], or a prodrug thereof, or a pharmaceutically acceptable salt thereof, and
[23] An antianxiety or antidepressant agent comprising as the active ingredient the compound as set forth in any one of the above [2] to [21-2], or a prodrug thereof, or a pharmaceutically acceptable salt thereof.

### BEST MODE FOR CARRYING OUT THE INVENTION

The present invention is illustrated in more detail below.

The definition for each group of the present invention may be applied to the cases wherein said group constitutes only a portion of other groups, unless specified otherwise.

Besides, the number of the substituents of the present specification is not necessarily specified and may be one or more as long as the substitution thereby is possible.

The "halogen atom" is fluorine atom, chlorine atom, bromine atom or iodine atom.

Preferable halogen atom for R³ and R⁴ is, for example, fluorine atom.

The "alkyl group" includes a straight chain or branched chain alkyl group having 1 to 10 carbon atoms, for example, methyl, ethyl, propyl, isopropyl, butyl, isobutyl, sec-butyl, tert-butyl, pentyl, 1-methylbutyl, 2-methylbutyl, 3-methylbutyl, 1-ethylpropyl, hexyl, heptyl, octyl, nonyl and decyl. Preferable alkyl group may be a straight chain or branched chain alkyl group having 1 to 6 carbon atoms.

The "alkenyl group" includes a straight chain or branched chain alkenyl group having at least one double bond and having 2 to 6 carbon atoms, for example, vinyl, 1-propenyl, 2-propenyl, 1-methyl-1-propenyl, 1-butenyl, 2-butenyl, 3-butenyl and 1-methyl-1-butenyl. Preferable alkenyl group may be a straight chain or branched chain alkenyl group having 3 to 6 carbon atoms.

The "alkynyl group" includes a straight chain or branched chain alkynyl group having at least one triple bond and having 2 to 6 carbon atoms, for example, ethynyl, 1-propynyl, 2-propynyl, 1-methyl-2-propynyl, 1-butynyl, 2-butynyl, 3-butynyl and 1-methyl-2-butynyl. Preferable alkynyl group may be a straight chain or branched chain alkynyl group having 3 to 6 carbon atoms.

The "cycloalkyl group" includes a saturated or unsaturated cycloalkyl group having 3 to 8 carbon atoms, for example, cyclopropyl, cyclobutyl, cyclopentyl, cyclohexyl, cycloheptyl, cyclooctyl, cyclobutenyl, cyclopentenyl, cyclohexenyl, cycloheptenyl, and cycloctenyl. Preferable cycloalkyl group may be a saturated or unsaturated cycloalkyl group having 3 to 6 carbon atoms.

The "alkoxy group" includes a straight chain or branched chain alkoxy group having 1 to 10 carbon atoms, for example, methoxy, ethoxy, propoxy, butoxy, isopropoxy, isobutoxy, sec-butoxy, tert-butoxy, pentoxy, hexoxy, heptoxy, octoxy, nonyloxy, and decyloxy. Preferable alkoxy group may be a straight chain or branched chain alkoxy group having 1 to 6 carbon atoms.

The "alkanoyl group" includes a straight chain or branched chain alkanoyl group having 1 to 10 carbon atoms, for example, formyl, acetyl, propionyl, butyryl, isobutyryl, valeryl, isovaleryl, pivaloyl, hexanoyl, heptanoyl, octanoyl, nonanoyl, and decanoyl. Preferable alkanoyl group may be a straight chain or branched chain alkanoyl group having 1 to 6 carbon atoms.

The "alkanoyloxy group" includes a straight chain or branched chain alkanoyloxy group having 1 to 10 carbon atoms, for example, formyloxy, acetyloxy, propionyloxy, butyryloxy, isobutyryloxy, valeryloxy, isovaleryloxy, pivaloyloxy, hexanoyloxy, heptanoyloxy, octanoyloxy, nonanoyloxy, and decanoyloxy. Preferable alkanoyloxy group may be an alkanoyloxy group having a straight chain or branched chain alkanoyl group having 1 to 6 carbon atoms.

The "alkoxycarbonyl group" includes a straight chain or branched chain alkoxycarbonyl group having 2 to 11 carbon atoms, for example, methoxycarbonyl, ethoxycarbonyl, propoxycarbonyl, butoxycarbonyl, isopropoxycarbonyl, isobutoxycarbonyl, sec-butoxycarbonyl, tert-butoxycarbonyl, pentoxycarbonyl, hexoxycarbonyl, heptoxycarbonyl, octoxycarbonyl, nonyloxycarbonyl, and decyloxycarbonyl, etc. Preferable alkoxycarbonyl group may be an alkoxycarbonyl group having a straight chain or branched chain alkoxy group having 1 to 6 carbon atoms.

The "alkylthio group" includes an alkylthio group having 1 to 10 carbon atoms, for example, methylthio, ethylthio, propylthio, butylthio, isopropylthio, isobutylthio, sec-butylthio, tert-butylthio, pentylthio, hexylthio, heptylthio, octylthio, nonylthio, and decylthio, etc. Preferable alkylthio group may be an alkylthio group having a straight chain or branched chain alkyl group having 1 to 6 carbon atoms.

The "alkylsulfinyl group" includes an alkylsulfinyl group having 1 to 10 carbon atoms, for example, methylsulfinyl, ethylsulfinyl, propylsulfinyl, butylsulfinyl, isopropylsulfinyl, isobutylsulfinyl, sec-butylsulfinyl, tert-butylsulfinyl, pentylsulfinyl, hexylsulfinyl, heptylsulfinyl, octylsulfinyl, nonylsulfinyl, and decylsulfinyl. Preferable alkylsulfinyl group may be an alkylsulfinyl group having a straight chain or branched chain alkyl group having 1 to 6 carbon atoms.

The "alkylsulfonyl group" includes alkylsulfonyl group having 1 to 10 carbon atoms, for example, methylsulfonyl, ethylsulfonyl, propylsulfonyl, butylsulfonyl, isopropylsulfonyl, isobutylsulfonyl, sec-butylsulfonyl, tert-butylsulfonyl, pentylsulfonyl, hexylsulfonyl, heptylsulfonyl, octylsulfonyl, nonylsulfonyl, and decylsulfonyl. Preferable alkylsulfonyl group may be an alkylsulfonyl group having a straight chain or branched chain alkyl group having 1 to 6 carbon atoms.

The "trihalomethyl group" includes, for example, trifluoromethyl, trichloromethyl and tribromomethyl, etc.

The substituent of the "substituted alkyl group", "substituted alkenyl group", "substituted alkynyl group", "substituted alkoxy group", "substituted cycloalkyl group", "substituted alkanoyl group", "substituted alkoxycarbonyl group", "substituted alkylthio group", "substituted alkylsulfinyl group" and "substituted alkylsulfonyl group" may be, for example, a halogen atom, a hydroxy group, a nitro group, a cyano group, an alkoxy group, a cycloalkyl group, an amino group, an alkylamino group, a dialkylamino group, an alkanoylamino group, an alkoxycarbonylamino group, an alkylsulfonyl group, or an arylsulfonyl group, etc.

The substituent of the substituted alkyl, substituted alkoxy, and substituted alkynyl group includes, in addition to the above substituents, an optionally substituted aryl group and an optionally substituted heteroaryl group.

The substituent of the substituted cycloalkyl group includes, in addition to the above substituents, an alkyl group.

The preferable substituents for the "substituted alkyl group", "substituted alkenyl group", "substituted alkynyl group", "substituted alkoxy group", "substituted cycloalkyl group", "substituted alkanoyl group", "substituted alkoxycarbonyl group", "substituted alkylthio group", "substituted alkylsulfinyl group", and "substituted alkylsulfonyl group" may be, for example, a halogen atom, a hydroxy group, an amino group, an alkylamino group, or a dialkylamino group, etc.

In addition, the preferable substituent for the "substituted alkyl group" represented by R⁹, R¹⁰ and R¹³ may be, for example, an aryl group or a heteroaryl group, etc.

The "aryl group" includes, for example, an aryl group having not more than 10 carbon atoms, for example, phenyl and naphthyl.

The "heteroaryl group" includes, for example, a 5- or 6-membered aromatic heteromonocyclic group or a 9- or 10-membered aromatic heterobicyclic group having 1 to 4 heteratoms selected from a nitrogen atom, a sulfur atom and an oxygen atom, such as pyridyl (the nitrogen atom thereof may be oxidized), thienyl, furyl, pyrrolyl, pyrazolyl, imidazolyl, pyrazyl, pyrimidyl, pyridazyl, oxazolyl, thiazolyl, oxadiazolyl, triazolyl, tetrazolyl, quinolyl, benzothienyl, benzofuryl, indolyl, quinazolinyl, benzothiazolyl, benzoxazolyl, benzimidazolyl, naphthyridinyl, and imidazopyridinyl, etc. Preferable heteroaryl group may be a 5- or 6-membered aromatic heteromonocyclic group having 1 to 3 heteroatoms selected from a nitrogen atom, a sulfur atom and an oxygen atom, and more preferable one is pyridyl.

The "aroyl group" may be, for example, an arylcarbonyl group having a C₆₋₁₀ aryl group such as benzoyl, toluoyl, naphthoyl, etc.

The aryl moiety or heteroaryl moeity of the "aryloxycarbonyl group", "aryloxy group", "arylthio group", "arylsulfinyl group", "arylsulfonyl group", "heteroarylcarbonyl group" and "heteroaryloxycarbonyl group" is the same as defined above.

The substituent of the "substituted aryl group" and "substituted heteroaryl group" includes, for example, a halogen atom, a hydroxy group, a nitro group, a cyano group, an alkyl group (said alkyl group may optionally be substituted, for example, by a halogen atom, a hydroxy group, an alkanoyloxy group, an optionally substituted amino group, or a saturated heterocyclic group optionally substituted by an alkyl group optionally substituted by a hydroxy group, an alkanoyl group, a halogen atom, a hydroxy group or an alkoxycarbonyl group), an alkoxy group (said alkoxy group may optionally be substituted, for example, by a halogen atom, a hydroxy group, a carboxyl group, a cycloalkyl group, an optionally substituted amino group, or a saturated heterocyclic group optionally substituted by an alkyl group, an alkanoyl group, a halogen atom, a hydroxy group or an alkoxycarbonyl group), an alkoxycarbonyl group, a carboxyl group, an alkanoyl group (said alkanoyl group may optionally be substituted, for example, by a halogen atom, etc.), an amino group(said amino group may optionally be substituted, for example, by one or two groups selected from an unsubstituted alkyl group, an alkanoyl group, an alkoxycarbonyl group and a saturated heterocyclic group), a carbamoyl group (said carbamoyl group may optionally have, for example, 1 or 2 groups selected from an unsubstituted alkyl group and an alkyl group substituted by a dialkylamino group or a saturated heterocyclic group), a sulfamoyl group (said sulfamoyl group may optionally be substituted by one or two alkyl groups, etc.), an aryl group, a saturated heterocyclic group (said saturated heterocyclic group may optionally be substituted by an alkyl group optionally substituted by a hydroxy group), an aryloxy group, an alkylsulfonyl group and an arylsulfonyl group, etc.

The substituent of the substituted aryl group may include an alkylenedioxy group such as methylenedioxy, ethylenedioxy, etc.

In addition, the substituted aryl group may include a group of the formula (7): 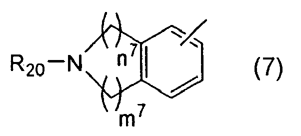 wherein n⁷ is 0, 1 or 2, m⁷ is1, 2, 3 or 4, the sum of n⁷ and m⁷ is 2, 3, or 4, and R²⁰ is a hydrogen atom, an optionally substituted alkyl group, an optionally substituted alkanoyl group, or an optionally substituted alkoxycarbonyl group).

The substituent of the substituted aryl and the substituted heteroaryl for the substituent of the substituted alkyl group and the substituted alkoxy group, and the substituent of the "substituted aryloxycarbonyl group", "substituted arylthio group", "substituted arylsulfinyl group", "substituted arylsulfonyl group", "substituted heteroaryloxycarbonyl group" and "substituted o-phenylene group" are the same as the substituents of the above-mentioned "substituted aryl group" and "substituted heteroaryl group".

The preferable substituent of the substituted aryl group and the substituted heteroaryl group as the substituent of the "substituted alkyl group" and "substituted alkoxy group" for R¹ and R², and the preferable substituent of the "substituted aryl group", "substituted heteroaryl group", "substituted aryloxycarbonyl group", "substituted arylthio group", "substituted arylsulfinyl group", "substituted arylsulfonyl group", "substituted heteroaryloxycarbonyl group" and "substituted o-phenylene group" are, for example, a halogen atom, a hydroxy group, a nitro group, a cyano group, an alkyl group (said alkyl group may optionally be substituted, for example, by a halogen atom, a hydroxy group or an amino group), an alkoxy group (said alkoxy group may optionally be substituted, for example, by a halogen atom, etc.), an alkoxycarbonyl group, a carboxyl group, an amino group, an alkylamino group, a dialkylamino group, a carbamoyl group or an alkylenedioxy group, etc.

The saturated heterocycle of the "saturated heterocyclic group" includes, for example, a 4- to 8-membered saturated heteromonocyclic group having 1 to 4 heteroatoms selected from a nitrogen atom, a sulfur atom and an oxygen atom, such as pyrrolidine, piperidine, piperazine, morpholine, thiomorpholine, perhydroazepine, tetrahydrofuran, and tetrahydropyrane, etc.

In addition, these saturated heterocyclic groups may be condensed with a benzene ring.

The binding position of the saturated heterocyclic group is either at the carbon atom or at the nitrogen atom.

Preferable saturated heterocyclic group includes a 5- or 6-membered saturated heteromonocyclic group having 1 to 2 heteroatoms selected from a nitrogen atom and an oxygen atom, for example, ones represented by the following formulae where a saturated heterocyclic group is condensed with a benzene ring.

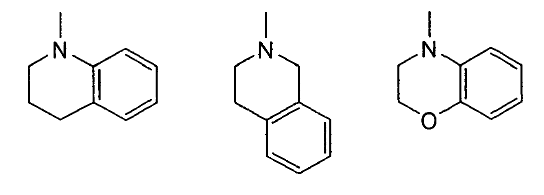

The substituent of the substituted saturated heterocyclic group includes, for example, a halogen atom, a hydroxy group, an alkyl group (said alkyl group may optionally be substituted, for example, by a halogen atom or a hydroxy group, etc.), and an alkoxy group (said alkoxy group may optionally be substituted, for example, by a halogen atom, etc.).

The substituent of the "substituted amino group" includes, for example, an alkyl group, an alkyl group optionally substituted by an aryl group, an aryl group optionally substituted by an alkyl group, a halogen atom, an alkoxy group or a trihalomethyl group, an alkanoyl group, an alkoxycarbonyl group and an aroyl group, etc.

The substituent of the "substituted carbamoyl group", "substituted sulfamoyl group" and "ureido group" includes, for example, an alkyl group, an alkyl group optionally substituted by an aryl group optionally substituted by an alkyl group or a halogen atom, and an aryl group optionally substituted by an alkyl group or a halogen atom.

The "alkylene group" includes, for example, an alkylene group having 1 to 5 carbon atoms such as methylene, ethylene, trimethylene, tetramethylene or pentamethylene, and one of the carbon atoms thereof can be replaced by an oxygen atom, a sulfur atom or -NR¹³- (in which R¹³ may be, for example, a hydrogen atom or an optionally substituted alkyl group, etc.). In addition, a double bond may optionally be formed between any adjacent atoms of said alkylene group.

Preferable alkylene group for Z¹ and Z^{1'} includes an alkylene group having 3 or 4 carbon atoms, and an alkylene group having 2 or 3 carbon atoms and one oxygen atom.

Preferable alkylene group for Z² and Z^{2'} includes an alkylene group having 2 or 3 carbon atoms.

The substituent of the "optionally substituted alkylene group" includes, for example, a halogen atom, a hydroxy group, an alkyl group (said alkyl group may optionally be substituted, for example, by a hydroxy group or a halogen atom), an alkoxy group (said alkoxy group may optionally be substituted, for example, by a halogen atom, etc.), an optionally substituted amino group, etc.

The group for R⁶ is preferably a group of the formula -E-A (in which E and A are as defined above). The substituted aryl group for A includes, for example, a group of the formula (7): 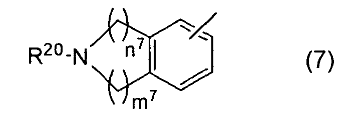 (in which, n⁷ is 0, 1 or 2, m⁷ is 1, 2, 3 or 4, the sum of n⁷ and m⁷ is 2, 3, or 4, R²⁰ is a hydrogen atom, an optionally substituted alkyl group, an optionally substituted alkanoyl group, or an optionally substituted alkoxycarbonyl group). In addition, E is preferably a single bond.

Further, preferably the present invention excludes 2-[6-[(dimethylamino)sulfonyl]-2-oxo-1,3-benzoxazol-3(2H)-yl]-N-(1-phenylethyl)acetamide and 2-[6-[(dimethylamino)sulfonyl]-2-oxo-1,3-benzoxazol-3(2H)-yl]-N-methyl-N-phenylacetamide.

The present compound (1) or a pharmaceutically acceptable salt thereof may be prepared, for example, by the following method.

### Method 1

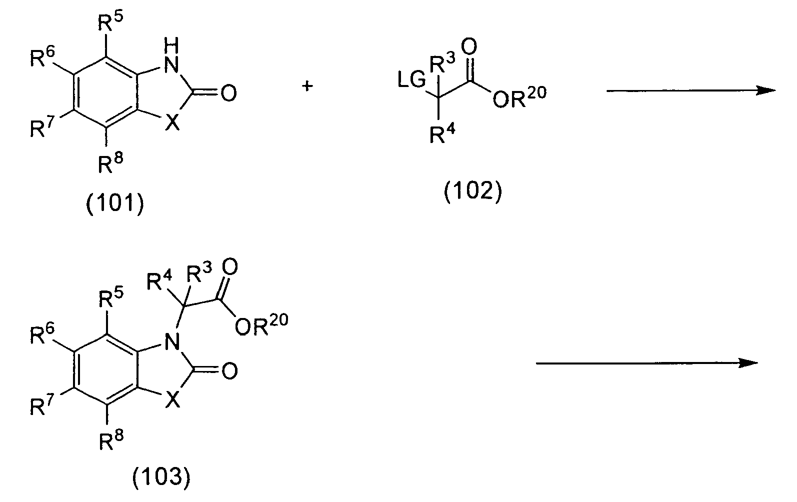

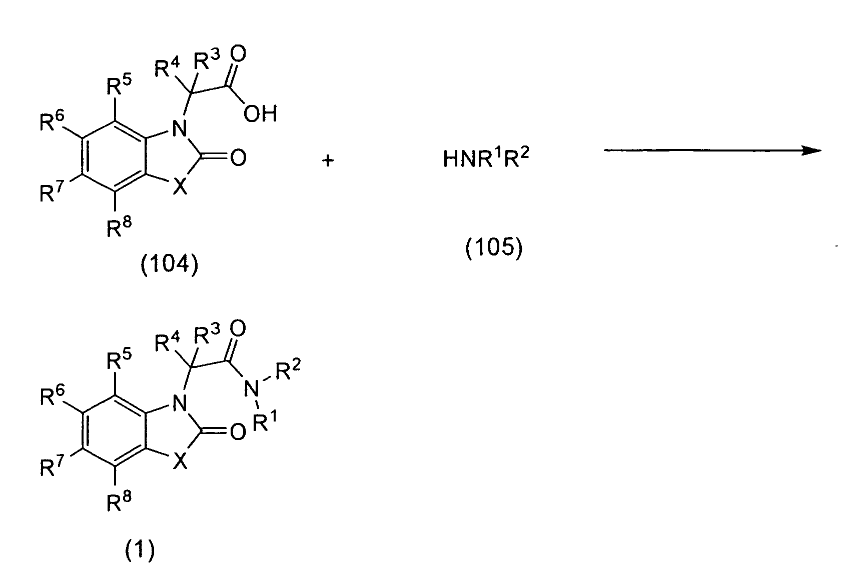

(in which, R¹, R², R³, R⁴, R⁵, R⁶, R⁷, R⁸ and X are as defined above, LG is a leaving group (e.g., a halogen atom such as chlorine atom, bromine atom or iodine atom, an acyloxy group such as acetoxy, or a sulfonyloxy group such as tosyloxy or mesyloxy, etc.), R²⁰ is an alkyl group (e.g., methyl, ethyl or t-butyl, etc.))

### Step 1 (alkylation)

The compound (101) or a salt thereof is reacted with the compound (102) or a salt thereof to give the intermediate (103). The reaction is carried out in the presence of a base, if necessary, or possibly in the presence of a phase-transfer catalyst, in a suitable inert solvent at a temperature of from about -20°C to a boiling point of the solvent to be used, for 10 minutes to 48 hours.

The base includes, for example, an organic base such as triethylamine or pyridine, etc., an inorganic base such as potassium carbonate, sodium hydroxide or sodium hydride, etc., and a metal alkoxide such as sodium methoxide or potassium tert-butoxide, etc.

The phase-transfer catalyst includes, for example, tetrabutylammonium hydrogen sulfate, etc.

The inert solvent includes, for example, acetonitrile, halogenated hydrocarbons such as chloroform or dichloromethane, aromatic hydrocarbons such as benzene, toluene, etc., ethers such as diethyl ether, tetrahydrofuran or 1,4-dioxane, etc., alcohols such as methanol, ethanol or 2-propanol, etc., and aprotic polar solvents such as N,N-dimethylformamide, N-methylpyrrolidone or dimethylsulfoxide, etc., and a mixed solvent of these solvents.

### Step 2 (hydrolysis)

The intermediate (103) is hydrolyzed to give the intermediate (104). The reaction is carried out in a suitable solvent under an acidic or basic conditions at a temperature of from about 0°C to a boiling point of the solvent to be used, for 10 minutes to 48 hours. The solvent includes, for example, alcohols such as methanol, ethanol, 2-propanol, ethers such as 1,4-dioxane, etc., water, and a mixed solvent of these solvents. The acid includes, for example, an inorganic acid such as hydrochloric acid or sulfuric acid, or an organic acid such as formic acid, acetic acid, propionic acid, and oxalic acid, etc. The base includes, for example, an alkali metal hydroxide such as lithium hydroxide, sodium hydroxide or potassium hydroxide, and an alkali metal carbonate such as sodium carbonate or potassium carbonate, etc.

### Step 3 (condensation)

The intermediate (104) or a salt thereof is reacted with the compound (105) or a salt thereof for amide bond formation to give the compound (1). The amide bond formation reaction is carried out by a conventional method, for example, acid chloride method using thionyl chloride or oxalyl chloride, etc., an acid anhydride method using a corresponding acid anhydride, a mixed acid anhydride method using chlorocarbonic acid ester, etc., or a method using a condensing agent such as dicyclohexylcarbodiimide or carbonyl diimidazole, etc.

The compound (1) or a pharmaceutically acceptable salt thereof may also be prepared, for example, by the following method.

### Method 2 (alkylation)

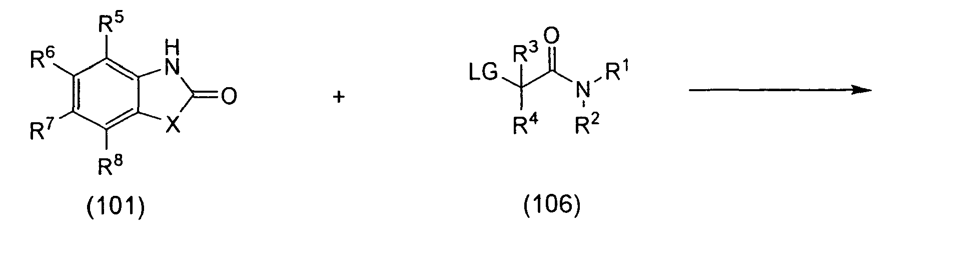

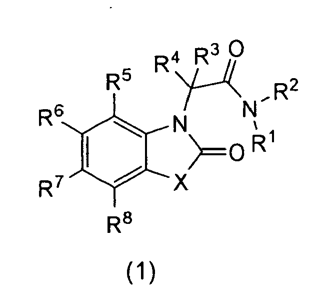

(wherein R¹, R², R³, R⁴, R⁵, R⁶, R⁷, R⁸ and X are as defined above, and LG is as defined in Method 1)

The compound (101) or a salt thereof is reacted with the compound (106) or a salt thereof to give the compound (1). The reaction is carried out in the presence of a base, if necessary, or possibly in the presence of a phase-transfer catalyst in a suitable inert solvent at a temperature of from about -20°C to a boiling point of the solvent to be used for 10 minutes to 48 hours.

The base, the phase-transfer catalyst and the inert solvent are as defined in the above-mentioned Method 1, Step 1.

The above-mentioned compound (106) or a salt thereof may be prepared, for example, by the following method.

### Method 3

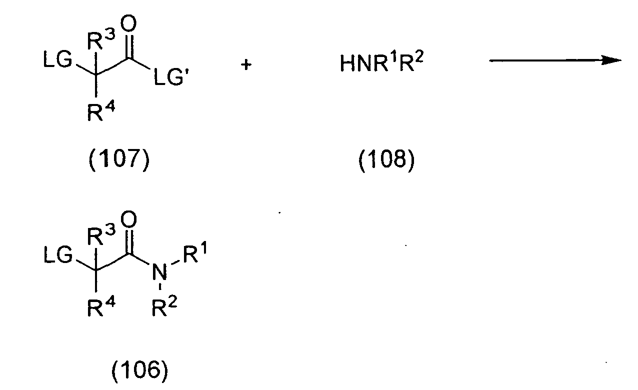

(wherein R¹, R², R³ and R⁴ are defined above, LG is as defined in Method 1, and LG' is a leaving group (e.g., a halogen atom such as chlorine atom or bromine atom), where LG' is different from LG, but is a preferably more reactive leaving group than LG)

The compound (107) or a salt thereof is reacted with the compound (108) or a salt thereof to give the compound (106). The reaction is carried out in the presence of a base, if necessary, in a suitable inert solvent at a temperature of from -20°C to a boiling point of the solvent to be used for 10 minutes to 48 hours.

The base and the inert solvent may be ones as exemplified in the above-mentioned Method 1, Step 1.

The present compound (3) or a pharmaceutically acceptable salt thereof may be prepared, for example, by the following method.

### Method 4

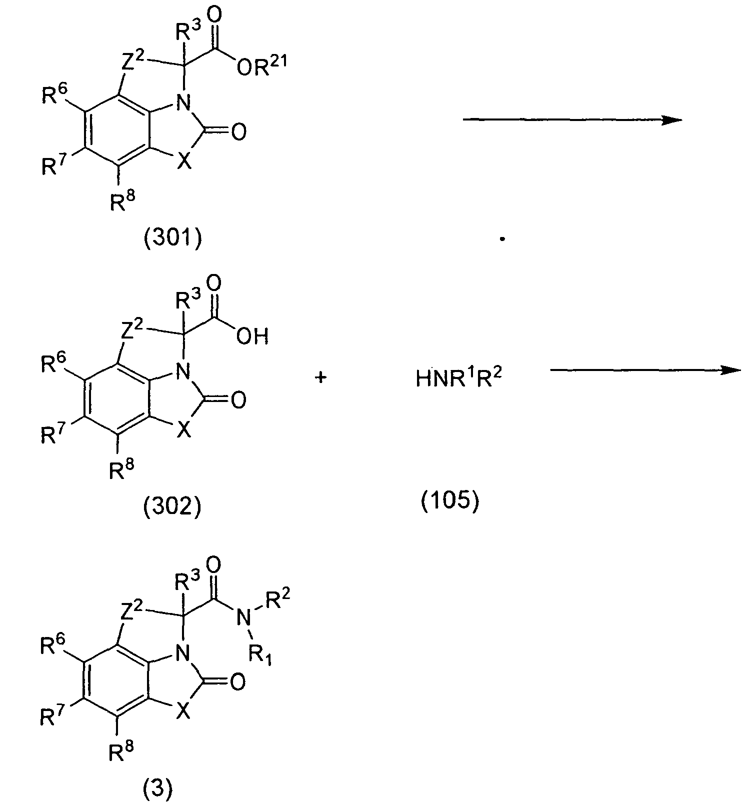

(wherein R¹, R², R³, R⁶, R⁷, R⁸, X and Z² are as defined above, and R²¹ is an alkyl group such as methyl, ethyl and t-butyl)

### Step 1 (hydrolysis)

The compound (301) is hydrolyzed to give the intermediate (302). The reaction is carried out in a suitable solvent under acidic or basic conditions at a temperature of from about 0°C to a boiling point of the solvent to be used for 10 minutes to 48 hours. The solvent, the acid and the base are as defined in the above Method 1, Step 2.

### Step 2 (condensation)

The intermediate (302) or a salt thereof is reacted with the compound (105) or a salt thereof for amide bond formation to give the compound (3). The amide bond formation reaction may be carried out by a conventional method as exemplified in Method 1, Step 3.

### Method 5

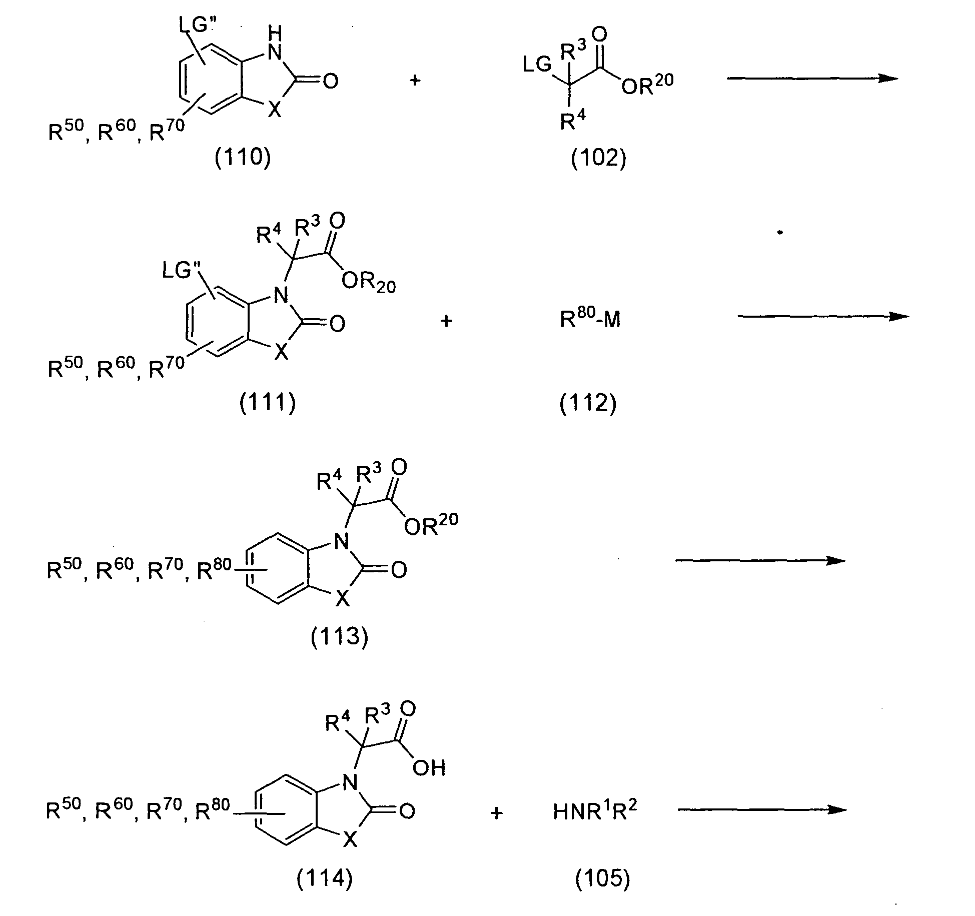

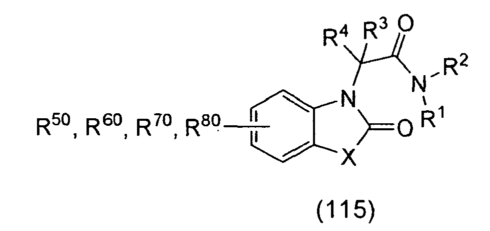

(wherein R¹, R², R³, R⁴ and X are as defined above, R⁵⁰, R⁶⁰, R⁷⁰, R⁸⁰ are the same as defined for R⁵, R⁶, R⁷, R⁸, respectively, and LG and R²⁰ are as defined in Method 1, LG" is chlorine atom, bromine atom, iodine atom or trifluoromethanesulfonyloxy, etc., M is trimethyltin, triethyltin, tributyltin, catecholborane, B(OR²²)₂ (in which R²² is a hydrogen atom, methyl, ethyl or isopropyl), or a group of the following formula (116): 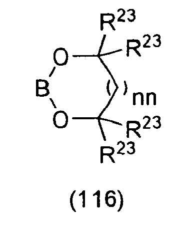 (in which R²³ is a hydrogen atom or methyl, and nn is an integer of 0 or 1)).

### Step 1 (alkylation)

The compound (110) or a salt thereof is reacted with the compound (102) or a salt thereof to give the intermediate (111). This alkylation reaction is carried out in a similar manner to Method 1, Step 1.

### Step 2 (coupling reaction)

The intermediate (111) is reacted with the compound (112) in an amount of 1 to 3 equivalents, preferably in an amount of 1 to 1.5 equivalent in a suitable inert solvent at a temperature of from 20°C to 150°C, preferably at a temperature of from 50°C to 120°C in the presence of a palladium catalyst and a base to give the intermediate (113).

The palladium catalyst includes, for example, palladium on carbon, palladium hydroxide, palladium (II) acetate, tetrakistriphenylphosphine palladium (0), tris(dibenzylideneacetone)dipalladium (0), bis(triphenylphosphine)palladium (II) chloride, 1,1'-bis(diphenylphosphino)ferrocene palladium (II) chloride, etc. The preferable catalyst is tetrakistriphenylphosphine palladium (0).

The base includes, for example, an organic base such as triethylamine or pyridine, etc., an inorganic base such as potassium carbonate, sodium hydroxide or sodium hydride, etc., and a metal alkoxide such as sodium methoxide or potassium tert-butoxide, etc.

The inert solvent includes, for example, acetonitrile, halogenated hydrocarbons such as chloroform or dichloromethane, aromatic hydrocarbons such as benzene, toluene, etc., ethers such as diethyl ether, tetrahydrofuran or 1,4-dioxane, etc., alcohols such as methanol, ethanol or 2-propanol, etc., and aprotic polar solvents such as N,N-dimethylformamide, N-methylpyrrolidone or dimethyl sulfoxide, etc., and a mixed solvent of these solvents. The preferable solvent is ethers.

### Step 3 (hydrolysis)

The intermediate (113) is hydrolyzed to give the intermediate (114). The reaction is carried out in a similar manner to Method 1, Step 2.

### Step 4 (condensation)

The intermediate (114) or a salt thereof is reacted with the compound (105) or a salt thereof for amide bond formation to give the compound (115). This amide bond formation reation is carried out in a similar manner to Method 1, Step 3.

### Method 6

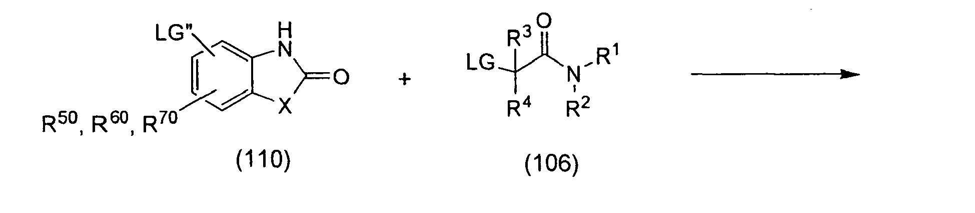

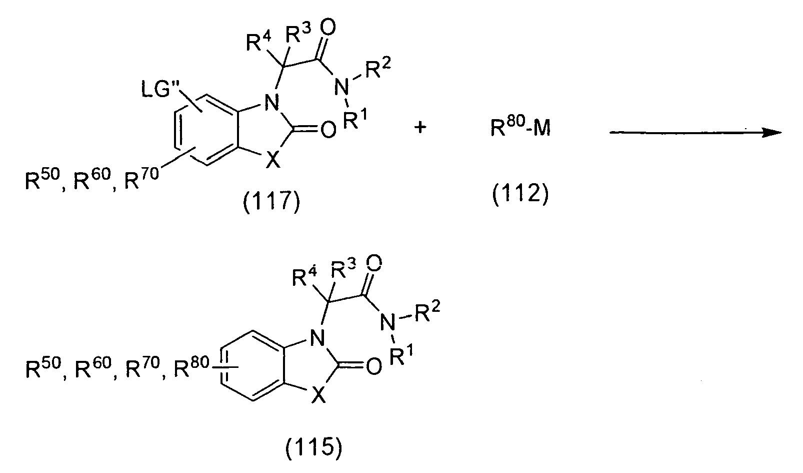

(wherein R¹, R², R³, R⁴ and X are as defined above, R⁵⁰, R⁶⁰, R⁷⁰, R⁸⁰ are the same as R⁵, R⁶, R⁷, R⁸, respectively, LG and R²⁰ are as defined in Method 1, and LG" and M are as defined in Method 5)

### Step 1 (alkylation)

The compound (110) or a salt thereof is reacted with the compound (106) or a salt thereof to give the compound (117). This alkylation reaction is carried out in a similar manner to Method 1, Step 1.

### Step 2 (coupling reaction)

The compound (117) is reacted with the compound (112) in an amount of 1 to 3 equivalents, preferably in an amount of 1 to 1.5 equivalent, in the presence of a palladium catalyst and a base in a suitable inert solvent at a temperature of from 20°C to 150°C, preferably at a temperature of from 50°C to 120°C, to give the compound (115). The palladium catalyst, the base and the inert solvent are ones as exemplified in the above Method 5, Step 2.

### Method 7

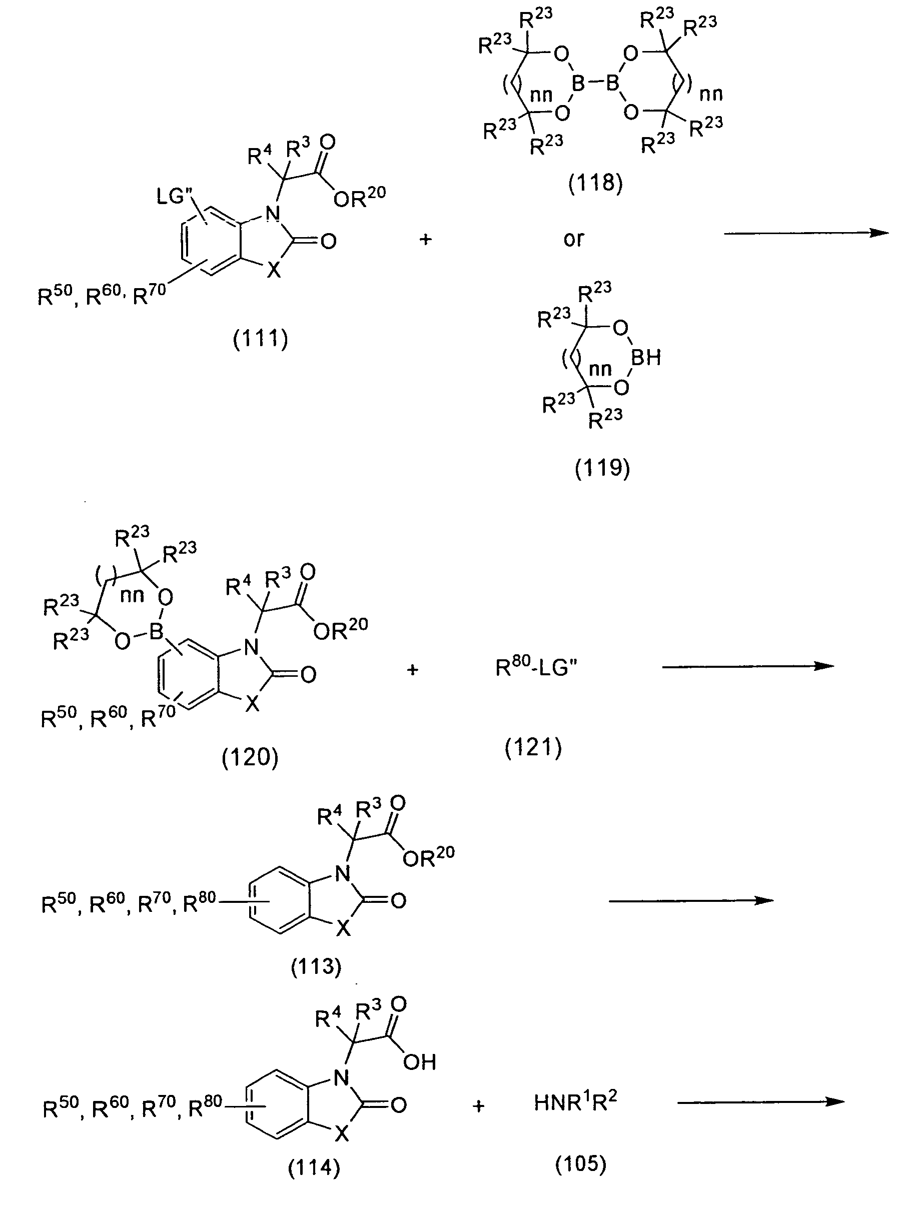

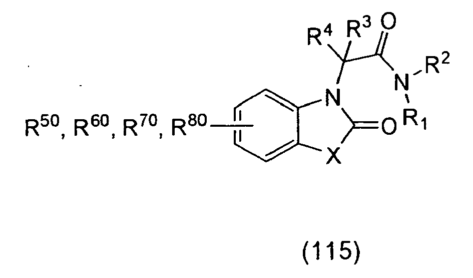

(wherein R¹, R², R³, R⁴ and X are as defined above, R⁵⁰, R⁶⁰ R⁷⁰ R⁸⁰ are the same as R⁵, R⁶, R⁷, R⁸, respectively, R²⁰ is the same as defined in Method 1, and LG", R²³ and nn are the same as defined in Method 5)

### Step 1 (metalation reaction)

The compound (111), which is obtained from the compound (110) by the reaction procedure as disclosed in the above Method 5, Step 1, is reacted with the compound (118) or the compound (119) in an amount of 1 to 3 equivalents, preferably in an amount of 1 to 1.5 equivalent in a suitable inert solvent at a temperature of from 20°C to 150°C, preferably at a temperature of from 50°C to 120°C, in the presence of a palladium catalyst, a base or a phosphine ligand, if applicable, to give the intermediate (120).

The palladium catalyst includes, for example, palladium (II) acetate, tetrakistriphenylphosphine palladium (0), tris(dibenzilidenacetone)dipalladium (0), 1,1'-bis(diphenylphosphino)ferrocene palladium (II) chloride, etc.

The base includes, for example, an organic base such as triethylamine or pyridine, an inorganic base such as potassium carbonate, sodium hydroxide or sodium hydride, and a metal alkoxide such as sodium methoxide or potassium tert-butoxide.

The phosphine ligand includes, for example, tri-tert-butylphosphine, tricyclohexylphosphine, 2-(dicyclohexylphosphino)-2'-(N,N-dimethylamino)biphenyl, 2-(di-tert-butylphosphino)biphenyl, etc.

The inert solvent includes, for example, acetonitrile, halogenated hydrocarbons such as chloroform, dichloromethane, etc., aromatic hydrocarbons such as benzene, toluene, etc., ethers such as diethyl ether, tetrahydrofuran, 1,4-dioxane, etc., alcohols such as methanol, ethanol or 2-propanol, etc., and aprotic polar solvents such as N,N-dimethylformamide, N-methylpyrrolidone or dimethylsulfoxide, etc., and a mixed solvent of these solvents. The preferable solvent is ethers.

### Step 2 (coupling reaction)

The intermediate (120) is reacted with the compound (121) in an amount of 1 to 3 equivalents, preferably in an amount of 1 to 1.5 equivalent, in a suitable inert solvent at a temperature of from 20°C to 150°C, preferably at a temperature of from 50°C to 120°C in the presence of a palladium catalyst and a base to give the intermediate (113). The palladium catalyst, the base and the inert solvent are as defined in the above Method 5, Step 2.

### Step 3 (hydrolysis)

The intermediate (113) is hydrolyzed to give the intermediate (114). The reaction is carried out in a similar manner to Method 1, Step 2.

### Step 4 (condensation reaction)

The intermediate (114) or a salt thereof is reacted with the compound (105) or a salt thereof for amide bond formation to give the compound (115). This amide bond formation reaction is carried out in a similar manner to Method 1, Step 3.

### Method 8

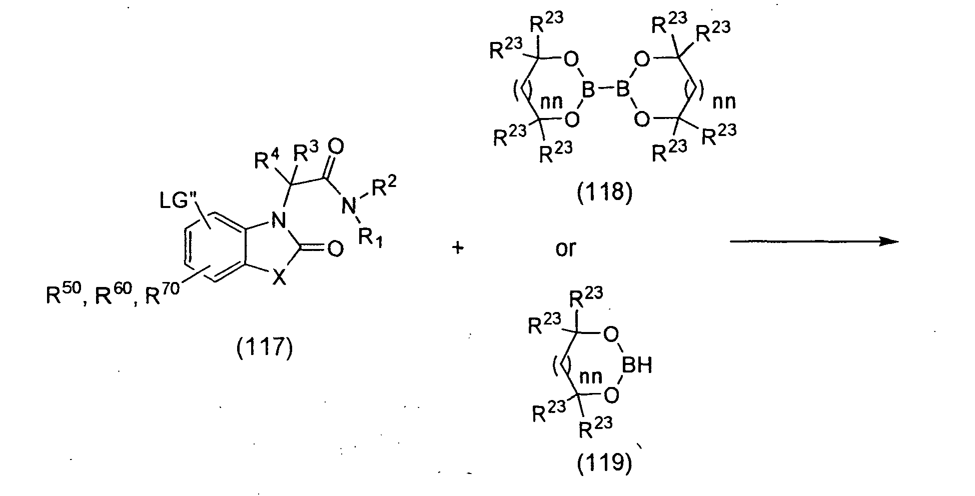

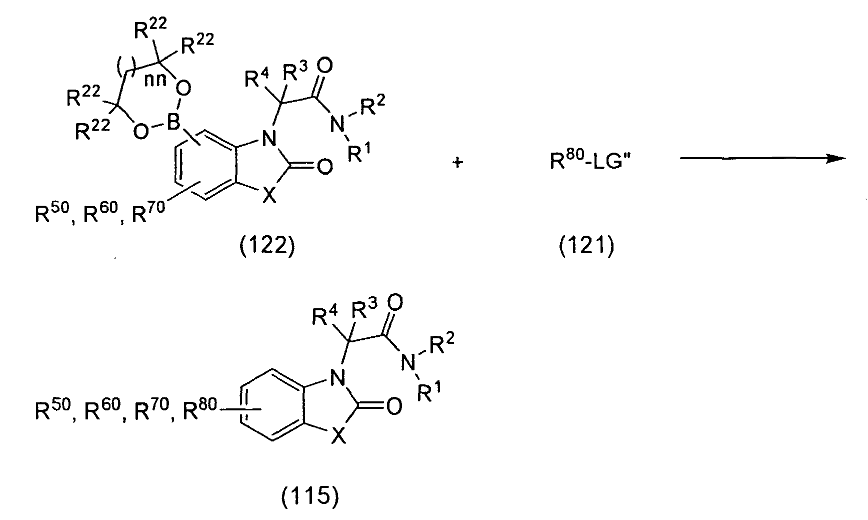

(wherein R¹, R², R³, R⁴ and X are as defined above, R⁵⁰, R⁶⁰, R⁷⁰ R⁸⁰ are the same as defined for R⁵, R⁶, R⁷, R⁸, respectively, and LG", R²³ and nn are as defined in Method 5)

### Step 1 (metalation reaction)

The compound (117), which is obtained from the compound (110) by the same reaction procedure as defined in the above Method 6, Step 1, is reacted with the compound (118) or the compound (119) in an amount of 1 to 3 equivalents, preferably in an amount of 1 to 1.5 equivalent, at a temperature of from 20°C to 150°C, preferably at a temperature of from 50°C to 120°C in a suitable inert solvent in the presence of a palladium catalyst, a base, or a phosphine ligand, if applicable, to give the intermediate (122). The palladium catalyst, the base, the phosphine ligand and the inert solvent are the same as defined in the above Method 7, Step 1.

### Step 2 (coupling reaction)

The intermediate (122) is reacted with the compound (121) in an amount of 1 to 3 equivalents, preferably in an amount of 1 to 1.5 equivalent in a suitable inert solvent at a temperature of from 20°C to 150°C, preferably at a temperature of from 50°C to 120°C in the presence of a palladium catalyst and a base to give the compound (115). The palladium catalyst, the base and the inert solvent are the same as defined in the above Method 5, Step 2.

### Method for preparing the intermediates

Among the compounds (101), the compound where X is NR¹⁰, R⁸ and R¹⁰ combine to form a group of the formula (201): 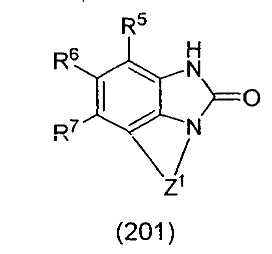 (in which R⁵, R⁶, R⁷ and Z¹ are as defined above), and the compound (301) may be prepared by the method disclosed in the literatures (J. Org. Chem., (1997), 62, 6582-6587 and J. Med. Chem., (1997), 40, 639-646) or a modified method thereof. In addition, among the compounds (101), the compound where X is an oxygen atom may be prepared by the method disclosed in the literature (J. Heterocyclic Chem., (1991), 28, 933-937) or a modified method thereof, and the compound where X is a sulfur atom may be prepared by the method disclosed in the literature (J. Heterocyclic Chem., (1988), 25, 1183-1190) or a modified method thereof, the compound where X is NR¹⁰ may be prepared by the method disclosed in the literature (Synthesis, (2001), 541-543) or a modified method thereof, and the compound where X is CR¹¹R¹² may be prepared by the method disclosed in the literatures (Tetrahedron Lett., (1979), 20, 2857-2860 and Tetrahedron Lett., (2002), 43, 193-195) or a modified method thereof.

Further, the compounds of the above formula (1) can be converted into another compound of the formula (1) by suitably exchanging the function groups thereof. The conversion of the function group is carried out by a conventional common method (e.g., see Comprehensive Organic Transformations, R. C. Larock, (1989), etc.).

Throughout the present specification, the protecting groups and the condensation agents may be expressed by the general designations named by IUPAC-IUB (Biochemical Nomenclature Committee), which are widely used in this technical field.

Suitable salts and pharmaceutically acceptable salts of the starting compounds and the desired compounds are the conventional non-toxic salts, and can be selected by a skilled person in this art, for example, from an acid addition salt such as salts with an organic acid (e.g., acetate, trifluoroacetate, maleate, fumarate, citrate, tartrate, methanesulfonate, benzenesulfonate, formate or toluenesulfonate, etc.) and salts with an inorganic acid (e.g., hydrochloride, hydrobromide, hydroiodide, sulfate, nitrate or phosphate, etc.), a salt with an amino acid (e.g., arginine, aspartic acid, or glutamic acid), an alkali metal salt (e.g., sodium salt or potassium salt) and an alkaline earth metal salt (e.g., calcium salt or magnesium salt), ammonium salt, or salt with an organic base (e.g., trimethylamine salt, triethylamine salt, pyridine salt, picoline salt, dicyclohexylamine salt or N,N'-dibenzylethylenediamine salt, etc.).

In the above-mentioned methods, when any functions groups other than the reaction site are reacted under the prescribed conditions or are not suitable for those methods, then these functions groups other than the reaction site are previously protected and de-protected after the reaction to give the desired compounds. The protecting group may be, for example, conventional protecting groups as disclosed in the literature (e.g., Protective Groups in Organic Synthesis, T. W. Greene, John Wiley & Sons Inc., (1981), etc.), and more particularly, the protecting group for amine is ethoxycarbonyl, tert-butoxycarbonyl, acetyl or benzyl, etc., and the protecting group for a hydroxy group is a trialkylsilyl, acetyl or benzyl, etc.

The introduction or removal of the protecting group is carried out by a conventional method widely employed in the organic synthetic chemistry field (for example, see the above-mentioned Protective Groups in Organic Synthesis) or a modified method thereof.

The intermediates and the desired compounds in the above Methods can be isolated and purified by a conventional purification method which is usually employed in the organic synthesis chemistry field, such as neutralization, filtration, extraction, washing, drying, concentration, recrystallization, various chromatographies, etc. In addition, the intermediates may be used in a subsequent reaction without specifically purified.

Among the present compounds (1), some may have tautomers, and the present invention also includes these tautomers, and all other possible isomers and a mixture thereof.

When a pharmaceutically acceptable salt of the present compound (1) is needed, and the compound (1) is obtained in the form of a pharmaceutically acceptable salt thereof, then the obtained pharmaceutically acceptable salt of the compound (1) is purified as it stands. When the compound (1) is obtained in the free form, then the obtained free compound (1) is dissolved or suspended in a suitable organic solvent and converted into a salt thereof by adding an acid or a base thereto. Further, the compound (1) and a pharmaceutically acceptable salt thereof may exist in the form of a hydrate or an adduct with various solvents, and these adducts are also included in the scope of the present invention. The present compound (1) may have one or more stereoisomers based on an asymmetric carbon atom, and all of these isomers and a mixture thereof may be included in the scope of the present invention.

The prodrug of the present compound (1) can be included in the scope of the present invention. In the present invention, the prodrug includes a compound which can easily be acid-hydrolyzed or enzymatically degraded in the living body, and can produce the compound of the above formula (1). For example, when the compound of the above formula (1) has a hydroxy group or an amino group, or a carboxyl group, then these groups may be modified by a conventional method to give a prodrug of the compound (1).

For example, the compound (1) has a carboxyl group, then the prodrug thereof is compounds wherein said carboxyl group can be replaced by an alkoxycarbonyl group, an alkylthiocarbonyl group, or an alkylaminocarbonyl group.

In addition, when the compound (1) has an amino group, then the prodrug thereof is compounds wherein said amino group is substituted by an alkanoyl group to form an alkanoylamino group, or substituted by an alkoxycarbonyl group to form an alkoxycarbonylamino group, or converted into an acyloxymethylamino group or a hydroxylamine.

When the compound of the formula (1) has a hydroxy group, the prodrug thereof is, for example, compounds wherein said hydroxy group is substituted by an acyl group as mentioned above and converted into an acyloxy group, or converted into a phosphate ester, or converted into an acyloxymethyloxy group.

The alkyl moiety of groups being used for making a prodrug may be the above-mentioned alkyl groups, and said alkyl group may optionally be substituted, for example, by an alkoxy group, etc. The preferable examples of the alkyl moiety are as follows.

For example, with regard to the compounds wherein a carboxyl group is converted into an alkoxycarbonyl group, an alkoxycarbonyl group such as methoxycarbonyl, ethoxycarbonyl, or an alkoxycarbonyl group being substituted by an alkoxy group such as methoxymethoxycarbonyl, ethoxymethoxycarbonyl, 2-methoxyethoxycarbonyl, 2-methoxyethoxymethoxycarbonyl, or pivaloyloxymethoxycarbonyl are exemplified.

The present compound exhibits a benzodiazepine ω3 receptor agonistic activity, and hence, the present compound is useful in the treatment or prophylaxis of central nervous diseases such as anxiety disorders and related diseases thereof, depression, cognitive dysfunction or convulsion.

For the clinical use, the present compounds can be formulated into a pharmaceutical preparation as a mixture with a pharmaceutically acceptable excipient such as solid or liquid organic or inorganic excipient being suitable for oral, parenteral or external administration, including local, enteral, intravenous, intramuscular, inhalation, nasal, intra-articular, intrathecal, transtracheal, or transocular administrations. The pharmaceutical preparations include solid, semisolid or liquid preparations, for example, capsules, tablets, pellets, sugar-coated tablets, powders, granules, suppositories, ointments, creams, lotions, inhalants, injections, cataplasms, gels, tapes, eye-drops, solutions, syrups, aerosols, suspensions, emulsions, etc., and these preparations can be formulated by a conventional method. If necessary, auxiliary agents, stabilizers, wetting agents or emulsifying agents, buffering agents or other conventional additives may be added to these pharmaceutical preparations.

The dosage of the present compound may vary according to the ages and conditions of the patients, but 0.1 mg, 1 mg, 10 mg, 50 mg, 100 mg, 250 mg, 500 mg and 1,000 mg of the compound (1) contained in an average dosage unit are effective to the central nervous diseases such as anxiety disease and related disease thereof, depression, cognitive dysfunction and convulsion. When the present compound is administered to human, it is usually administered at a dose of 0.1 mg/person to about 1,000 mg/person per day, preferably at a dose of 1 mg/person to about 100 mg/person per day.

The present invention is illustrated in more detail by the following Examples and Experiments, but should not be construed to be limited thereto. In the present specification, the following abbreviations are used for simplifying the description.
- Me:: Methyl
- Et:: Ethyl
- Pr:: Propyl
- i-Pr:: Isopropyl
- t-Bu:: tert-Butyl
- Ph:: Phenyl
- Py:: Pyridyl
- Bn:: Benzyl
- Boc:: tert-Butoxycarbonyl

### Reference Example 1

### 2-Amino-4-bromophenol

To a solution of 4-bromo-2-nitrophenol (25.0 g, 115 mmol) in tetrahydrofuran (250 mL) is added a 5 % rhodium carbon (2.20 g), and the mixture is stirred at 20-25°C for 4.5 hours under hydrogen atmosphere. After the reaction, the rhodium carbon is filtered off, and the solvent is evaporated under reduced pressure to give 2-amino-4-bromophenol (21.6 g, 98 %).

IR (cm⁻¹): 1200, 1279, 1437, 1497, 2791

### Reference Example 2

### 5-Bromo-1,3-benzoxazol-2(3H)-one

To a solution of 2-amino-4-bromophenol (3.50 g, 18.6 mmol) in tetrahydrofuran (100 mL) is added 1,1'-carbonyldiimidazole (3.62 g, 22.3 mmol) at 20-25°C, and the mixture is refluxed for 1.5 hour. After the reaction, the reaction solution is cooled to 20-25°C, and thereto is added a 2N aqueous hydrochloric acid solution, and the mixture is extracted with ethyl acetate. The resulting organic layer is washed with a saturated aqueous sodium hydrogen carbonate solution and a saturated saline solution, and dried over anhydrous sodium sulfate. The resultant is filtered, and the solvent is evaporated under reduced pressure to give 5-bromo-1,3-benzoxazol-2(3H)-one (3.89 g, quantitative).

IR (cm⁻¹): 960, 1149, 1474, 1622, 1751

### Reference Example 3

### tert-Butyl (5-bromo-2-oxo-1,3-benzoxazol-3(2H)-yl)acetate

To a solution of 5-bromo-1,3-benzoxazol-2(3H)-one (47.8 g, 223 mmol) in acetone (400 mL)/dimethylformamide (40 mL) are added potassium carbonate (3.28 g, 23.7 mmol), tert-butyl bromoacetate (36.3 mL, 246 mmol) at 20-25°C, and the mixture is stirred at 20-25°C for 3 hours. After filtration, the solvent is evaporated under reduced pressure, and the obtained residue is washed with hexane to give tert-butyl (5-bromo-2-oxo-1,3-benzoxazol-3(2H)-yl)acetate (71.5 g, 98 %).

IR (cm⁻¹): 1151, 1242, 1485, 1736, 1782

### Reference Example 4

### (5-Bromo-2-oxo-1,3-benzoxazol-3(2H)-yl)acetic acid

To a solution of tert-butyl (5-bromo-2-oxo-1,3-benzoxazol-3(2H)-yl)acetate (71.5 g, 218 mmol) in 1,4-dioxane (360 mL) are added a 4N hydrochloric acid/ 1,4-dioxane solution (340 mL, 1.36 mmol) and acetic acid (360 mL) at 20-25°C, and the mixture is stirred at 50°C for 4.5 hours. After the reaction, water is added to the mixture, and the mixture is extracted with ethyl acetate. The organic layer is washed with a saturated saline solution, dried over anhydrous sodium sulfate, and filtered. The solvent is evaporated under reduced pressure and the resulting residue is washed with hexane to give (5-bromo-2-oxo-1,3-benzoxazol-3(2H)-yl)acetic acid (58.3 g, 98 %).

IR (cm⁻¹): 1028, 1227, 1483, 1736, 2953

### Reference Example 5

### (2-Oxo-5-phenyl-1,3-benzoxazol-3(2H)-yl)acetic acid

(2-Oxo-5-phenyl-1,3-benzoxazol-3(2H)-yll)acetic acid is obtained from 2-amino-4-phenylphenol as a starting compound in a similar manner to Reference Examples 2 to 4.

IR (cm⁻¹): 1030, 1241, 1483, 1728, 1763

### Reference Example 6

### (5-Nitro-2-oxo-1,3-benzoxazol-3(2H)-yl)acetic acid

(5-Nitro-2-oxo-1,3-benzoxazol-3(2H)-yl)acetic acid is obtained from 2-amino-4-nitrophenol as a starting compound in a similar manner to Reference Examples 2-4.

IR (cm⁻¹): 1020, 1252, 1487, 1728, 1782

### Reference Example 7

### [2-Oxo-5-(3-thienyl)-1,3-benzoxazol-3(2H)-yl]acetic acid

To a solution of the compound synthesized in Reference Example 3 (328 mg, 1 mmol) in 1,4-dioxane (7.5mL) are added 3-thiophene boronic acid (154 mg, 1.20 mmol), tetrakistriphenylphosphine palladium (35.0 mg, 30.0 µmol), and an aqueous solution (1.5 mL) of potassium carbonate (415 mg, 3.00 mmol) at 20-25°C, and the mixture is purged with nitrogen gas, and stirred at 120°C for 3 hours. After the reaction, water is added to the mixture, and the mixture is extracted with ethyl acetate. The organic layer is washed with a saturated saline solution, dried over anhydrous sodium sulfate, and filtered. The solvent is evaporated under reduced pressure to give crude tert-butyl [2-oxo-5-(3-thienyl)-1,3-benzoxazol-3(2H)-yl]acetate. Subsequently, the same procedures as in Reference Example 4 are repeated to give [2-oxo-5-(3-thienyl)-1,3-benzoxazol-3(2H)-yl]acetic acid.

IR(cm⁻¹): 1030, 1250, 1491, 1724, 1782

### Reference Example 8

### (5-Bromo-2-oxo-1,3-benzothiazol-3(2H)-yl)acetic acid

The title compound is obtained according to the literature (WO 97/43282).

IR (cm⁻¹): 1182, 1342, 1437, 1635, 1743

### Reference Example 9

### (7-Bromo-2-oxo-1,3-benzothiazol-3(2H)-yl)acetic acid

According to the literature (WO 97/43282), the tile compound is synthesized in a similar manner to Reference Example 8.

IR (cm⁻¹): 1109, 1234, 1570, 1684, 1733

### Reference Example 10

### (6-Bromo-3-methyl-2-oxo-2,3-dihydro-1H-benzimidazol-1-yl)acetic acid

To a solution of ethyl 6-bromo-2-oxo-2,3-dihydro-1H-benzimidazole-1-carboxylate (2.85 g, 10.0 mmol) obtained by the method disclosed in the literature (J. Org. Chem., (1995), 60, 1565-1582), methanol (962 mg, 30.0 mmol) and triphenylphosphine (3.93 g, 15.0 mmol) in tetrahydrofuran (60 mL) is added diethyl azodicarboxylate (40 % toluene solution, 6.53 g, 15.0 mmol), and the mixture is stirred at 20-25°C for 7 hours. The reaction solution is concentrated under reduced pressure, and the residue is purified by silica gel column chromatography (hexane/ethyl acetate = 5/ 1 to 2/1) to give a mixture of ethyl 6-bromo-3-methyl-2-oxo-2,3-dihydro-1H-benzimidazole-1-carboxylate and an impurity derived from diethyl azodicarboxylate. To this mixture is added toluene (25mL), and the mixture is stirred, and the precipitated crystals are collected by filtration to give crude ethyl 6-bromo-3-methyl-2-oxo-2,3-dihydro-1H-benzimidazole-1-carboxylate.

Subsequently, to a suspension of ethyl 6-bromo-3-methyl-2-oxo-2,3-dihydro-1H-benzimidazole-1-carboxylate (2.88 g, 9.63 mmol) in methanol (70 mL) is added a 5N aqueous sodium hydroxide solution (4 mL), and the mixture is stirred at 20-25°C for 30 minutes. The reaction solution is concentrated under reduced pressure, and water (50 mL) is added to the residue, and thereto is added a 4N aqueous hydrochloric acid solution until the pH value of the mixture becomes pH 1. The resulting suspension is stirred for 30 minutes, and filtered. The product remained on the filter is dried under reduced pressure at 50°C. The obtained solid is suspended in toluene (25 mL), and heated to 110°C, and further gradually cooled to 20-25°C. The insoluble product is collected by filtration, and dried under reduced pressure to give 5-bromo-1-methyl-1,3-dihydro-2H-benzimidazol-2-one (1.29 g).

5-Bromo-1-methyl-1,3-dihydro-2H-benzimidazol-2-one is treated in a similar manner to Reference Examples 3, 4 to give (6-bromo-3-methyl-2-oxo-2,3-dihydro-1H-benzimidazol-1-yl)acetic acid.

¹H-NMR (400 MHz, DMSO-d₆): δ 3.32 (s, 3H), 4.62 (s, 2H), 7.13 (d, 1H, J=8.3Hz), 7.24 (dd, 1H, J=8.3, 1.8Hz), 7.48 (d, 1H, J=1.8Hz), 13.12 (br, 1H).

### Reference Example 11

### (5-Bromo-3-methyl-2-oxo-2,3-dihydro-1H-benzimidazol-1-yl)acetic acid

By the method disclosed in the literature (J. Org. Chem., (1995), 60, 1565-1582), tert-butyl 5-bromo-2-oxo-2,3-dihydro-1H-benzimidazole-1-carboxylate is synthesized, which is further methylated in a similar manner to Reference Example 10 to give tert-butyl 5-bromo-3-methyl-2-oxo-2,3-dihydro-1H-benzimidazole-1-carboxylate.

Subsequently, to a solution of tert-butyl 5-bromo-3-methyl-2-oxo-2,3-dihydro-1H-benzimidazole-1-carboxylate (1.65 g, 5.04 mmol) in acetic acid (5 mL) is added a 4N solution of hydrochloric acid in 1,4-dioxane solution (5 mL, 20.0 mmol), and the mixture is stirred at 20-25°C for one hour. The reaction solution is concentrated under reduced pressure, and thereto is added toluene. The mixture is evaporated under reduced pressure again to give 6-bromo-1-methyl-1,3-dihydro-2H-benzimidazol-2-one (1.16 g).

Further, the same procedures in Reference Examples 3, 4 are repeated to give (5-bromo-3-methyl-2-oxo-2,3-dihydro-1H-benzimidazol-1-yl)acetic acid.

¹H-NMR (400 MHz, DMSO-d₆): δ 3.33 (s, 3H), 4.60 (s, 2H), 7.13 (d, 1H, J=8.3Hz), 7.21 (dd, 1H, J=8.3, 1.9Hz), 7.44 (d, 1H, J=1.8Hz), 13.14 (br, 1H).

### Reference Example 12

### Methyl 3,4-dihydroquinoline-1(2H)-carboxylate

To a solution of tetrahydroquinoline (18.0 mL, 143 mmol) in N,N-dimethylformamide (100 mL) is added potassium carbonate (79.3 g, 574 mmol) at 20-25°C, and thereto is added dropwise methyl chloroformate (33.2 mL, 430 mmol) at 0°C, and the mixture is stirred at 50°C for 6 hours. After the reaction, the mixture is cooled to 20-25°C, and water is added thereto, and the mixture is extracted with ethyl acetate/toluene (1/ 1). The organic layer is washed with water, a 2N aqueous hydrochloric acid solution, a saturated aqueous sodium hydrogen carbonate solution, and a saturated saline solution, and dried over anhydrous sodium sulfate. The resultant is filtered, and the solvent is evaporated under reduced pressure to give methyl 3,4-dihydroquinoline-1(2H)-carboxylate (27.2 g, 99 %).

IR (cm⁻¹): 1036, 1134, 1327, 1493, 1701

### Reference Example 13

### Methyl 6-bromo-3,4-dihydroquinoline-1 (2H)-carboxylate

To a solution of methyl 3,4-dihydroquinoline-1(2H)-carboxylate (4.50 g, 23.5 mmol) in N,N-dimethylformamide (20 mL) is added N-bromosuccinimide at 0°C, and the mixture is stirred at 20-25°C for 3 hours. After the reaction, water is added to the mixture, and the mixture is extracted with ethyl acetate. The organic layer is washed with water and a saturated saline solution, and dried over anhydrous sodium sulfate. The mixture is filtered, and the solvent is evaporated under reduced pressure to give methyl 6-bromo-3,4-dihydroquinoline-1(2H)-carboxylate (6.06 g, 95 %).

IR (cm⁻¹): 1038, 1130, 1321, 1441, 1701

### Reference Example 14

### Methyl 6-bromo-8-nitro-3,4-dihydroquinoline-1(2H)-carboxylate

To a solution of nitronium tetrafluoroborate (4.06 g, 30.6 mmol) in acetonitrile (100 mL) is added a solution of methyl 6-bromo-3,4-dihydroquinoline-1(2H)-carboxylate (5.90 g, 21.8 mmol) in acetonitrile (100 mL) at 0°C, and the mixture is stirred at the same temperature for 10 minutes. After the reaction, water is added to the mixture at 0°C, and the mixture is extracted with ethyl acetate. The organic layer is washed with a saturated aqueous sodium hydrogen carbonate solution and a saturated saline solution, and dried over anhydrous sodium sulfate. The resultant is filtered, and the solvent is evaporated under reduced pressure to give methyl 6-bromo-8-nitro-3,4-dihydroquinoline-1(2H)-carboxylate (7.19 g, quantitative).

IR (cm⁻¹): 810, 1174, 1321, 1439, 1701

### Reference Example 15

### 8-Bromo-5,6-dihydro-4H-imidazo[4,5,1-ij]quinolin-2(1H)-one

A solution of reduced iron (24.1 g, 431 mmol) in acetic acid (250 mL) is heated to about 70°C, and thereto is added dropwise a solution of methyl 6-bromo-8-nitro-3,4-dihydroquinoline-1(2H)-carboxylate (19.4 g, 61.6 mmol) in acetic acid (200 mL) over a period of one hour, and the mixture is stirred at about 80°C for 2 hours. After the reaction, the mixture is cooled to 20-25°C, and thereto are added cerite (10 g) and ethyl acetate (200 mL). The mixture is stirred for 30 minutes, and filtered through cerite. To the obtained filtrate is added a 1N aqueous hydrochloric acid solution (500 mL), and the mixture is stirred at 20-25°C for 30 minutes, and extracted with ethyl acetate. The organic layer is washed with water (twice), and a saturated saline solution, and dried over anhydrous sodium sulfate. The resultant is filtered, and the solvent is evaporated under reduced pressure to give crude 8-bromo-5,6-dihydro-4H-imidazo[4,5,1-ij]quinolin-2(1H)-one (14.6 g, 94 %).

IR (cm⁻¹): 1144, 1491, 1657, 1707, 3143

### Reference Example 16

### tert-Butyl (8-bromo-2-oxo-5,6-dihydro-4H-imidazo[4,5,1-ij]quinolin-1(2H)-yl)acetate

8-Bromo-5,6-dihydro-4H-imidazo[4,5,1-ij]quinolin-2(1H)-one is treated in a similar manner to Reference Example 3 to give tert-butyl (8-bromo-2-oxo-5,6-dihydro-4H-imidazo[4,5,1-ij]quinolin-1(2H)-yl)acetate.

IR (cm⁻¹): 1153, 1421, 1498, 1697, 1741

### Reference Example 17

### (8-Bromo-2-oxo-5,6-dihydro-4H-imidazo[4,5,1-ij]quinolin-1(2H)-yl)acetic acid

tert-Butyl (8-bromo-2-oxo-5,6-dihydro-4H-imidazo[4,5,1-ij]-quinolin-1(2H)-yl)acetate is treated in a similar manner to Reference Example 4 to give (8-bromo-2-oxo-5,6-dihydro-4H-imidazo[4,5,1-ij]-quinolin-1(2H)-yl)acetic acid.

IR (cm⁻¹): 980, 1217, 1240, 1624, 1718

### Reference Example 18

### (2-Oxo-8-phenyl-5,6-dihydro-4H-imidazo[4,5,1-ij]quinolin-1 (2H)-yl)-acetic acid

The compound synthesized in Reference Example 16 and phenylboric acid are treated in a similar manner to Reference Example 7 to give tert-butyl (2-oxo-8-phenyl-5,6-dihydro-4H-imidazo[4,5,1-ij]-quinolin-1 (2H)-yl)acetate.

Subsequently, (2-oxo-8-phenyl-5,6-dihydro-4H-imidazo-quinolin-1(2H)-yl)acetic acid is obtained in a similar manner to Reference Example 4.

IR (cm⁻¹): 1111, 1223, 1429, 1643, 1728

### Reference Example 19

### Ethyl 2-(8-bromo-2-oxo-5,6-dihydro-4H-imidazo[4,5,1-ij]quinolin-1(2H)-yl)propanoate

To a solution of the compound obtained in Reference Example 15 (1.00 g, 3.95 mmol) in N,N-dimethylformamide (10.0 ml) are added successively potassium carbonate (819 mg, 5.93 mmol), ethyl 2-bromo-propanoate (616 µL, 4.74 mmol), and the mixture is stirred at 50°C for 1.5 hour. After the reaction, the reaction solution is cooled to 20-25°C, and the mixture is poured into a 1N aqueous hydrochloric acid solution at 0°C. The mixture is extracted with ethyl acetate/toluene (1/1), and the organic layer is washed with water, and dried over anhydrous sodium sulfate. The resultant is filtered, and the solvent is evaporated under reduced pressure to give ethyl 2-(8-bromo-2-oxo-5,6-dihydro-4H-imidazo[4,5,1-ij]quinolin-1(2H)-yl)propanoate (1.64 g, quantitative).

IR (cm⁻¹): 1024, 1406, 1497, 1693, 1733

### Reference Example 20

### 2-(8-Bromo-2-oxo-5,6-dihydro-4H-imidazo[4,5,1-ij]quinolin-1(2H)-yl)propionic acid

To a solution of ethyl 2-(8-bromo-2-oxo-5,6-dihydro-4H-imidazo [4,5,1-ij]quinolin-1(2H)-yl)propanoate (1.35 g, 3.82 mmol) in tetrahydrofuran (10 mL) is added a solution of lithium hydroxide (275 mg, 11.5 mmol) in water (10 mL) at 20-25°C, and the mixture is stirred at the same temperature for 2.5 hours. After the reaction, the reaction solution is poured into a 1N aqueous hydrochloric acid solution under ice-cooling, and the mixture is extracted with ethyl acetate. The organic layer is washed with a saturated saline solution, and dried over anhydrous sodium sulfate. The resultant is filtered, and the solvent is evaporated under reduced pressure, and the obtained residue is recrystallized from ethyl acetate-hexane to give 2-(8-bromo-2-oxo-5,6-dihydro-4H-imidazo[4,5,1-ij]quinolin-1(2H)-yl)propionic acid (815 mg, 66 %) .

IR (cm⁻¹): 1070, 1201, 1414, 1635, 1653

### Reference Example 21

### 8-Bromo-4-(hydroxymethyl)-5,6-dihydro-4H-imidazo[4,5,1-ij]quinolin-2(1H)-one

Methyl 1,2,3,4-tetrahydroquinoline-2-carboxylate hydrochloride, which is obtained by the method disclosed in the literature (J. Med. Chem., (1994), 37, 3956-3968), is treated in a similar manner to Reference Examples 12 to 15 to give methyl 8-bromo-2-oxo-1,2,5,6-tetrahydro-4H-imidazo[4,5,1-ij]quinoline-4-carboxylate.

A suspension of methyl 8-bromo-2-oxo-1,2,5,6-tetrahydro-4H-imidazo[4,5,1-ij]quinoline-4-carboxylate (50.0 mg, 161 mmol) and sodium borohydride (60.8 mg, 1.61 mmol) in tetrahydrofuran (1.5 mL) is heated at 50°C, and thereto is added dropwise a solution of methanol/tetrahydrofuran (0.40 mL/0.5 mL) over a period of 10 minutes. The mixture is stirred at the same temperature for one hour, and after the reaction, thereto is added dropwise a saturated aqueous ammonium chloride solution at 0°C. The mixture is extracted with ethyl acetate, and the organic layer is washed with a saturated saline solution, and dried over anhydrous sodium sulfate. The mixture is filtered, and the solvent is evaporated under reduced pressure to give 8-bromo-4-hydroxymethyl-5,6-dihydro-4H-imidazo[4,5,1-ij]quinolin-2(1H)-one (48.5 mg, quantitative).

IR (cm⁻¹): 1066, 1196, 1398, 1487, 1684

### Reference Example 22

### Methyl 8-bromo-1-methyl-2-oxo-1,2,5,6-tetrahydro-4H-imidazo[4,5,1-ij]quinoline-4-carboxylate

To a solution of methyl 8-bromo-2-oxo-1,2,5,6-tetrahydro-4H-imidazo[4,5,1-ij]quinoline-4-carboxylate (100 mg, 321 µmol) in N,N-dimethylformamide (1.0 mL) are added successively potassium carbonate (66.6 mg, 482 µmol) and methyl iodide (60.0 µL, 964 µmol) at 20-25°C, and the mixture is stirred for one hour. After the reaction, water is added to the mixture, and the mixture is extracted with ethyl acetate/toluene (1/1). The organic layer is washed with water and a saturated saline solution, and dried over anhydrous sodium sulfate. The mixture is filtered, and the solvent is evaporated under reduced pressure, and the obtained residue is purified by silica gel column chromatography (hexane/ethyl acetate = 2/1) to give methyl 8-bromo-1-methyl-2-oxo-1,2,5,6-tetrahydro-4H-imidazo[4,5,1-ij]quinolin-4-carboxylate (77.9 mg, 75 %).

IR (cm⁻¹): 1007, 1159, 1400, 1500, 1701

### Reference Example 23

### 8-Bromo-1-methyl-2-oxo-1,2,5,6-tetrahydro-4H-imidazo[4,5,1-ij]-quinoline-4-carboxylic acid

To a solution of methyl 8-bromo-1-methyl-2-oxo-1,2,5,6-tetrahydro-4H-imidazo[4,5,1-ij]quinoline-4-carboxylate (62.2 mg, 191 µmol) in tetrahydrofuran (0.60 mL)-methanol (0.60 mL) is added a solution of lithium hydroxide (13.7 mg, 574 µmol) in water (0.20 mL) at 20-25°C, and the mixture is stirred for 1.5 hour. After the reaction, a 1N aqueous hydrochloric acid solution is added to the mixture, and the mixture is extracted with ethyl acetate. The organic layer is washed with a saturated saline solution, and dried over anhydrous sodium sulfate. The resultant is filtered, and the solvent is evaporated under reduced pressure to give 8-bromo-1-methyl-2-oxo-1,2,5,6-tetrahydro-4H-imidazo[4,5,1-ij]quinoline-4-carboxylic acid (55.7 mg, 94 %).

IR (cm⁻¹): 1007, 1041, 1209, 1498, 1705

### Reference Example 24

### Methyl [5-[(tert-butoxycarbonyl)amino]-2-oxo-5,6-dihydro-4H-imidazo-[4,5,1-ij]quinolin-1(2H)-yl]acetate

To a solution of tert-butyl (2-oxo-1,2,5,6-tetrahydro-4H-imidazo[4,5,1-ij]quinolin-5-yl)carbamate (1.30 g, 4.49 mmol), which is prepared by the method disclosed in the literature (WO 90/15058), in N,N-dimethylformamide (10 mL) are added methyl bromoacetate (450 µL, 4.70 mmol) and potassium carbonate (871 mg, 6.30 mmol), and the mixture is stirred at 20-25°C for one hour. Then, the mixture is further stirred at about 50°C for 4.5 hours. The reaction solution is poured into a 5 % aqueous potassium hydrogen sulfate solution (40 mL) under ice-cooling, and the mixture is extracted with ethyl acetate/toluene (1/1). The organic layer is washed with a saturated saline solution and dried over anhydrous sodium sulfate. The resultant is filtered, and the solvent is evaporated under reduced pressure. The obtained residue is suspended and washed by suspending in hexane and diethyl ether, and the precipitates are collected by filtration to give methyl [5-[(tert-butoxycarbonyl)amino]-2-oxo-5,6-dihydro-4H-imidazo-[4,5,1-ij]quinolin-1(2H)-yl]acetate (1.02 g, 63 %).

IR (cm⁻¹): 1003, 1246, 1423, 1684, 1743

### Reference Example 25

### [5-[(tert-Butoxycarbonyl)amino]-2-oxo-5,6-dihydro-4H-imidazo[4,5,1-ij]quinolin-1(2H)-yl]acetic acid

A solution of methyl [5-[(tert-butoxycarbonyl)amino]-2-oxo-5,6-dihydro-4H-imidazoquinolin-1(2H)-yl]acetate (500 mg, 1.38 mmol) in methanol/tetrahydrofuran (1/ 1, 10 mL) is cooled with ice, and thereto are added sodium hydroxide (166 mg, 4.15 mmol) and water (2 mL), and the mixture is stirred for 40 minutes. The mixture is further stirred at 20-25°C for one hour. To the reaction solution are added a 1N aqueous hydrochloric acid solution (5 mL) and water (5 mL), and the mixture is extracted with ethyl acetate. The organic layer is washed with a saturated saline solution, and dried over anhydrous sodium sulfate. The resultant is filtered, and the solvent is evaporated under reduced pressure to give [5-[(tert-butoxycarbonyl)amino]-2-oxo-5,6-dihydro-4H-imidazo[4,5,1-ij]quinolin-1(2H)-yl]acetic acid (533 mg, quantitative).

IR (cm⁻¹): 1157, 1219, 1491, 1635, 1684

### Reference Example 26

### tert-Butyl (2-amino-3-nitrophenoxy)acetate

To a mixture of 2-amino-3-nitrophenol (2.51 g, 16.3 mmol) and N,N-dimethylformamide (15 mL) are added potassium carbonate (3.15 g, 22.8 mmol) and tert-butyl bromoacetate (2.55 mL, 17.3 mmol) with stirring at 20-25°C, and the mixture is stirred at 20-25°C for 2.5 hours. The reaction solution is poured into water, and the mixture is extracted with toluene. The organic layer is washed successively with water and a saturated saline solution, dried over anhydrous sodium sulfate, and filtered. The solvent is concentrated under reduced pressure, and the residue is purified by silica gel column chromatography (hexane/ethyl acetate = 10/ 1) to give tert-butyl (2-amino-3-nitrophenoxy)acetate (3.59 g, 82 %).

IR (cm⁻¹): 1151, 1236, 1433, 1736, 3350

### Reference Example 27

### tert-Butyl (2-amino-5-bromo-3-nitrophenoxy)acetate

To a solution of tert-butyl (2-amino-3-nitrophenoxy)acetate (2.54 g, 9.47 mmol) in N,N-dimethylformamide (15 mL) is added N-bromosuccinimide (1.77 g, 9.94 mmol) with stirring under ice-cooling. The reaction mixture is stirred under ice-cooling for one hour, and then stirred at 20-25°C for 3 hours. The reaction solution is poured into a 10 % aqueous sodium thiosulfate solution, and thereto is added diethyl ether. The mixture is stirred at 20-25°C for 30 minutes, and the organic layer and the aqueous layer are separated. The diethyl ether layer is washed successively with water and a saturated saline solution, and dried over anhydrous sodium sulfate. The resultant is filtered, and the solvent is concentrated under reduced pressure to give tert-butyl (2-amino-5-bromo-3-nitrophenoxy)acetate (3.26 g, 99 %).

IR (cm⁻¹): 1151, 1209, 1514, 1743, 3369

### Reference Example 28

### 7-Bromo-5-nitro-2H-1,4-benzoxazin-3(4H)-one

A mixture of tert-butyl (2-amino-5-bromo-3-nitrophenoxy)-acetate (2.33 g, 6.71 mmol), p-toluenesulfonic acid monohydrate (100 mg, 0.526 mmol) and toluene (10 mL) is stirred at 80°C for 2 hours. The reaction solution is concentrated under reduced pressure, and to the residue is added a saturated aqueous sodium hydrogen carbonate solution. The mixture is extracted with chloroform, and the organic layer is washed with a saturated saline solution, and dried over anhydrous magnesium sulfate. The resultant is filtered, and the solvent is concentrated under reduced pressure to give 7-bromo-5-nitro-2H-1,4-benzoxazin-3(4H)-one (1.82 g, 99 %).

IR (cm⁻¹): 1063, 1284, 1483, 1525, 1697

### Reference Example 29

### 5-Nitro-7-phenyl-2 H-1,4-benzoxazin-3 (4H)-one

A mixture of 7-bromo-5-nitro-2H-1,4-benzoxazin-3(4H)-one (1.02 g, 3.74 mmol), phenylboric acid (547 mg, 4.49 mmol), potassium carbonate (1.55 g, 11.2 mmol), tetrakistriphenylphosphine palladium (130 mg, 0.112 mmol), 1,4-dioxane (10 mL) and water (2 mL) is refluxed for 2 hours. The reaction solution is cooled to 20-25°C, and thereto are added water and chloroform and the mixture is separated. The aqueous layer is acidified with a 5 % aqueous potassium hydrogen sulfate, and the mixture is extracted with ethyl acetate. The organic layer is washed with a saturated saline solution, and dried over anhydrous magnesium sulfate. The resultant is filtered and the solvent is concentrated under reduced pressure to give [(4-amino-5-nitrobiphenyl-3-yl)oxy]acetic acid (880.5 mg, 87 %).

Subsequently, a mixture of the obtained product (869 mg, 3.01 mmol), p-toluenesulfonic acid monohydrate (90.1 mg, 0.474 mmol) and toluene (15 mL) is refluxed for 1.5 hour, and the reaction solution is concentrated under reduced pressure. To the residue is added a saturated aqueous sodium hydrogen carbonate solution, and the mixture is extracted with chloroform. The organic layer is washed with a saturated saline solution, dried over anhydrous magnesium sulfate, and the mixture is filtered. The solvent is concentrated under reduced pressure to give 5-nitro-7-phenyl-2H-1,4-benzoxazin-3(4H)-one (772 mg, 95%).

IR (cm⁻¹): 1176, 1270, 1336, 1541, 1709

### Reference Example 30

### (2-Oxo-8-phenyl-4,5-dihydroimidazo[1,5,4-de][1,4]benzoxazin-1(2H)-yl)-acetic acid

To a suspension of lithium aluminum hydride (96.8 mg, 2.55 mmol) in tetrahydrofuran (3 mL) is added dropwise a solution of 5-nitro-7-phenyl-2H-1,4-benzoxazin-3(4H)-one (173 mg, 0.638 mmol) in tetrahydrofuran (5 mL) with stirring under reflux over a period of 10 minutes, and the mixture is further refluxed for one hour. The reaction solution is cooled with ice, and thereto are added dropwise successively water (0.1 mL), a 15 % aqueous sodium hydroxide solution (0.1 mL) and water (0.3 mL). To the mixture are added diethyl ether and anhydrous potassium carbonate, and the mixture is stirred at 20-25°C for 30 minutes. The mixture is filtered, and concentrated under reduced pressure to give crude 7-phenyl-3,4-dihydro-2H-1,4-benzoxazin-5-amine (121 mg).

The obtained crude product is treated in a similar manner to Reference Example 4 to give (2-oxo-8-phenyl-4,5-dihydroimidazo[1,5,4-de] [1,4]benzoxazin-1(2H)-yl)acetic acid.

IR (cm⁻¹): 1036, 1201, 1336, 1653, 1724

### Reference Example 31

### tert-Butyl (2-oxo-9-phenyl-4,5,6,7-tetrahydroimidazo[4,5,1-jk][1]-benzazepin-1(2H)-yl)acetate

2,3,4,5-Tetrahydro-1H-1-benzazepine, which is prepared by the method disclosed in the literature (Tetrahedron Lett., (1983), 24, 4711-4712), is treated in a similar manner to Reference Example 16 to give tert-butyl (9-bromo-2-oxo-4,5,6,7-tetrahydroimidazobenzazepin-1(2H)-yl)acetate.

To a solution of tert-butyl (9-bromo-2-oxo-4,5,6,7-tetrahydroimidazobenzazepin-1(2H)-yl)acetate (1.84 g, 4.83 mmol), phenylboric acid (706 mg, 5.79 mol) and tetrakistriphenylphosphine palladium (168 mg, 145 µmol) in 1,4-dioxane (20 mL) is added a solution of potassium carbonate (2.00 g, 14.5 mmol) in water (4.0 mL), and the mixture is purged with nitrogen gas. The mixture is refluxed with stirring for 5 hours. The reaction solution is cooled to 20-25°C, and thereto are added a 5 % aqueous potassium carbonate solution and chloroform, and the mixture is separated. The organic layer is washed with a saturated saline solution, dried over anhydrous magnesium sulfate, and the mixture is filtered. The solvent is concentrated under reduced pressure, and the resulting residue is suspended in diethyl ether/hexane for crystallization, and the precipiated crystals are collected by filtration to give tert-butyl (2-oxo-9-phenyl-4,5,6,7-tetrahydroimidazo-[4,5,1-jk][1]benzazepin-1(2H)-yl)acetate (1.74 g, 95 %).

IR (cm⁻¹): 1153, 1234, 1481, 1701, 1741

### Reference Example 32

### (2-Oxo-9-phenyl-4,5,6,7-tetrahydroimidazo[4,5,1-jk][1]benzazepin-1(2H)-yl)acetic acid

tert-Butyl (2-oxo-9-phenyl-4,5,6,7-tetrahydroimidazo[4,5,1-jk][1]benzazepine-1(2H)-yl)acetate is treated in a similar manner to Reference Example 4 to give (2-oxo-9-phenyl-4,5,6,7-tetrahydroimidazo[4,5,1-jk][1]benzazepin-1(2H)-yl)acetic acid.

IR (cm⁻¹): 1200, 1433, 1483, 1660, 1730

### Reference Example 33

### 2-Bromo-N-methyl-N-phenylacetamide

To a solution of N-methylaniline (1.90 mL, 17.5 mmol) and triethylamine (2.44 mL, 17.5 mmol) in ethyl acetate (40 mL) is added a solution of bromoacetyl bromide (1.52 mL, 17.5 mmol) in ethyl acetate (40 mL) at 0°C, and the mixture is stirred at 20-25°C for 30 minutes. After the reaction, the mixture is filtered, and the solvent is evaporated under reduced pressure to give 2-bromo-N-methyl-N-phenylacetamide (4.60 g, quantitative).

IR (cm⁻¹): 1109, 1375, 1593, 1624, 1683

### Reference Example 34

### 2-Bromo-2,2-difluoro-N-methyl-N-phenylacetamide

To a solution of N-methylaniline (560 µL, 5.17 mmol) and triethylamine (721 µL, 5.17 mmol) in ethyl acetate (10 mL) is added a solution of bromo(difluoro)acetyl chloride (1.00 g, 5.17 mmol) in ethyl acetate (10 mL) at 0°C, and the mixture is stirred at 20-25°C for 1.5 hour. After the reaction, the mixture is filtered, and the solvent is evaporated under reduced pressure to give 2-bromo-2,2-difluoro-N-methyl-N-phenylacetamide (1.41 g, quantitative).

IR (cm⁻¹): 933, 1103, 1144, 1497, 1684

### Reference Example 35

### tert-Butyl [2-oxo-5-(4,4,5,5-tetramethyl-1,3,2-dioxoboran-2-yl)-1,3-benzoxazol-3(2H)-yl]acetate

To a mixture of the compound obtained in Reference Example 3 (5.00 g, 15.2 mmol), bis(pinacolato)diboron (4.26 g, 16.8 mmol), potassium acetate (2.25 g, 22.9 mmol), tricyclohexylphosphine (1.03 g, 3.67 mmol) and 1,4-dioxane (95 mL) is added with stirring tris(dibenzylideneacetone) dipalladium (0) (699.5 mg, 0.764 mmol) at 20-25°C, and the mixture is heated under reflux for 24 hours. The reaction mixture is cooled to 20-25°C, and poured into a saturated aqueous sodium hydrogen carbonate solution, and the mixture is extracted with ethyl acetate. The organic layer is washed successively with water and a saturated saline solution, dried over anhydrous sodium sulfate, and concentrated under reduced pressure. The residue is purified by silica gel column chromatography (hexane/ethyl acetate = 20/1 to 5/ 1) to give tert-butyl [2-oxo-5-(4,4,5,5-tetramethyl-1,3,2-dioxaboran-2-yl)-1,3-benzoxazol-3(2H)-yl]acetate (4.46 g, 78 %).

### Reference Example 36

### N-Methyl-2-[2-oxo-5-(4,4,5,5-tetramethyl-1,3,2-dioxaboran-2-yl)-1,3-benzoxazol-3(2H)-yl]-N-phenylacetamide

The title compound is obtained from the compound obtained in Example 27 in a similar manner to Reference Example 35.

IR (cm⁻¹): 704, 1140, 1458, 1666, 1786

### Reference Example 37

### (5-Chloro-2-oxo-1,3-benzoxazol-3(2H)-yl)acetic acid

The title compound is obtained from 5-chloro-1,3-benzoxazol-2(3H)-one in a similar manner to Reference Example 3 and Reference Example 4.

IR (cm⁻¹): 798, 1240, 1724, 1774, 3064

### Reference Example 38

### (5-Cyano-2-oxo-1,3-benzoxazol-3(2H)-yl)acetic acid

The title compound is obtained from 4-hydroxy-3-nitrobenzonitrile in a similar manner to Example 28 and Reference Examples 2 to 4.

IR (cm⁻¹): 673, 1243, 1490, 1730, 3084

### Example 1

### 2-(2-Oxo-5-phenyl-1,3-benzoxazol-3(2H)-yl)-N,N-dipropylacetamide

To a solution of the compound obtained in Reference Example 5 (269 mg, 1.00 mmol) in N,N-dimethylformamide (1.0 mL) are added successively 1-ethyl-3-(3-dimethylaminopropyl)-carbodiimide hydrochloride (230 mg, 1.20 mmol), 1-hydroxybenzotriazole (135 mg, 1.00 mmol) and dipropylamine (137 µL, 1.00 mmol) at 20-25°C, and the mixture is stirred at 20-25°C for 3 hours. After the reaction, water is added to the reaction solution, and the mixture is extracted with a mixed solvent of ethyl acetate/toluene (1/1). The organic layer is washed with water, a 5% aqueous sodium hydrogen sulfate solution, a saturated aqueous sodium hydrogen carbonate solution and a saturated saline solution, and dried over anhydrous sodium sulfate. The resultant is filtered, and the solvent is evaporated under reduced pressure, and the resulting residue is purified by silica gel column chromatography (chloroform) to give 2-(2-oxo-5-phenyl-1,3-benzoxazol-3(2H)-yl)-N,N-dipropylacetamide (219 mg, 62 %).

IR (cm⁻¹): 1147, 1485, 1647, 1772, 1790

### Example 2

### 3-[2-(3,4-Dihydroquinolin-1(2H)-yl)-2-oxoethyl]-5-phenyl-1,3-benzoxazol-2(3H)-one

The title compound is obtained from the compound synthesized in Reference Example 5 in a similar manner to Example 1.

IR (cm⁻¹): 1022, 1387, 1487, 1643, 1784

### Example 3

### 3-[2-(3,4-Dihydroisoquinolin-2(1H)-yl)-2-oxoethyl]-5-phenyl-1,3-benzoxazol-2(3H)-one

The title compound is obtained from the compound synthesized in Reference Example 5 in a similar manner to Example 1.

IR (cm⁻¹): 1251, 1448, 1481, 1649, 1778

### Example 4

### Methyl 3-{methyl[(5-phenyl-1,3-benzoxazol-3(2H)-yl)acetyl]amino}-benzoate

To a suspension of the compound obtained in Reference Example 5 (1.08 mg, 4.00 mmol) in dichloromethane (15 mL) is added oxalyl chloride (384 µL, 4.40 mmol) at 20-25°C, and thereto is further added N,N-dimethylformamide (one drop), and the mixture is stirred for one hour. After the reaction, the solvent is evaporated under reduced pressure, and toluene is added thereto, and the mixture is evaporated under reduced pressure again. Then, the solvent is removed to the fullest extent by a vacuum pump, and tetrahydrofuran (10 mL) is added thereto to give an acid chloride solution. To a solution of methyl 3-(methylamino)benzoate (793 mg, 4.80 mmol) in tetrahydrofuran (10 mL) is added dropwise the above acid chloride solution at 20-25°C, and the mixture is stirred for 30 minutes. After the reaction, water is added to the mixture, and the mixture is extracted with ethyl acetate. The organic layer is washed with a 1N aqueous hydrochloric acid solution and a saturated saline solution, and dried over anhydrous sodium sulfate. The resultant is filtered, and the solvent is evaporated under reduced pressure, and the residue is purified by silica gel column chromatography (hexane/ethyl acetate = 3/ 1 to 1/ 1) to give methyl 3-{methyl[(5-phenyl-1,3-benzoxazol-3(2H)-yl)acetyl]amino}benzoate (1.37 g, 82%).

IR (cm⁻¹): 1086, 1242, 1483, 1672, 1774

The compounds of Examples 5-25 are obtained from the compound synthesized in Reference Example 5 in a similar manner to Example 1 or Example 4.

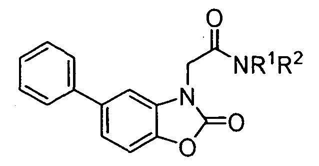

| Ex. No. | R¹ | R² | IR (cm⁻¹) |
|---|---|---|---|
| 5 | Me | Ph | 1120,1385,1485,1662,1778 |
| 6 | Me | 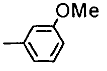 | 1039,1383,1485,1674,1782 |
| 7 | Me | 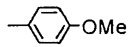 | 1022,1248,1508,1670,1770 |
| 8 | Me | 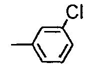 | 1020,1250,1481,1672,1770 |
| 9 | Me | 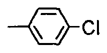 | 1090,1250,1483,1670,1768 |
| 10 | Me | 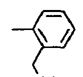 | 1020,1250,1383,1483,1664 |
| 11 | Me | 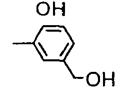 | 920,1120,1387,1481,1657 |
| 12 | Me | 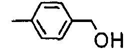 | 1124,1244,1483,1655,1778 |
| 13 | Me | 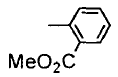 | 1082,1381,1483,1714,1770 |
| 14 | Me | 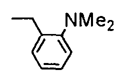 | 1020,1442,1479,1658,1778 |
| 15 | Me | 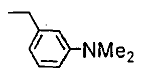 | 1392,1484,1600,1650,1783 |
| 16 | Me | 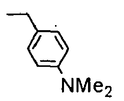 | 925,1486,1521,1646,1785 |
| 17 | Me | 2-Py | 1022,1252,1589,1670,1772 |
| 18 | Me | 3-Py | 1097,1381,1485,1670,1780 |
| 19 | Me | cyclohexyl | 1097,1238,1485,1643,1778 |
| 20 | Me | Bn | 1026,1483,1649,1749,1768 |
| 21 | Et | Ph | 1018,1257,1487,1664,1776 |
| 22 | Et | 3-Py | 1020,1284,1653,1670,1792 |
| 23 | Et | Bn | 1247,1392,1483,1646,1768 |
| 24 | Pr | -(CH₂)₂-OMe | 1252,1390,1483,1647,1782 |
| 25 | i-Pr | Ph | 1252,1481,1653,1670,1794 |

### Example 26

### N-Methyl-2-(5-nitro-2-oxo-1,3-benzoxazol-3(2H)-yl)-N-phenylacetamide

The title compound is obtained from the compound synthesized in Reference Example 6 in a similar manner to Example 1.

IR (cm⁻¹): 1338, 1485, 1522, 1664, 1790

### Example 27

### 2-(5-Bromo-2-oxo-1,3-benzoxazol-3(2H)-yl)-N-methyl-N-phenylacetamide

To a solution of the compound obtained in Reference Example 4 (10.0 g, 36.8 mmol) in N,N-dimethylformamide (1.0 mL) are added successively N-methylaniline (4.78 mL, 44.1 mmol), 1-ethyl-3-(3-dimethylaminopropyl)-carbodiimide hydrochloride (9.07 g, 47.3 mmol), and 1-hydroxybenzotriazole (4.97 g, 36.8 mmol) at 20-25°C, and the mixture is stirred for 16 hours. After the reaction, water is added to the reaction solution, and the mixture is extracted with a mixed solvent of ethyl acetate/ toluene (1/1). The organic layer is washed with water, and dried over anhydrous sodium sulfate. The resultant is filtered, and the solvent is evaporated under reduced pressure. The obtained residue is recrystallized from 2-propanol to give 2-(5-bromo-2-oxo-1,3-benzoxazol-3(2H)-yl)-N-methyl-N-phenylacetamide (11.1 g, 84 %).

IR (cm⁻¹): 1120, 1377, 1483, 1666, 1772

### Example 28

### N-Methyl-2-(2-oxo-1,3-benzoxazol-3(2H)-yl)-N-phenylacetamide

To a solution of a 10 % palladium on carbon (3.00 mg) in methanol (1.0 ml) is added a solution of the compound obtained in Example 27 (36.6 mg, 0.101 mmol) in methanol (3.0 ml) under nitrogen atmosphere. The mixture is stirred at 20-25°C for 2 hours under hydrogen atmosphere. The mixture is filtered through cerite, and evaporated under reduced pressure to give N-methyl-2-(2-oxo-1,3-benzoxazol-3(2H)-yl)-N-phenylacetamide (30.7 mg, 100 %).

IR (cm⁻¹): 1020, 1240, 1489, 1670, 1767

### Example 29

### N-Methyl-2-(2-oxo-5-pyridin-3-yl-1,3-benzoxazol-3(2H)-yl)-N-phenylacetamide

To a solution of the compound obtained in Example 27 (1.08 g, 3.00 mmol), 3-pyridineboric acid (443 mg, 3.60 mmol), and tetrakistriphenylphosphine palladium (104 mg, 90.0 µmol) in 1,4-dioxane (30 mL) is added a solution of potassium carbonate (1.24 g, 9.00 mmol) in water (6.0 mL), and the mixture is stirred under reflux for 2 hours. After the reaction, the mixture is poured into a mixed solution of a saturated aqueous sodium hydrogen carbonate solution/ethyl acetate at 0°C, and the mixture is extracted with ethyl acetate. The organic layer is washed with a saturated saline solution, and dried over anhydrous sodium sulfate. The resultant is filtered, and the solvent is evaporated under reduced pressure. The obtained residue is recrystallized from 2-propanol to give N-methyl-2-(2-oxo-5-pyridin-3-yl-1,3-benzoxazol-3(2H)-yl)-N-phenylacetamide (746 mg, 69 %).

IR (cm⁻¹): 1022, 1246, 1483, 1657, 1780

The compounds of Examples 30-37 are obtained from the compound synthesized in Example 27 in a similar manner to Example 29.

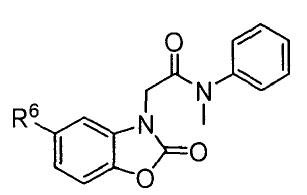

| Ex. No. | R⁶ | IR (cm⁻¹) |
|---|---|---|
| 30 | 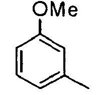 | 1020,1379,1483,1670,1778 |
| 31 | 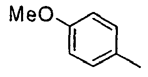 | 1020,1387,1489,1670,1772 |
| 32 | 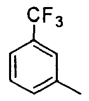 | 1016,1381,1489,1651,1788 |
| 33 | 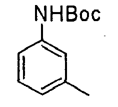 | 1018,1157,1238,1666,1766 |
| 34 | 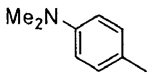 | 1232,1386,1490,1673,1762 |
| 35 | 2-Py | 1080,1387,1587,1660,1786 |
| 36 | 4-Py | 1016,1383,1485,1668,1780 |
| 37 | 3-thienyl | 1022,1371,1490,1658,1774 |

### Example 38

### 2-(5-(4-Aminophenyl)-2-oxo-1,3-benzoxazol-3(2H)-yl)-N-methyl-N-phenylacetamide hydrochloride

To a solution of the compound obtained in Example 33 (40.0 mg, 85.0 µmol) in 1,4-dioxane (0.20 mL) is added a 4N hydrochloric acid/ 1,4-dioxane (0.15 mL), and the mixture is stirred at 50°C for 2 hours. After the reaction, the solvent is evaporated under reduced pressure, and the resultant is washed by suspending in diethyl ether. The precipitates are collected by filtration, and dried to give 2-[5-(4-aminophenyl)-2-oxo-1,3-benzoxazol-3(2H)-yl]-N-methyl-N-phenylacetamide hydrochloride (33.0 mg, 96 %).

IR (cm⁻¹): 1120, 1243, 1382, 1483, 1774

The compounds of Examples 39-41 are obtained from the compound synthesized in Reference Example 3 in a similar manner to Reference Example 4, Example 1 and Example 29.

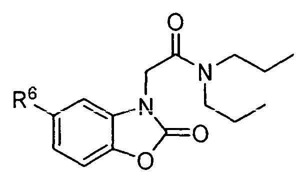

| Ex. No. | R⁶ | IR (cm⁻¹) |
|---|---|---|
| 39 | 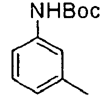 | 1155,1234,1484,1648,1778 |
| 40 | 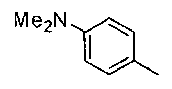 | 1018,1234,1488,1608,1770 |
| 41 | 3-thienyl | 1018,1147,1234,1646,1770 |

The compounds of Examples 42-45 are obtained from the compound synthesized in Reference Example 3 in a similar manner to Reference Example 4, Example 1 and Example 29.

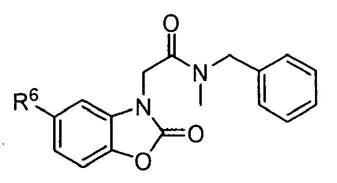

| Ex. No. | R⁶ | IR (cm⁻¹) |
|---|---|---|
| 42 | 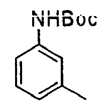 | 1160,1240,1484,1646,1785 |
| 43 | 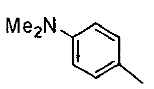 | 1243,1488,1610,1648,1781 |
| 44 | 4-Py | 1030,1485,1597,1647,1792 |
| 45 | 3-thienyl | 1253,1380,1494,1648,1785 |

### Example 46

### 2-[5-(4-Aminophenyl)-2-oxo-1,3-benzoxazol-3(2H)-yl]-N-benzyl-N-methylacetamide hydrochloride

2-[5-(4-Aminophenyl)-2-oxo-1,3-benzoxazol-3(2H)-yl]-N-benzyl-N-methylacetamide hydrochloride is obtained from the compound synthesized in Example 42 in a similar manner to Example 38.

IR (cm⁻¹): 1024, 1251, 1484, 1652, 1770

### Example 47

### 2-(5-Anilino-2-oxo-1,3-benzoxazol-3(2H)-yl)-N-methyl-N-phenylacetamide

A solution of tris(dibenzylidineacetone) dipalladium (22.9 mg, 25.0 µmol) and 4,5-bis(diphenylphosphino)-9,9'-dimethylxanthene (43.4 mg, 75.0 µmol) in toluene (4.0 mL) is purged with nitrogen gas, and the mixture is stirred at 30°C for 30 minutes. The reaction solution is cooled to 20-25°C, and thereto are added the compound synthesized in Example 27 (181 mg, 500 µmol), cesium carbonate (228 mg, 700 µmol), and aniline (68.3 µL, 750 µmol). The mixture is purged with nitrogen gas, and stirred under reflux for 5 hours. After the reaction, the reaction solution is cooled to 20-25°C, and thereto is added a saturated aqueous sodium hydrogen carbonate solution, and the mixture is extracted with chloroform. The organic layer is washed with a saturated saline solution, and dried over anhydrous sodium sulfate. The resultant is filtered, and the solvent is evaporated under reduced pressure. The residue is purified by silica gel column chromatography (hexane/ethyl acetate = 2/ 1) to give 2-(5-anilino-2-oxo-1,3-benzoxazol-3(2H)-yl)-N-methyl-N-phenylacetamide (88.0 mg, 47 %).

IR (cm⁻¹): 1020, 1387, 1489, 1595, 1757

The compounds of Example 48 and Example 49 are obtained from the compound synthesized in Example 27 in a similar manner to Example 47.

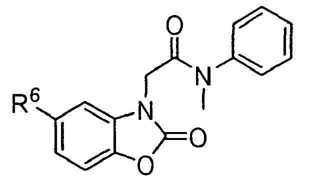

| Ex. No. | R⁶ | IR (cm⁻¹) |
|---|---|---|
| 48 | 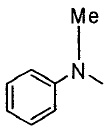 | 1227,1392,1495,1655,1768 |
| 49 | 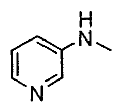 | 1018,1491,1581,1627,1770 |

### Example 50

### N-Methyl-2-(2-oxo-5-phenoxy-1,3-benzoxazol-3(2H)-yl)-N-phenylacetamide

To a solution of phenol (188 mg, 2.0 0mmol) in pyridine (2.0 mL) are added the compound synthesized in Example 27 (181 mg, 0.500 mmol), potassium carbonate (415 mg, 3.00 mmol) and copper (II) oxide (199 mg, 2.50 mmol), and the mixture is purged with nitrogen gas. The mixture is stirred under reflux for 11 hours. After the reaction, the reaction solution is cooled to 20-25°C and diluted with chloroform. The mixture is filtered, and water is added to the filtrate, and extracted with chloroform. The organic layer is washed with a 2N aqueous hydrochloric acid solution, and dried over anhydrous sodium sulfate. The resultant is filtered, and the solvent is evaporated under reduced pressure, and the obtained residue is purified by silica gel column chromatography (hexane/ethyl acetate = 3/ 1) to give N-methyl-2-(2-oxo-5-phenoxy-1,3-benzoxazol-3(2H)-yl)-N-phenylacetamide (100 mg, 53 %).

IR (cm⁻¹): 1016, 1387, 1487, 1664, 1778

The compounds of Examples 51-54 are obtained from the compound synthesized in Reference Example 8 or Reference Example 9 in a similar manner to Example 1 and Example 29.

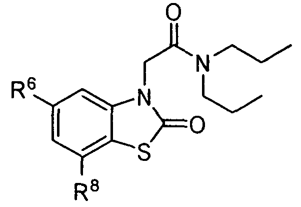

| Ex. No. | R⁶ | R⁸ | IR (cm⁻¹) |
|---|---|---|---|
| 51 | Br | H | 1234,1325,1471,1643,1695 |
| 52 | H | Br | 1145,1236,1456,1641,1695 |
| 53 | Ph | H | 1232,1336,1467,1635,1687 |
| 54 | H | Ph | 1144,1236,1470,1643,1680 |

The compounds of Examples 55-58 are obtained from the compound synthesized in Reference Example 8 or Reference Example 9 in a similar manner to Example 1 and Example 29.

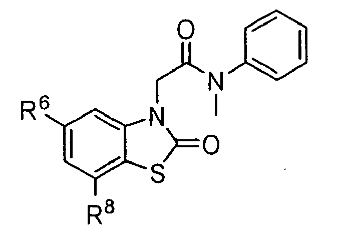

| Ex. No. | R⁶ | R⁸ | IR (cm⁻¹) |
|---|---|---|---|
| 55 | Br | H | 1331,1470,1583,1660,1684 |
| 56 | H | Br | 1313,1465,1589,1662,1684 |
| 57 | Ph | H | 1118,1330,1495,1593,1670 |
| 58 | H | Ph | 1322,1429,1495,1593,1664 |

The compounds of Example 59 and Example 60 are obtained from the compound synthesized in Reference Example 10 or Reference Example 11 in a similar manner to Example 1 and Example 29.

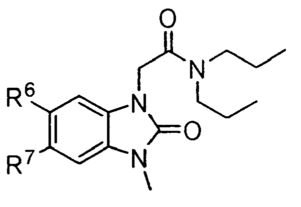

| Ex. No. | R⁶ | R⁷ | IR(cm⁻¹) |
|---|---|---|---|
| 59 | H | Ph | 1232,1396,1449,1653,1712 |
| 60 | Ph | H | 1230,1406,1440,1647,1712 |

The compounds of Examples 61-66 are obtained from the compound synthesized in Reference Example 10 or Reference Example 11 in a similar manner to Example 1, and Example 29 or Example 47.

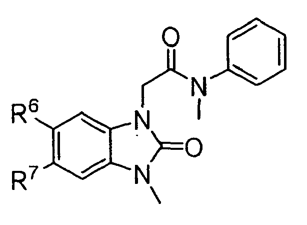

| Ex. No. | R⁶ | R⁷ | IR (cm⁻¹) |
|---|---|---|---|
| 61 | H | Ph | 1389,1435,1489,1672,1716 |
| 62 | Ph | H | 1389,1441,1495,1670,1695 |
| 63 | H | 3-Py | 1290,1392,1490,1670,1720 |
| 64 | 3-Py | H | 1118,1425,1493,1659,1697 |
| 65 | H | 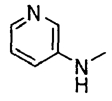 | 1389,1497,1579,1655,1713 |
| 66 | 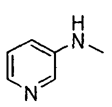 | H | 1313,1390,1504,1662,1695 |

The compounds of Examples 67-69 are obtained from the compound synthesized in Reference Example 10 or Reference Example 11 in a similar manner to Example 1 or Example 4, and Example 29.

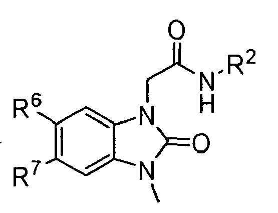

| Ex. No. | R² | R⁶ | R⁷ | IR (cm⁻¹) |
|---|---|---|---|---|
| 67 | 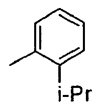 | H | Ph | 1249,1444,1529,1651,1724 |
| 68 | 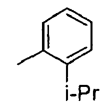 | Ph | H | 1168,1259,1439,1649,1716 |
| 69 | 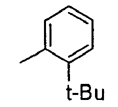 | Ph | H | 1238,1438,1662,1691,1710 |

### Example 70

### 1-(2-Oxo-2-piperidin-1-ylethyl)-8-phenyl-5,6-dihydro-4H-imidazo[4,5,1-ij]quinolin-2(1H)-one

The title compound is obtained from the compound synthesized in Reference Example 18 in a similar manner to Example 1.

IR (cm⁻¹): 1011, 1228, 1498, 1643, 1697

### Example 71

### 1-[2-(3,4-Dihydroquinolin-1(2H)-yl)-2-oxoethyl]-8-phenyl-5,6-dihydro-4H-imidazo[4,5, 1-ij]quinolin-2(1H)-one

The title compound is obtained from the compound synthesized in Reference Example 18 in a similar manner to Example 1.

IR (cm⁻¹): 1105, 1230, 1435, 1641, 1720

### Example 72

### 1-[2-(3,4-Dihydroisoquinolin-2(1H)-yl)-2-oxoethyl]-8-phenyl-5,6-dihydro-4H-imidazo[4,5,1-ij]quinolin-2(1H)-one

The title compound is obtained from the compound synthesized in Reference Example 18 in a similar manner to Example 1.

IR (cm-1): 1412, 1464, 1491, 1654, 1704

### Reference Example 73

### 1-[2-(2,3-Dihydro-4H-1,4-benzoxazin-4-yl)-2-oxoethyl]-8-phenyl-5,6-dihydro-4H-imidazo[4,5,1-ij]quinolin-2(1H)-one

The title compound is obtained from the compound synthesized in Reference Example 18 in a similar manner to Example 4.

IR (cm⁻¹): 1257, 1394, 1429, 1490, 1670

The compounds of Examples 74-109 are obtained from the compound synthesized in Reference Example 18 in a similar manner to Example 1 or Example 4.

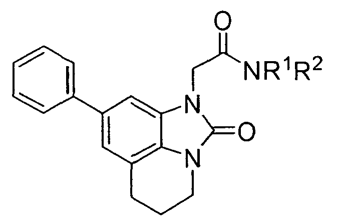

| Ex. No. | R¹ | R² | IR (cm⁻¹) |
|---|---|---|---|
| 74 | H | Ph | 1196,1238,1497,1558,1687 |
| 75 | H | 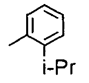 | 972,1230,1423,1662,1713 |
| 76 | Me | Ph | 1122,1230,1423,1666,1713 |
| 77 | Me | 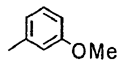 | 970,1284,1489,1674,1693 |
| 78 | Me | 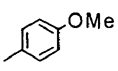 | 1120,1238,1425,1662,1691 |
| 79 | Me | 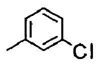 | 1018,1280,1492,1591,1674 |
| 80 | Me | 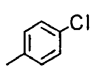 | 1012,1284,1425,1670,1695 |
| 81 | Me | 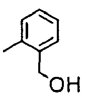 | 1103,1491,1660,1676,1705 |
| 82 | Me | 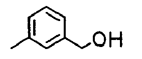 | 1065,1234,1488,1653,1691 |
| 83 | Me | 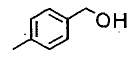 | 970,1016,1421,1674,1693 |
| 84 | Me | 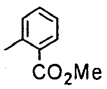 | 1290,1421,1493,1676,1705 |
| 85 | Me | 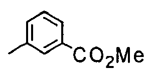 | 972,1103,1423,1662,1705 |
| 86 | Me | 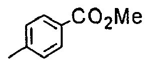 | 974,1275,1425,1659,1701 |
| 87 | Me | 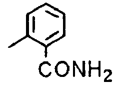 | 1234,1383,1425,1491,1662 |
| 88 | Me | 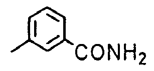 | 1107,1383,1645,1674,1697 |
| 89 | Me | 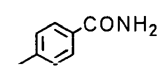 | 1387,1429,1492,1666,1700 |
| 90 | Me | 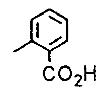 | 1132,1232,1383,1491,1699 |
| 91 | Me | 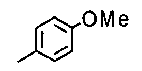 | 972,1228,1491,1655,1695 |
| 92 | Me | 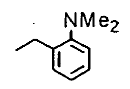 | 1105,1236,1490,1646,1700 |
| 93 | Me | 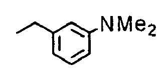 | 1427,1494,1604,1654,1691 |
| 94 | Me | 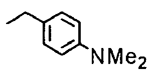 | 1429,1496,1521,1658,1712 |
| 95 | Me | 2-Py | 974,1134,1313,1660,1697 |
| 96 | Me | 3-Py | 972,1232,1423,1666,1709 |
| 97 | Me | 4-Py | 970,1238,1429,1587,1672 |
| 98 | Me | 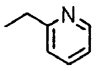 | 1101,1238,1423,1650,1697 |
| 99 | Me | 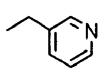 | 1236,1419,1496,1643,1704 |
| 100 | Me | 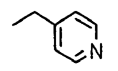 | 1288,1415,1496,1652,1708 |
| 101 | Me | Bn | 1124,1236,1493,1653,1693 |
| 102 | Me | cyclohexyl | 1146,1230,1495,1643,1716 |
| 103 | Me | 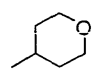 | 1084,1230,1495,1649,1713 |
| 104 | Et | Ph | 1132,1230,1423,1664,1713 |
| 105 | Et | Bn | 975,1261,1425,1652,1704 |
| 106 | Et | 3-Py | 1136,1281,1425,1670,1713 |
| 107 | Pr | Pr | 1101,1230,1495,1649,1705 |
| 108 | Pr | -(CH₂)₂-OMe | 1446,1494,1649,1689,1706 |
| 109 | i-Pr | Ph | 1117,1298,1425,1659,1689 |

### Example 110

### 2-(8-Bromo-2-oxo-5,6-dihydro-4H-imidazo[4,5,1-ij]quinolin-1(2H)-yl)-N,N-dipropylacetamide

The title compound is obtained from the compound synthesized in Reference Example 17 in a similar manner to Example 1.

IR (cm⁻¹): 978, 1232, 1409, 1641, 1707

### Example 111

### 2-(8-Bromo-2-oxo-5,6-dihydro-4H-imidazo[4,5,1-ij]quinolin-1(2H)-yl)-N-methyl-N-phenylacetamide

The title compound is obtained from the compound synthesized in Reference Example 17 in a similar manner to Example 1.

IR (cm⁻¹): 970, 1103, 1504, 1662, 1705

### Example 112

### N-Benzyl-2-(8-bromo-2-oxo-5,6-dihydro-4H-imidazo[4,5,1-ij]quinolin-1(2H)-yl)-N-methylacetamide

The title compound is obtained from the compound synthesized in Reference Example 17 in a similar manner to Example 1.

IR (cm⁻¹): 1016, 1232, 1408, 1655, 1695

### Example 113

### N-Methyl-2-(2-oxo-5,6-dihydro-4H-imidazo[4,5,1-ij]quinolin-1(2H)-yl)-N-phenylacetamide

The title compound is obtained from the compound synthesized in Example 111 in a similar manner to Example 28.

IR (cm⁻¹): 970, 1099, 1421, 1660, 1699

The compounds of Examples 114-122 are obtained from the compound synthesized in Example 111 in a similar manner to Example 29.

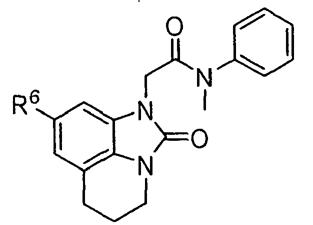

| Ex. No. R⁶ | | IR (cm⁻¹) |
|---|---|---|
| 114 | 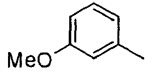 | 972,1236,1491,1660,1705 |
| 115 | 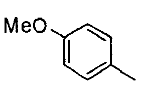 | 1034,1242,1425,1487,1668 |
| 116 | 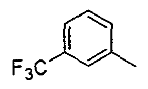 | 1072,1117,1329,1660,1716 |
| 117 | 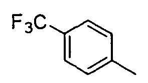 | 974,1065,1323,1660,1709 |
| 118 | 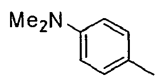 | 1124,1419,1504,1670,1689 |
| 119 | 2-Py | 1126,1230,1425,1666,1705 |
| 120 | 3-Py | 974,1421,1491,1659,1691 |
| 121 | 4-Py | 974,1234,1497,1662,1709 |
| 122 | 3-thienyl | 971,1427,1494,1662,1704 |

The compounds of Examples 123-127 are obtained from the compound synthesized in Reference Example 16 in a similar manner to Reference Example 4, Example 1 or Example 4, and Example 29.

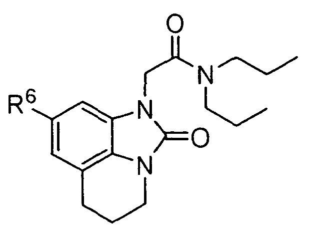

| Ex. No. | R⁶ | IR (cm⁻¹) |
|---|---|---|
| 123 | 2-Py | 974,1228,1437,1643,1701 |
| 124 | 3-Py | 1147,1228,1414,1643,1691 |
| 125 | 4-Py | 1147,1230,1412,1647,1705 |
| 126 | 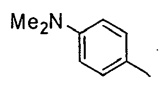 | 1230,1504,1608,1652,1700 |
| 127 | 3-thienyl | 1145,1230,1508,1652,1704 |

The compounds of Examples 128-132 are obtained from the compound synthesized in Reference Example 16 in a similar manner to Reference Example 4, Example 1 or Example 4, and Example 29.

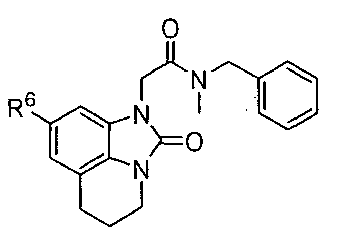

| Ex. No. | R⁶ | IR(cm⁻¹) |
|---|---|---|
| 128 | 2-Py | 1113,1230,1466,1643,1713 |
| 129 | 3-Py | 1111,1240,1433,1651,1682 |
| 130 | 4-Py | 991,1105,1497,1647,1716 |
| 131 | 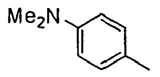 | 1118,1427,1506,1662,1691 |
| 132 | 3-thienyl | 1097,1409,1508,1654,1700 |

The compounds of Examples 133-135 are obtained from the compound synthesized in Example 111 in a similar manner to Example 47.

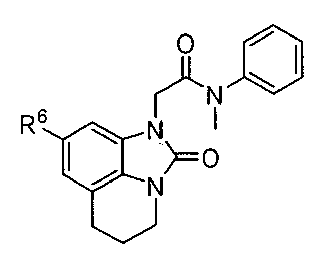

| Ex. No. | R⁶ | IR (cm⁻¹) |
|---|---|---|
| 133 | 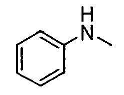 | 974,1124,1493,1668,1691 |
| 134 | 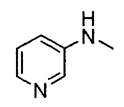 | 1122,1327,1429,1581,1647 |
| 135 | 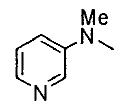 | 1122,1423,1486,1656,1712 |

### Example 136

### N-Methyl-2-(2-oxo-8-phenoxy-5,6-dihydro-4H-imidazo[4,5,1-ij]quinolin-1 (2H)-yl)-N-phenylacetamide

The title compound is obtained from the compound synthesized in Example 111 in a similar manner to Example 50.

IR (cm⁻¹): 957, 1124, 1209, 1423, 1674

### Example 137

### 2-(8-Bromo-2-oxo-5,6-dihydro-4H-imidazo[4,5,1-ij]quinolin-1(2H)-yl)-N-methyl-N-phenylpropanamide

The title compound is obtained from the compound synthesized in Reference Example 20 in a similar manner to Example 4.

¹H-NMR (CDCl₃) δ 1.52 (d, 3H, J=7.1Hz), 2.02 (quintet, 2H, J=5.9Hz), 2.78 (t, 2H, J=6.0Hz), 3.25 (s, 3H), 3.59-3.53 (m, 1H), 3.70-3.64 (m, 1H), 5.23 (q, 1H, J=7.1Hz), 7.00-6.97 (m, 3H), 7.24 (d, 1H, J=1.1Hz), 7.37-7.33 (m,3H),

### Example 138

### 2-(8-Bromo-2-oxo-5,6-dihydro-4H-imidazo[4,5,1-ij]quinolin-1(2H)-yl)-2,2-difluoro-N-methyl-N-phenylacetamide

The title compound is obtained from the compounds synthesized in Reference Example 15 and Reference Example 34 in a similar manner to Reference Example 3.

¹H-NMR (CDCl₃) δ 7.24 (d, 2H, J=7.7Hz), 2.05 (quintet, 2H, J=5.8Hz), 2.75 (t, 2H, J=6.0Hz), 3.40 (s, 3H), 3.77 (t, 2H, J=5.8Hz), 7.00 (s, 2H), 7.10 (t, 1H, J=7.2Hz), 7.19 (t, 3H, J=7.4Hz), 7.24 (d, 2H, J=7.7Hz).

### Example 139

### 2-[8-Bromo-4-(hydroxymethyl)-2-oxo-5,6-dihydro-4H-imidazo[4,5,1-ij]-quinolin-1(2H)-yl]-N-methyl-N-phenylacetamide

To a solution of the compound synthesized in Reference Example 21 (43.5 mg, 154 µmol) in N,N-dimethylformamide (0.50 mL) is added a solution of potassium carbonate (31.9 mg, 230 µmol) and the compound synthesized in Reference Example 33 (45.6 mg, 200 µmol) in N,N-dimethylformamide (0.50 mL) at 20-25°C, and the mixture is stirred at 50°C for 1.5 hour. After the reaction, water is added to the reaction solution, and the mixture is extracted with ethyl acetate/toluene (1/ 1). The organic layer is washed with water and a saturated saline solution, and dried over anhydrous sodium sulfate. The resultant is filtered, and the solvent is evaporated under reduced pressure. The obtained residue is purified by silica gel column chromatography (chloroform/methanol = 30/ 1) to give 2-[8-bromo-4-(hydroxymethyl)-2-oxo-5,6-dihydro-4H-imidazo[4,5,1-ij]quinolin-1(2H)-yl]-N-methyl-N-phenylacetamide (22.7 mg, 34 %).

¹H-NMR (CDCl₃) δ 1.93-1.85 (m, 1H), 2.11-2.04 (m, 1H), 2.87-2.79 (m, 2H), 3.32 (s, 3H), 3.95-3.79 (m, 2H), 4.16-4.08 (m, 1H), 4.34 (s, 2H), 4.97 (dd, 1H, J=9.9Hz, 3.6Hz), 6.87 (s, 1H), 7.02 (d, 1H, J1.4Hz), 7.34 (d, 2H, J=7.2Hz), 7.43 (t, 1H, J=7.4Hz), 7.51 (t, 2H, J=7.8Hz).

### Example 140

### 2-[4-(Hydroxymethyl)-2-oxo-8-phenyl-5,6-dihydro-4H-imidazo[4,5,1-ij]quinolin-1(2H)-yl]-N-methyl-N-phenylacetamide

The title compound is obtained from the compound synthesized in Example 139 in a similar manner to Example 29.

¹H-NMR (CDCl₃) 1.97-1.89 (m, 1H), 2.15-2.09 (m, 1H), 2.97-2.92 (m, 2H), 3.31 (s, 3H), 3.99-3.84 (m, 2H), 4.17-4.11 (m, 1H), 4.43 (s, 2H), 5.23 (dd, 1H, J=10.1Hz, 3.4Hz), 6.91 (s, 1H), 7.09 (d, 1H, J=1.0Hz), 7.35-7.30 (m, 3H), 7.43 (t, 3H, J=4.1Hz), 7.54-7.48 (m, 4H).

### Example 141

### 8-Bromo-N,1-dimethyl-2-oxo-N-phenyl-1,2,5,6-tetrahydro-4H-imidazo-[4,5,1-ij]quinoline-4-carboxamide

The title compound is obtained from the compound synthesized in Reference Example 23 in a similar manner to Example 4.

¹H-NMR (CDCl₃) δ 7.53-7.49 (m, 4H), 1.90-1.81 (m, 1H), 2.18-2.09 (m, 1H), 2.70 (dt, 1H, J=16.4Hz, 4.2Hz), 3.01-2.93 (m, 1H), 4.87 (dd, 1H, J=5.4Hz, 3.3Hz), 6.95 (s, 1H), 7.00 (s, 1H), 7.43-7.39 (m, 1H), 7.53-7.49 (m, 4H).

### Example 142

### N,1-Dimethyl-2-oxo-N-phenyl-1,2,5,6-tetrahydro-4H-imidazo[4,5,1-ij]quinoline-4-carboxamide

The title compound is obtained from the compound synthesized in Example 141 in a similar manner to Example 28.

IR (cm⁻¹): 1005, 1119, 1342, 1659, 1693

### Example 143

### tert-Butyl (1-{2-[methyl(phenyl)amino]-2-oxoethyl}2-oxo-1,2,5,6-tetrahydro-4H-imidazo[4,5,1-ij]quinolin-5-yl)carbamate

The title compound is obtained from the compound synthesized in Reference Example 25 in a similar manner to Example 27.

IR (cm⁻¹): 1166, 1284, 1425, 1496, 1689

### Example 144

### 2-[5-(Diethylamino)-2-oxo-5,6-dihydro-4H-imidazo[4,5,1-ij]quinolin-1(2H)-yl]-N-methyl-N-phenylacetamide

To a solution of the compound synthesized in Example 143 (145 mg, 0.330 mmol) in 1,4-dioxane (0.50 mL) is added a 4N hydrochloric acid/ 1,4-dioxane (0.45 mL), and the mixture is stirred at 50°C for 2.5 hours. After the reaction, the solvent is evaporated under reduced pressure, and the resultant is washed by suspending in diethyl ether. The precipitates are collected by filtration, and dried to give 2-(5-amino-2-oxo-5,6-dihydro-4H-imidazo[4,5,1-ij]quinolin-1(2H)-yl)-N-methyl-N-phenylacetamide hydrochloride (91.0 mg, 73 %).

Subsequently, to a solution of the obtained compound (30.0 mg, 80.0 µmol) in methanol (0.30 mL) are added acetaldehyde (ca. 50 µL) and sodium cyanoborohydride (10.0 mg, 160 µmol), and the mixture is stirred at 20-25°C for 4 hours. To the reaction mixture is added a 10 % aqueous potassium carbonate solution, and the mixture is extracted with ethyl acetate. The organic layer is washed with a saturated saline solution, and dried over anhydrous sodium sulfate. The resultant is filtered, and the solvent is evaporated under reduced pressure to give 2-[5-(diethylamino)-2-oxo-5,6-dihydro-4H-imidazo[4,5,1-ij]quinolin-1(2H)-yl]-N-methyl-N-phenylacetamide (28.0 mg, 91 %).

IR (cm⁻¹): 1390, 1425, 1496, 1654, 1708

The compounds of Examples 145-147 are obtained from the compound synthesized in Reference Example 30 in a similar manner to Example 1 or Example 4.

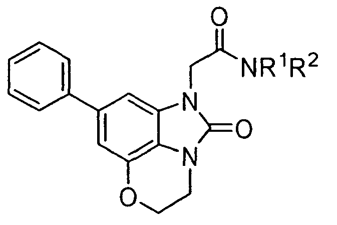

| Ex. No. | R¹ | R² | IR (cm⁻¹) |
|---|---|---|---|
| 145 | Me | Ph | 966,1284,1495,1662,1718 |
| 146 | Me | Bn | 966,1007,1194,1653,1728 |
| 147 | Pr | Pr | 1147,1194,1410,1624,1718 |

The compounds of Examples 148-150 are obtained from the compound synthesized in Reference Example 32 in a similar manner to Example 1 or Example 4.

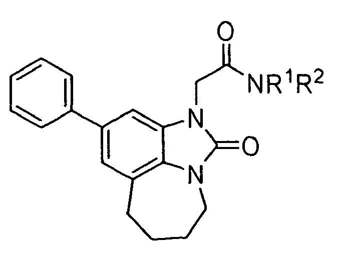

| Ex. No. | R¹ | R² | IR (cm⁻¹) |
|---|---|---|---|
| 148 | Me | Ph | 1122,1265,1425,1660,1705 |
| 149 | Me | Bn | 1119,1352,1483,1655,1705 |
| 150 | Pr | Pr | 1147,1232,1487,1651,1713 |

The compounds of Examples 151-158 are obtained from the compound synthesized in Reference Example 5 in a similar manner to Example 1 or Example 4.

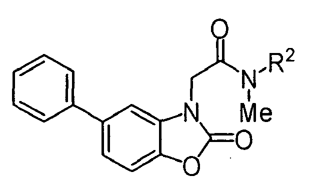

| Ex. No. | R² | IR (cm⁻¹) |
|---|---|---|
| 151 | 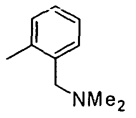 | 694,756,1483,1662,1778 |
| 152 | 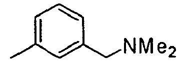 | 698,756,1481,1670,1772 |
| 153 | 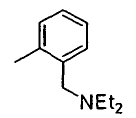 | 692,758,1483,1676,1778 |
| 154 | 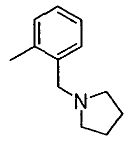 | 692,756,1481,1666,1778 |
| 155 | 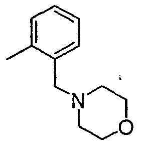 | 692,756,1113,1666,1772 |
| 156 | 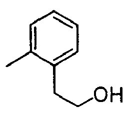 | 692,758,1481,1649,1786 |
| 157 | 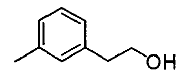 | 690,758,1483,1645,1787 |
| 158 | 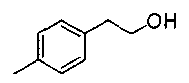 | 687,756,1383,1674,1767 |

The compounds of Examples 159-160 are obtained from the compound synthesized in Reference Example 5 in a similar manner to Example 1 or Example 4.

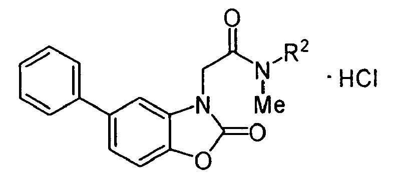

| Ex. No. | R² | IR (cm⁻¹) |
|---|---|---|
| 159 | 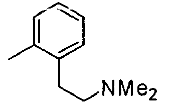 | 692,766,1483,1662,1768 |
| 160 | 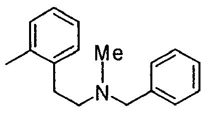 | 694,760,1483,1664,1770 |

The compounds of Examples 161-168 are obtained from the compound synthesized in Reference Example 5 in a similar manner to Example 1 or Example 4.

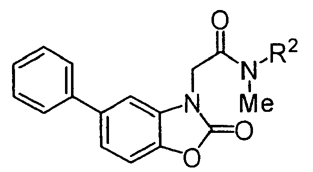

| Ex. No. | R² | IR(cm⁻¹) |
|---|---|---|
| 161 | 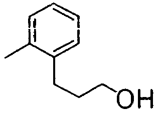 | 692,758,1483,1659,1767 |
| 162 | 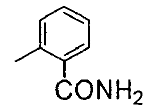 | 692,760,1250,1633,1780 |
| 163 | 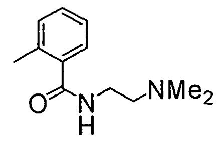 | 692,758,1383,1630,1780 |
| 164 | 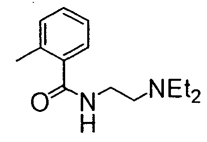 | 696,758,1483,1651,1774 |
| 165 | 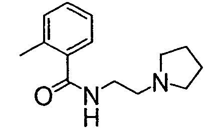 | 696,758,1483,1651,1765 |
| 166 | 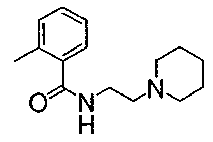 | 692,758,1649,1664,1786 |
| 167 | 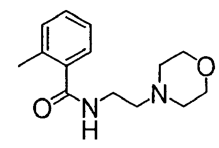 | 692,756,1657,1672,1765 |
| 168 | 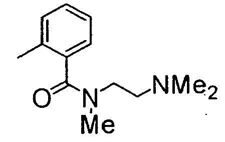 | 760,1381,1485,1624,1776 |

### Example 169

### N-(Hydroxyethyl)-2-(2-oxo-5-phenyl-1,3-benzoxazol-3(2H)-yl)-N-phenylacetamide

The title compound is obtained from the compound synthesized in Reference Example 5 in a similar manner to Example 1.

IR (cm⁻¹): 696, 1022, 1387, 1674, 1780

The compounds of Examples 170-175 are obtained from the compound synthesized in Example 27 in a similar manner to Example 29.

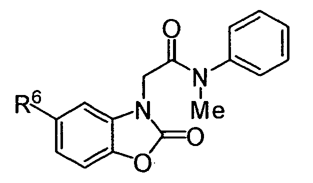

| Ex. No. | R⁶ | IR(cm⁻¹) |
|---|---|---|
| 170 | 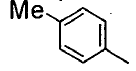 | 704,808,1493,1676,1778 |
| 171 | 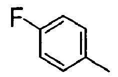 | 700,818,1493,1657,1780 |
| 172 | 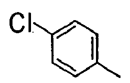 | 696,814,1481,1676,1782 |
| 173 | 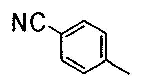 | 696,820,1489,1676,1780 |
| 174 | 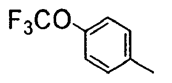 | 692,1151,1489,1662,1774 |
| 175 | 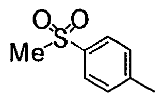 | 660,1151,1306,1668,1770 |

### Example 176

### N-Methyl-2-[2-oxo-5-(1,3-thiazol-5-yl)-1,3-benzoxazol-3(2H)-yl]-N-phenylacetamide

A solution of the compound synthesized in Example 27 (50.0 mg, 138 µmol), 5-(tributylstannyl)-1,3-thiazole (62.2 mg, 166 µmol), tetrakistriphenylphosphine palladium (8.00 mg, 6.92 µmol) in toluene (2.0 mL) is stirred under reflux for 2 hours. After the reaction, the reaction mixture is poured into a mixed solvent of a saturated aqueous sodium hydrogen carbonate solution/ethyl acetate at 0°C, and the mixture is extracted with ethyl acetate. The organic layer is washed with a saturated saline solution, and dried over anhydrous sodium sulfate. The drying agent is filtered off, and the solvent is evaporated under reduced pressure. The obtained residue is purified by silica gel column chromatography (hexane/ethyl acetate = 9/ 11) to give N-methyl-2-[2-oxo-5-(1,3-thiazol-5-yl)-1,3-benzoxazol-3(2H)-yl]-N-phenylacetamide (22.7 mg, 45 %).

IR (cm⁻¹): 696, 1240, 1377, 1660, 1774

### Example 177

### N-Methyl-2-[2-oxo-5-(phenylsulfonyl)-1,3-benzoxazol-3(2H)-yl]-N-phenylacetamide

To a solution of the compound synthesized in Example 27 (361 mg, 1.00 mmol) in dimethylformamide (3.0 mL) are added benzene-sulfinic acid sodium salt (263 mg, 1.00 mmol) and copper iodide (286 mg, 1.50 mmol) at room temperature, and the mixture is stirred at 110-120°C for 18 hours. After the reaction, the reaction mixture is cooled to room temperature, and poured into water. The mixture is extracted with ethyl acetate/toluene (1/1), and the organic layer is washed with water and dried over anhydrous sodium sulfate. The drying agent is removed by filtration, and the solvent is evaporated under reduced pressure, and the obtained crude product is purified by silica gel chromatography (hexane/ethyl acetate = 2/ 1) to give N-methyl-2-[2-oxo-5-(phenylsulfonyl)-1,3-benzoxazol-3(2H)-yl]-N-phenylacetamide (13.0 mg, 3 %).

IR (cm⁻¹): 621, 727, 1306, 1680, 1792

### Example 178

### 2-[5-[4-(Difluoromethoxy)phenyl]-2-oxo-1,3-benzoxazol-3(2H)-yl]-N-methyl-N-phenylacetamide

To a solution of the compound synthesized in Reference Example 36 (50.0 mg, 122 µmol), 1-bromo-4-(difluoromethoxy)benzene (20.1 µL, 147 µmol) and tetrakistriphenylphosphine palladium (7.08 mg, 6.12 µmol) in 1,4-dioxane (2.0 mL) is added a solution of potassium carbonate (50.8 mg, 367 µmol) in water (0.40 mL), and the mixture is stirred under reflux for 2 hours. After the reaction, the reaction mixture is poured into water at 0°C, and extracted with ethyl acetate. The organic layer is washed with a saturated saline solution, and dried over anhydrous sodium sulfate. The drying agent is removed by filtration, and the solvent is evaporated under reduced pressure. The obtained crude product is purified by silica gel chromatography (hexane/ethyl acetate = 3/1) to give 2-[5-[4-(difluoromethoxy)phenyl]-2-oxo-1,3-benzoxazol-3(2H)-yl]-N-methyl-N-phenylacetamide (21.1 mg, 41 %).

IR (cm⁻¹): 696, 1111, 1495, 1674, 1774

The compounds of Examples 179-290 are obtained from the compound synthesized in Reference Example 36 in a similar manner to Example 178.

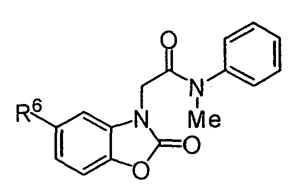

| Ex. No. | R⁶ | IR (cm⁻¹) |
|---|---|---|
| 179 | 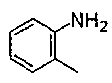 | 694,740,1385,1664,1765 |
| 180 | 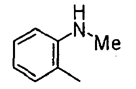 | 692,764,1493,1664,1778 |
| 181 | 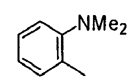 | 700,746,1483,1666,1775 |
| 182 | 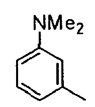 | 690,781,1020,1660,1782 |
| 183 | 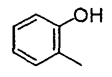 | 694,1254,1670,1757,3304 |
| 184 | 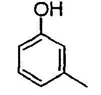 | 700,1113,1595,1767, 3369 |
| 185 | 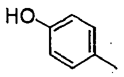 | 698,1227,1493,1670,3296 |
| 186 | 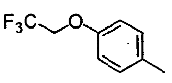 | 661,1163,1238,1680,1772 |
| 187 | 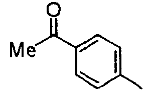 | 708,822,1254,1674,1778 |
| 188 | 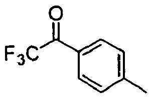 | 694,1151,1660,1705,1782 |
| 189 | 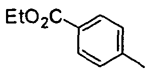 | 694,769,1099,1666,1778 |
| 190 | 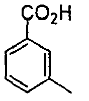 | 694,1385,1483,1660,1778 |
| 191 | 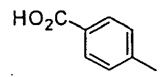 | 769,1105,1236,1711,1782 |
| 192 | 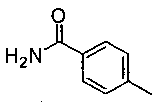 | 706,1489,1657,1678,1770 |
| 193 | 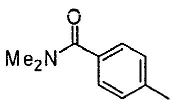 | 766,1387,1487,1659,1786 |
| 194 | 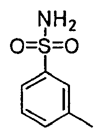 | 690,1169,1385,1657,1763 |
| 195 | 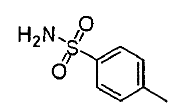 | 815,1165,1325,1655,1782 |
| 196 | 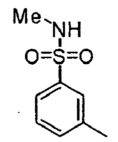 | 696,1325,1479,1657,1751 |
| 197 | 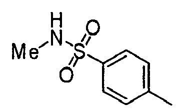 | 692,1163,1319,1655,1778 |
| 198 | 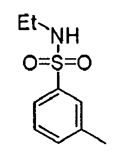 | 700,1153,1335,1659,1768 |
| 199 | 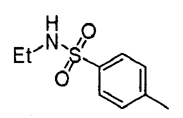 | 663,1313,1487,1659,1784 |
| 200 | 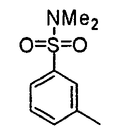 | 715,1167,1336,1660,1782 |
| 201 | 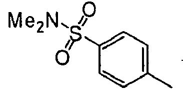 | 696,1163,1331,1672,1782 |
| 202 | 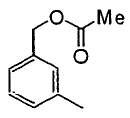 | 692,741,1236,1660,1774 |
| 203 | 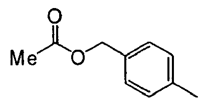 | 694,810,1252,1676,1716 |
| 204 | 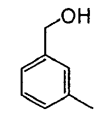 | 700,1387,1487,1659,1763 |
| 205 | 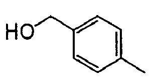 | 694,798,1490,1668,1784 |
| 206 | 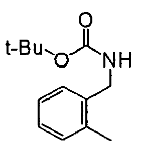 | 702,1244,1485,1662,1772 |
| 207 | 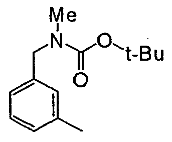 | 748,1144,1481,1666,1778 |
| 208 | 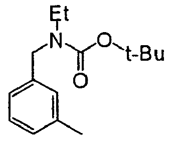 | 752,1144,1481,1674,1776 |
| 209 | 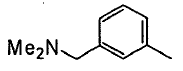 | 692,789,1487,1662,1778 |
| 210 | 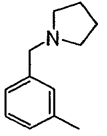 | 692,787,1487,1655,1786 |
| 211 | 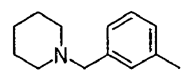 | 694,1020,1383,1662,1774 |
| 212 | 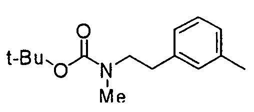 | 702,1165,1485,1670,1780 |
| 213 | 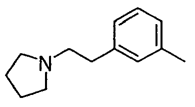 | 692,1383,1485,1662,1778 |
| 214 | 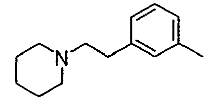 | 692,1117,1485,1664,1778 |
| 215 | 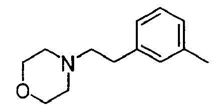 | 694,1115,1481,1670,1774 |
| 216 | 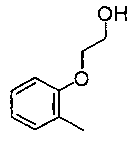 | 694,1244,1485,1653,1780 |
| 217 | 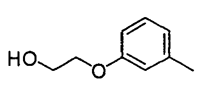 | 700,1246,1485,1662,1782 |
| 218 | 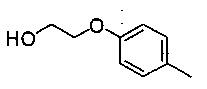 | 702,1043,1240,1674,1774 |
| 219 | 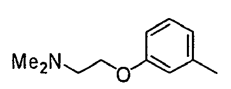 | 692,786,1487,1660,1774 |
| 220 | 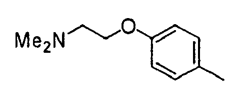 | 692,804,1489,1670,1772 |
| 221 | 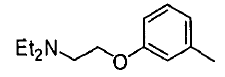 | 696,1242,1483,1672,1774 |
| 222 | 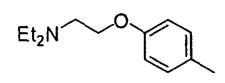 | 692,1234,1489,1672,1774 |
| 223 | 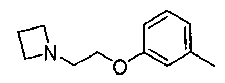 | 746,1242,1483,1670,1774 |
| 224 | 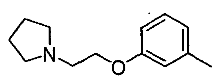 | 694,1018,1483,1670,1774 |
| 225 | 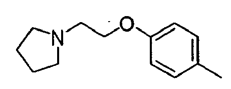 | 692,1236,1489,1672,1774 |
| 226 | 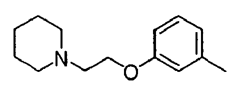 | 700,1126,1600,1651,1787 |
| 227 | 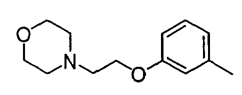 | 696,1115,1483,1670,1774 |
| 228 | 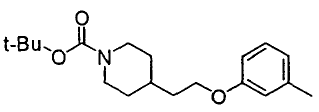 | 698,1167,1242,1670,1776 |
| 229 | 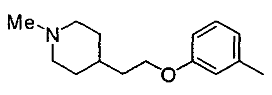 | 694,1020,1485,1664,1778 |
| 230 | 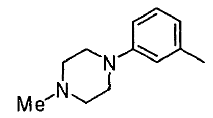 | 694,1242,1489,1660,1774 |
| 231 | 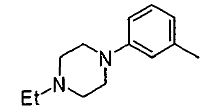 | 694,1383,1489,1659,1784 |
| 232 | 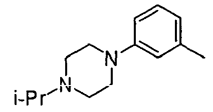 | 696,1244,1385,1659,1784 |
| 233 | 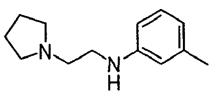 | 696,1485,1599,1670,1772 |
| 234 | 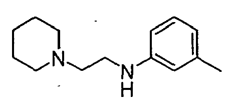 | 694,746,1485,1670,1772 |
| 235 | 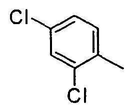 | 698,810,1475,1670,1774 |
| 236 | 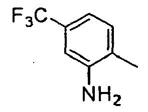 | 696,808,1113,1664,1790 |
| 237 | 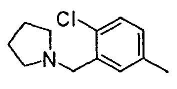 | 694,1020,1383,1668,1774 |
| 238 | 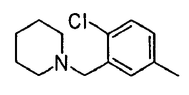 | 694,748,1246,1670,1776 |
| 239 | 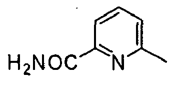 | 872,1120,1389,1651,1778 |
| 240 | 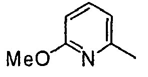 | 793,1252,1458,1668,1778 |
| 241 | 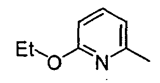 | 791,1246,1437,1659,1774 |
| 242 | 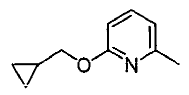 | 793,1250,1443,1668,1776 |
| 243 | 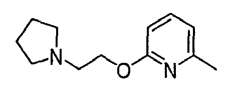 | 795,1254,1443,1670,1778 |
| 244 | 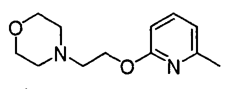 | 796,1254,1443,1668,1778 |
| 245 | 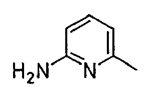 | 698,796,1462,1662,1782 |
| 246 | 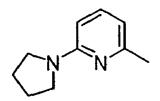 | 779,1387,1456,1664,1774 |
| 247 | 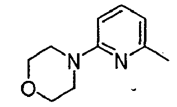 | 696,1246,1441,1668,1774 |
| 248 | 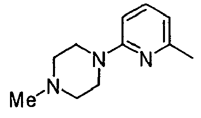 | 789,1254,1444,1674,1755 |
| 249 | 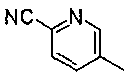 | 696,829,1477,1659,1780 |
| 250 | 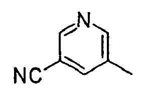 | 688,1022,1246,1657,1778 |
| 251 | 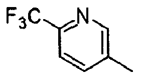 | 704,822,1088,1653,1776 |
| 252 | 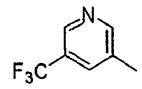 | 700,1020,1132,1655,1784 |
| 253 | 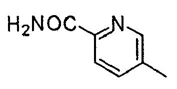 | 708,822,1655,1689,1786 |
| 254 | 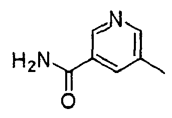 | 696,1387,1662,1753,3203 |
| 255 | 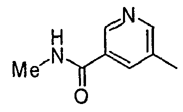 | 704,1383,1498,1662,1788 |
| 256 | 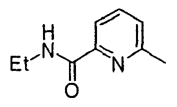 | 700,1022,1497,1659,1780 |
| 257 | 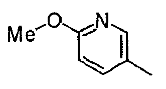 | 692,1281,1485,1664,1774 |
| 258 | 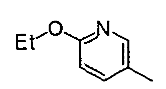 | 694,1284,1479,1662,1759 |
| 259 | 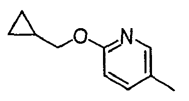 | 692,1282,1483,1662,1778 |
| 260 | 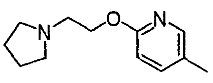 | 746,1281,1481,1670,1774 |
| 261 | 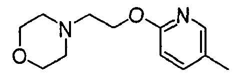 | 820,1290,1485,1655,1776 |
| 262 | 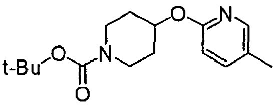 | 694,1022,1277,1672,1780 |
| 263 | 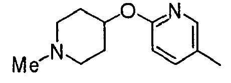 | 694,1041,1279,1479,1772 |
| 264 | 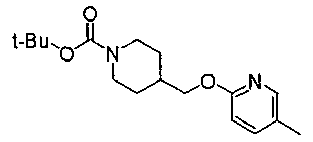 | 694,1018,1483,1670,1778 |
| 265 | 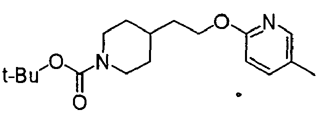 | 694,1165,1483,1670,1778 |
| 266 | 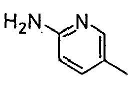 | 694,808,1487,1653,1765 |
| 267 | 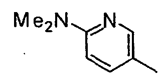 | 696,804,1489,1603,1774 |
| 268 | 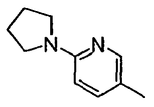 | 812,1493,1595,1672,1767 |
| 269 | 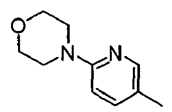 | 806,1117,1487,1670,1778 |
| 270 | 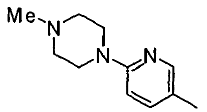 | 744,1235,1485,1670,1778 |
| 271 | 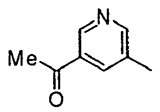 | 706,1020,1639,1682,1786 |
| 272 | 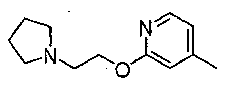 | 694,806,1250,1670,1778 |
| 273 | 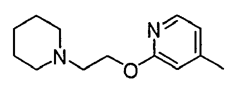 | 694,1014,1250,1670,1780 |
| 274 | 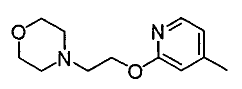 | 694,1115,1250,1668,1778 |
| 275 | 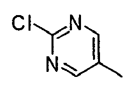 | 696,704,1389,1674,1786 |
| 276 | 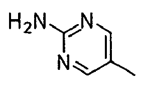 | 694,1024,1479,1655,1778 |
| 277 | 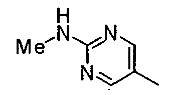 | 692,1117,1606,1759,3244 |
| 278 | 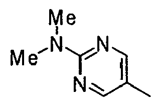 | 696,1383,1408,169,1788 |
| 279 | 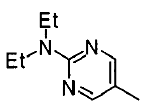 | 696,1020,1527,1601,1778 |
| 280 | 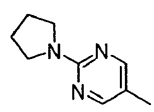 | 696,1246,1527,1597,1765 |
| 281 | 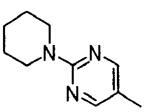 | 698,804,1242,1597,1765 |
| 282 | 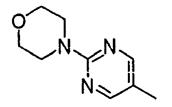 | 700,1240,1473,1676,1782 |
| 283 | 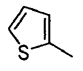 | 696,1014,1495,1664,1774 |
| 284 | 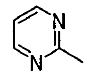 | 696,793,1383,1659,1782 |
| 285 | 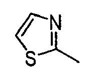 | 696,1014,1381,1662,1774 |
| 286 | 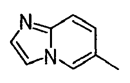 | 690,806,1491,1653,1761 |
| 287 | 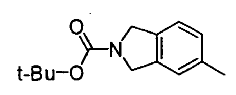 | 698,1101,1398,1686,1772 |
| 288 | 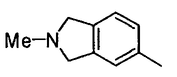 | 806,1016,1381,1659,1784 |
| 289 | 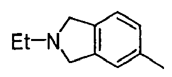 | 806,1385,1487,1657,1784 |
| 290 | 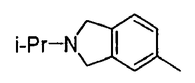 | 750,1385,1485,1674,1776 |

### Example 291

### N-Methyl-2-[2-oxo-5-[5-trifluoromethyl]pyridin-2-yl]-1,3-benzoxazol-3(2H)-yl]-N-phenylacetamide

A solution of palladium acetate (1.10 mg, 4.90 µmol), 2-dicyclohexylphosphino-2'-(N,N-dimethylamino)biphenyl (3.86 mg, 9.80 µmol) in tetrahydrofuran (1.5 mL) is stirred at 20-25°C for 10 minutes, and thereto are added the compound synthesized in Reference Example 36 (100 mg, 245 µmol), 2-chloro-5-trifluoromethylpyridine (53.4 mg, 294 µmol) and cesium fluoride (112 mg, 735 µmol), and the mixture is stirred under reflux for 10 hours. After the reaction, the reaction mixture is poured into a saturated aqueous sodium hydrogen carbonate solution, and extracted with ethyl acetate, and dried over anhydrous sodium sulfate. The drying agent is removed by filtration, and the solvent is evaporated under reduced pressure. To the residue is added methanol, and the precipitated crystals are collected by filtration to give N-methyl-2-[2-oxo-5-[5-trifluoromethyl]pyridin-2-yl]-1,3-benzoxazol-3(2H)-yl]-N-phenylacetamide (38.3 mg, 37 %).

IR (cm⁻¹): 696, 1122, 1319, 1672, 1780

The compounds of Examples 292-294 are obtained from the compound synthesized in Reference Example 36 in a similar manner to Example 291.

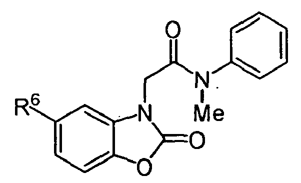

| Ex. No. | R⁶ | IR (cm⁻¹) |
|---|---|---|
| 292 | 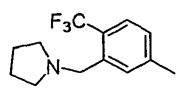 | 694,1109,1309,1670,1776 |
| 293 | 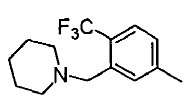 | 694,1109,1311,1674,1778 |
| 294 | 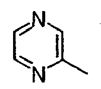 | 704,814,1468,1653,1782 |

### Example 295

### [3-(3-{2-[Methyl(phenyl)amino]-2-oxoethyl}-2-oxo-2,3-dihydro-1,3-benzoxazol-5-yl)phenoxy]acetic acid

In a similar manner to Reference Example 4, the title compound is obtained from tert-butyl [3-(3-{2-[methyl(phenyl)amino]-2-oxoethyl}-2-oxo-2,3-dihydro-1,3-benzoxazol-5-yl)phenoxy]acetate, which is synthesized from the compound obtained in Reference Example 36 in a similar manner to Example 178.

IR (cm⁻¹): 696, 1022, 1365, 1660, 1763

### Example 296

### [4-(3-{2-[Methyl(phenyl)amino]-2-oxoethyl}-2-oxo-2,3-dihydro-1,3-benzoxazol-5-yl)phenoxy]acetic acid

In a similar manner to Reference Example 4, the title compound is obtained from tert-butyl [4-(3-{2-[methyl(phenyl)amino]-2-oxoethyl}-2-oxo-2,3-dihydro-1,3-benzoxazol-5-yl)phenoxy]acetate, which is synthesized from the compound obtained in Reference Example 36 in similar manner to Example 178.

IR (cm⁻¹): 825, 1086, 1198, 1489, 1759

### Example 297

### 2-[5-[3-(Aminoethyl)phenyl]-2-oxo-1,3-benzoxazol-3(2H)-yl]-N-methyl-N-phenylacetamide hydrochloride

In similar manner to Example 38, the title compound is obtained from tert-butyl [3-(3-{2-[methyl(phenyl)amino]-2-oxoethoxy}-2-oxo-2,3-dihydro-1,3-benzoxazol-5-yl)benzyl]carbamate, which is synthesized from the compound obtained in Reference Example 36 in similar manner to Example 178.

IR (cm⁻¹): 694, 1387, 1487, 1643, 1768

### Example 298

### N-Methyl-2-[2-oxo-5-(3-piperazin-1-ylphenyl)-1,3-benzoxazol-3(2H)-yl]-N-phenylacetamide dihydrochloride

In similar manner to Example 38, the title compound is obtained from tert-butyl 4-[3-(3-{2-[methyl(phenyl)amino]-2-oxoethyl}-2-oxo-2,3-dihydro-1,3-benzoxazol-5-yl)phenyl]piperazine-1-carboxylate, which is synthesized from the compound obtained in Reference Example 36 in similar manner to Example 178.

IR (cm⁻¹): 690, 1242, 1493, 1641, 1784

### Example 299

### N-Methyl-2-[2-oxo-5-[3-(2-piperidin-4-ylethoxy)phenyl]-1,3-benzoxazol-3(2H)-yl]-N-phenylacetamide hydrochloride

The title compound is obtained from the compound synthesized in Example 228 in a similar manner to Example 38.

IR (cm⁻¹): 694, 1022, 1379, 1662, 1778

### Example 300

### N-methyl-2-[2-oxo-5-[6-(piperidin-4-yloxy)pyridin-3-yl]-1,3-benzoxazol-3(2H)-yl]-N-phenylacetamide hydrochloride

The title compound is obtained from the compound synthesized in Example 262 in a similar manner to Example 38.

IR (cm⁻¹): 694, 1126, 1248, 1653, 1776

### Example 301

### N-Methyl-2-[2-oxo-5-[6-(piperidin-4-ylmethoxy)pyridin-3-yl]-1,3-benzoxazol-3(2H)-yl]-N-phenylacetamide hydrochloride

The title compound is obtained from the compound synthesized in Example 264 in a similar manner to Example 38.

IR(cm⁻¹): 694, 1244, 1651, 1763, 3365

### Example 302

### N-Methyl-2-[2-oxo-5-[6-(piperidin-4-ylethoxy)pyridin-3-yl]-1,3-benzoxazol-3(2H)-yl]-N-phenylacetamide hydrochloride

The title compound is obtained from the compound synthesized in Example 265 in a similar manner to Example 38.

IR (cm⁻¹): 617, 1248, 1645, 1782, 3358

### Example 303

### 2-[5-(2,3-Dihydro-1H-isoindol-5-yl)-2-oxo-1,3-benzoxazol-3(2H)-yl]-N-methyl-N-phenylacetamide hydrochloride

The title compound is obtained from the compound synthesized in Example 287 in a similar manner to Example 38.

IR (cm⁻¹): 818, 1385, 1489, 1659, 1782

### Example 304

### 2-(5-Bromo-2-oxo-1,3-benzoxazol-3(2H)-yl)-N,N-diisopropylacetamide

The title compound is obtained from the compound synthesized in Reference Example 4 in a similar manner to Example 1.

IR (cm⁻¹): 798, 1020, 1489, 1650, 1782

The compounds of Examples 305-308 are obtained from the compound synthesized in Reference Example 35 in a similar manner to Example 178, Reference Example 4 and Example 1.

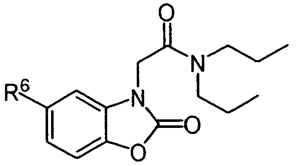

| Ex. No. | R⁶ | IR (cm⁻¹) |
|---|---|---|
| 305 | 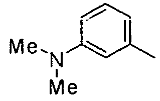 | 702,775,1020,1643,1784 |
| 306 | 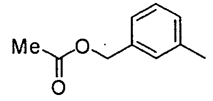 | 704,1020,1643,1733,1778 |
| 307 | 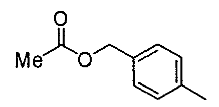 | 611,791,1252,1641,1770 |
| 308 | 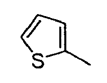 | 683,696,1248,1647,1778 |

The compounds of Examples 309-312 are obtained from the compound synthesized in Reference Example 35 in a similar manner to Example 178, Reference Example 4 and Example 1.

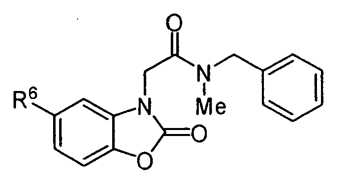

| Ex. No. | R⁶ | IR (cm⁻¹) |
|---|---|---|
| 309 | 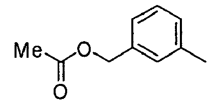 | 698,798,1651,1726,1782 |
| 310 | 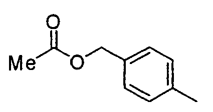 | 696,800,1240,1649,1784 |
| 311 | 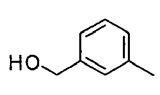 | 787,1012,1487,1651,1780 |
| 312 | 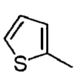 | 683,804,1489,1649,1784 |

The compounds of Examples 313-315 are obtained from the compound synthesized in Reference Example 35 in a similar manner to Example 178, Reference Example 4 and Example 1.

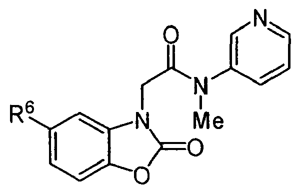

| Ex. No. | R⁶ | IR (cm⁻¹) |
|---|---|---|
| 313 | 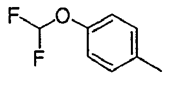 | 717,806,1111,1678,1770 |
| 314 | 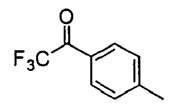 | 714,1140,1178,1668,1790 |
| 315 | 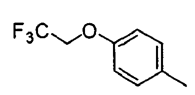 | 665,804,1491,1674,1770 |

### Example 316

### N-[2-(2-Hydroxyethyl)phenyl]-N-methyl-2-[2-oxo-5-[4-(trifluoromethyl)-phenyl]-1,3-benzoxazol-3(2H)-yl]acetarnide

The title compound is obtained from the compound synthesized in Reference Example 3 in a similar manner to Example 29, Reference Example 4 and Example 1.

IR (cm⁻¹): 692, 808, 1491, 1662, 1774

### Example 317

### N-Ethyl-2-[2-oxo-5-[4-(trifluoromethyl)phenyl]-1,3-benzoxazol-3(2H)-yl]-N-pyridin-3-ylacetamide

The title compound is obtained from the compound synthesized in Reference Example 3 in a similar manner to Reference Example 4, Example 1 and Example 29.

IR (cm⁻¹): 717, 827, 1068, 1327, 1670

The compounds of Examples 318-328 are obtained from the compound synthesized in Reference Example 3 in a similar manner to Example 29, Reference Example 4 and Example 1 or Example 4.

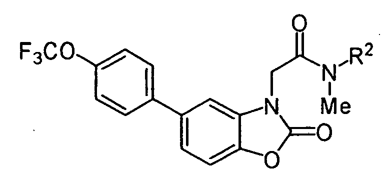

| Ex. No. | R² | IR (cm⁻¹) |
|---|---|---|
| 318 | 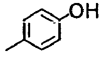 | 804.1207,1248,1651,1770 |
| 319 | 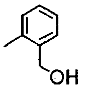 | 692,1207,1489,1659,1774 |
| 320 | 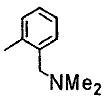 | 692,1205,1250,1659,1774 |
| 321 | 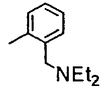 | 692,1157,1250,1662,1778 |
| 322 | 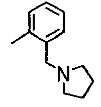 | 692,1158,1250,1659,1776 |
| 323 | 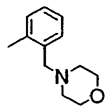 | 806,1119,1207,1659,1778 |
| 324 | 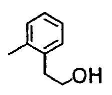 | 692,804,1248,1662,1774 |
| 325 | 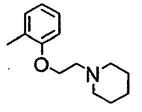 | 750,1205,1493,1659,1787 |
| 326 | 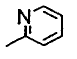 | 787,1151,1254,1668,1761 |
| 327 | 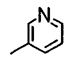 | 712,802,1207,1248,1666 |
| 328 | 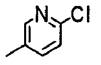 | 804,1248,1460,1676,1770 |

The compounds of Examples 329-330 are obtained from the compound synthesized in Reference Example 3 in a similar manner to Example 29, Reference Example 4 and Example 1 or Example 4.

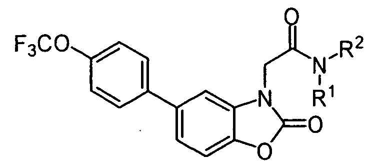

| Ex. No. | R¹ | R² | IR (cm⁻¹) |
|---|---|---|---|
| 329 | 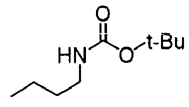 | 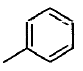 | 804,1248,1489,1670,1778 |
| 330 | Et | 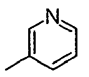 | 698,1140,1241,1670,1780 |

The compounds of Examples 331-344 are obtained from the compound synthesized in Reference Example 3 in a similar manner to Example 29, Reference Example 4 and Example 1.

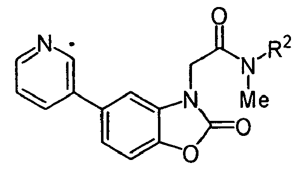

| Ex. No. | R² | IR (cm⁻¹) |
|---|---|---|
| 331 | 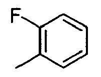 | 762,1022,1483,1670,1765 |
| 332 | 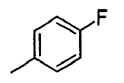 | 802,1248,1510,1660,1776 |
| 333 | 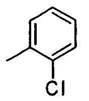 | 688,798,1479,1664,1776 |
| 334 | 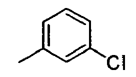 | 702,795,1477,1666,1792 |
| 335 | 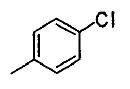 | 712,804,1481,1660,1776 |
| 336 | 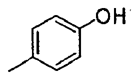 | 692,804,1244,1649,1767 |
| 337 | 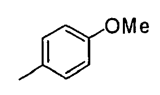 | 690,1022,1248,1655,1772 |
| 338 | 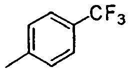 | 696,1120,1323,1674,1768 |
| 339 | 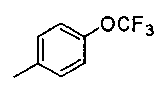 | 690,806,1153,1655,1759 |
| 340 | 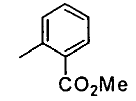 | 700,796,1674,1711,1772 |
| 341 | 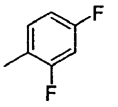 | 795,1024,1510,1662,1768 |
| 342 | 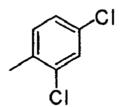 | 806,1020,1479,1670,1772 |
| 343 | 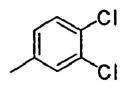 | 808,1120,1471,1655,1761 |
| 344 | 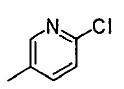 | 798,1103,1458,1668,1768 |

The compounds of Examples 345-346 are obtained from the compound synthesized in Reference Example 3 in a similar manner to Example 29, Reference Example 4 and Example 1.

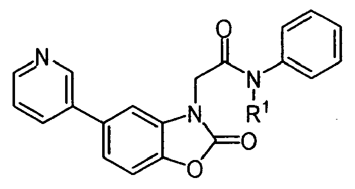

| Ex. No. | R¹ | IR (cm⁻¹) |
|---|---|---|
| 345 | Et | 692,710,1479,1653,1786 |
| 346 | i-Pr | 706,804,1296,1653,1772 |

### Example 347

### 3-[2-(3,4-Dihydroquinolin-1(2H)-yl)-2-oxoethyl]-5-pyridin-3-yl-1,3-benzoxazol-2(3H)-one

The title compound is obtained from the compound synthesized in Reference Example 3 in a similar manner to Example 29, Reference Example 4 and Example 1.

IR (cm⁻¹): 611, 712, 1022, 1639, 1774

The compounds of Examples 348-356 are obtained from the compound synthesized in Reference Example 3 in a similar manner to Example 29, Reference Example 4 and Example 1.

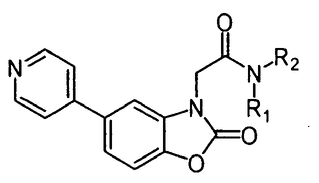

| Ex. No. | R¹ | R² | IR (cm⁻¹) |
|---|---|---|---|
| 348 | Me | 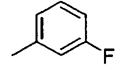 | 685,750,1605,1660,1768 |
| 349 | Me | 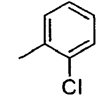 | 771,814,1481,1676,1788 |
| 350 | Me | 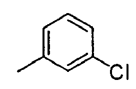 | 687,795,1589,1649,1790 |
| 351 | Me | 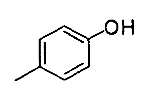 | 690,808,1485,1666,1774 |
| 352 | Me | 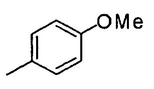 | 795,804,1248,1668,1780 |
| 353 | Me | 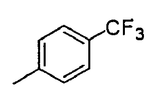 | 808,1068,1117,1331,1780 |
| 354 | Me | 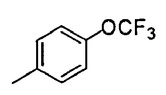 | 692,1161,1483,1674,1782 |
| 355 | Me | 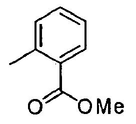 | 625,762,1271,1662,1776 |
| 356 | Et | 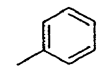 | 690,766,820,1664,1768 |

### Example 357

### 3-[2-(3,4-Dihydroquinolin-1(2H)-yl)-2-oxoethyl]-5-pyridin-4-yl-1,3-benzoxazol-2(3H)-one

The title compound is obtained from the compound synthesized in Reference Example 3 in a similar manner to Example 29, Reference Example 4 and Example 1.

IR (cm⁻¹): 688, 754, 1489, 1637, 1765

### Example 358

### 3-[2-(3,4-Dihydroisoquinolin-2(1H)-yl)-2-oxoethyl]-5-pyridin-4-yl-1,3-benzoxazol-2(3H)-one

The title compound is obtained from the compound synthesized in Reference Example 3 in a similar manner to Example 29, Reference Example 4 and Example 1.

IR (cm⁻¹): 742, 808, 1595, 1639, 1780

### Example 359

### 2-(5-Amino-2-oxo-1,3-benzoxazol-3(2H)-yl)-N-methyl-N-phenylacetamide

The title compound is synthesized from the compound obtained in Example 26 in a similar manner to Example 28.

IR (cm⁻¹): 629, 1115, 1483, 1660, 1763

### Example 360

### 2-[5-[(4-Chlorophenyl)amino]-2-oxo-1,3-benzoxazol-3(2H)-yl]-N-methyl-N-phenylacetamide

To a solution of the compound synthesized in Example 359 (50.0 mg, 168 µmol), 4-chlorophenylboric acid (52.6 mg, 336 µmol), and copper (II) acetate (30.5 mg, 168 µmol) in methylene chloride (1.0 mL) is added triethylamine (46.8 µL, 336 µmol), and the mixture is stirred at 20-25°C for 15 hours. After the reaction, the reaction solution is poured into water, and extracted with ethyl acetate. The organic layer is washed with a saturated saline solution, and dried over anhydrous sodium sulfate. The drying agent is removed by filtration, and the solvent is evaporated under reduced pressure to give 2-[5-[(4-chlorophenyl)amino]-2-oxo-1,3-benzoxazol-3(2H)-yl]-N-methyl-N-phenylacetamide (84.3 mg, 100 %).

IR (cm⁻¹): 695, 808, 1489, 1767, 3388

### Example 361

### N-Methyl-2-[2-oxo-5-{[(4-trifluoromethoxy)phenyl]amino}-1,3-benzoxazol-3(2H)-yl]-N-phenylacetamide

The title compound is obtained from the compound synthesized in Example 359 in a similar manner to Example 360.

IR (cm⁻¹): 692, 1151, 1497, 1662, 1759

### Example 362

### 2-(5-Hydroxy-2-oxo-1,3-benzoxazol-3(2H)-yl)-N-methyl-N-phenylacetamide

1,4-Bis(benzyloxy)-2-nitrobenzene is treated in a similar manner to Example 28, Reference Example 2 to give 5-hydroxy-1,3-benzoxazol-2(3H) one.

To a solution of 5-hydroxy-1,3-benzoxazol-2(3H)-one (302 mg, 2.00 mmol) in dimethylformamide (3.0 mL) are added tert-butyl dimethylsilyl chloride (317 mg, 2.10 mmol) and imidazole (163 mg, 2.40 mmol), and the mixture is stirred at 20-25°C for 3.5 hours. After the reaction, the reaction solution is poured into water at 0°C, and extracted with ethyl acetate/toluene (1/1). The organic layer is washed with water and a saturated saline solution, and dried over anhydrous sodium sulfate. The drying agent is removed by filtration, and the solvent is evaporated under reduced pressure. The obtained crude product is purified by silica gel chromatography (hexane/ethyl acetate = 2/ 1) to give 5-{[tert-butyl(dimethyl)silyl]oxy}-1,3-benzoxazol-2(3H)-one (386 mg, 73 %).

5-{[tert-Butyl(dimethyl)silyl]oxy}-1,3-benzoxazol-2(3H)-one is treated in a similar manner to Example 139 to give 2-[5-{[tert-butyl (dimethyl)silyl]oxy}-2-oxo-1,3-benzoxazol-3(2H)-yl]-N-methyl-N-phenylacetamide.

To a solution of 2-[5-{[tert-butyl(dimethyl)silyl]oxy}-2-oxo-1,3-benzoxazol-3(2H)-yl]-N-methyl-N-phenylacetamide (165 mg, 400 µmol) in tetrahydrofuran (3.0 mL) is added a 12N aqueous hydrochloric acid solution (1.0 mL) at 20-25°C, and the mixture is stirred for one hour. After the reaction, the reaction solution is poured into water at 0°C, and extracted with chloroform. The organic layer is washed with a saturated saline solution, and dried over anhydrous sodium sulfate. The drying agent is removed by filtration, and the solvent is evaporated under reduced pressure to give 2-(5-hydroxy-2-oxo-1,3-benzoxazol-3(2H)-yl)-N-methyl-N-phenylacetamide (116 mg, 97%).

IR (cm⁻¹): 698, 835, 1676, 1743, 3305

The compounds of Examples 363-365 are obtained from the compound synthesized in Example 362 in a similar manner to Example 360.

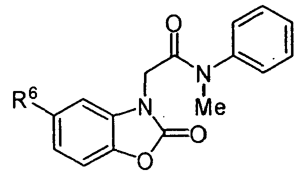

| Ex. No. | R⁶ | IR (cm⁻¹) |
|---|---|---|
| 363 | 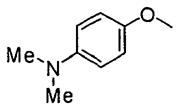 | 700,808,1491,1668,1780 |
| 364 | 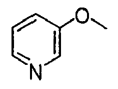 | 694,704,1223,1660,1778 |
| 365 | 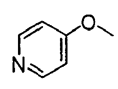 | 698,810,1491,1676,1774 |

The compounds of Examples 366-369 are obtained from the compound synthesized in Reference Example 37 in a similar manner to Example 1 or Example 4.

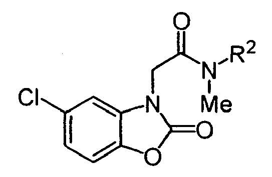

| Ex. No. | R² | IR (cm⁻¹) |
|---|---|---|
| 366 | 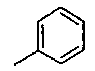 | 688,798,1381,1487,1655 |
| 367 | 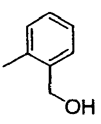 | 690,1491,1655,1790,3385 |
| 368 | 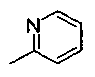 | 787,916,1373,1664,1778 |
| 369 | 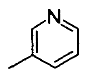 | 669,920,1018,1675,1787 |

The compounds of Examples 370-372 are obtained from the compound synthesized in Reference Example 38 in a similar manner to Example 1 or Example 4.

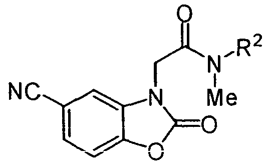

| Ex. No. | R² | IR (cm⁻¹) |
|---|---|---|
| 370 | 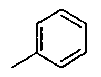 | 710,1252,1493,1668,1776 |
| 371 | 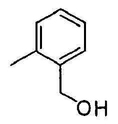 | 690,1012,1491,1655,1797 |
| 372 | 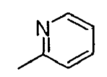 | 742,785,1252,1660,1788 |

### Example 373

### 2-(5-Benzoyl-2-oxo-1,3-benzoxazol-3(2H)-yl)-N-methyl-N-phenylacetamide

The title compound is obtained from 4-hydroxybenzophenone in a similar manner to Reference Example 14, Reference Example 15, Reference Example 2 and Example 139.

IR (cm⁻¹): 688, 1252, 1495, 1653, 1788

### Example 374

### 2-(7-Bromo-2-oxo-1,3-benzoxazol-3(2H)-yl)-N-methyl-N-phenylacetamide

The title compound is obtained from 2-bromo-6-nitrophenol in a similar manner to Reference Example 15, Reference Example 2 and Example 139.

IR (cm⁻¹): 698, 766, 1016, 1670, 1782

The compounds of Examples 375-378 are obtained from the compound synthesized in Reference Example 374 in a similar manner to Example 29.

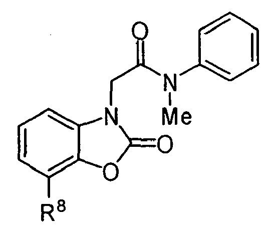

| Ex. No. | R⁸ | IR (cm⁻¹) |
|---|---|---|
| 375 | 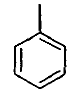 | 696,748,1435,1664,1774 |
| 376 | 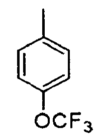 | 696,1158,1252,1668,1774 |
| 377 | 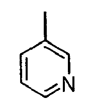 | 700,1007,1464,1664,1772 |
| 378 | 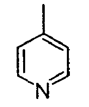 | 640,777,1597,1664,1765 |

### Example 379

### 2-(5-Bromo-7-fluoro-2-oxo-1,3-benzoxazol-3(2H)-yl)-N-methyl-N-phenylacetamide

The title compound is obtained from 4-bromo-2-fluoro-6-nitrophenol in a similar manner to Reference Example 15, Reference Example 2 and Example 139.

IR (cm⁻¹): 638, 684, 1497, 1657, 1784

The compounds of Examples 380-383 are obtained from the compound synthesized in Reference Example 379 in a similar manner to Example 29.

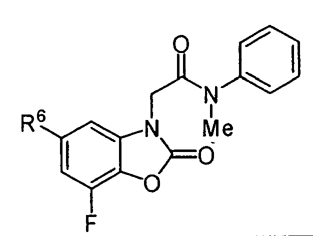

| Ex. No. | R⁶ | IR(cm⁻¹) |
|---|---|---|
| 380 | 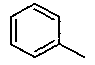 | 646,760,1495,1651,1786 |
| 381 | 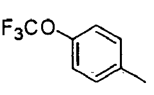 | 694,1259,1497,1660,1782 |
| 382 | 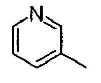 | 700,806,1066,1662,1778 |
| 383 | 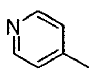 | 700,825,1331,1676,1786 |

### Example 384

### 2-(7-Fluoro-2-oxo-1,3-benzoxazol-3(2H)-yl)-N-methyl-N-phenylacetamide

The title compound is obtained from the compound synthesized in Example 379 in a similar manner to Example 28.

IR (cm⁻¹): 696, 1059, 1483, 1660, 1788

### Example 385

### 2-(5-Bromo-7-chloro-2-oxo-1,3-benzoxazol-3(2H)-yl)-N-methyl-N-phenylacetamide

The title compound is obtained from 4-bromo-2-chlorophenol in a similar manner to Reference Example 14, Reference Example 15, Reference Example 2 and Example 139.

IR (cm⁻¹): 633, 702, 1477, 1659, 1794

The compounds of Examples 386-388 are obtained from the compound synthesized in Reference Example 385 in a similar manner to Example 29.

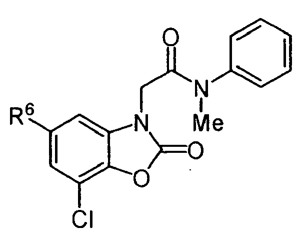

| Ex. No. | R⁶ | IR (cm⁻¹) |
|---|---|---|
| 386 | 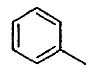 | 696,754,1009,1659,1792 |
| 387 | 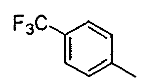 | 705,831,1326,1675,1794 |
| 388 | 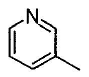 | 640,708,1477,1670,1776 |

### Example 389

### 2-(7-Acetyl-5-bromo-2-oxo-1,3-benzoxazol-3(2H)-yl)-N-methyl-N-phenylacetamide

The title compound is obtained from 1-(5-bromo-2-hydroxy-3-nitrophenyl)ethanone in a similar manner to Reference Example 15, Reference Example 2 and Example 139.

IR (cm⁻¹): 698, 1369, 1464, 1651, 1790

### Example 390

### 2-[7-Acetyl-2-oxo-5-[4-(trifluoromethoxy)phenyl]-1,3-benzoxazol-3(2H)-yl]-N-methyl-N-phenylacetamide

The title compound is obtained from the compound synthesized in Reference Example 389 in a similar manner to Example 29.

IR (cm⁻¹): 696, 1201, 1269, 1666, 1795

### Example 391

### 2-(7-Acetyl-2-oxo-5-pyridin-4-yl-1,3-benzoxazol-3(2H)-yl)-N-methyl-N-phenylacetamide

The title compound is obtained from the compound synthesized in Reference Example 389 in a similar manner to Example 29.

IR (cm⁻¹): 700, 1387, 1595, 1662, 1792

### Example 392

### 2-(7-Acetyl-5-bromo-2-oxo-1,3-benzoxazol-3(2H)-yl)-N-methyl-N-pyridin-3-ylacetamide

The title compound is obtained from 1-(5-bromo-2-hydroxy-3-nitrophenyl)ethanone in a similar manner to Reference Example 15, Reference Examples 2-4 and Example 1.

IR (cm⁻¹): 629, 839, 1373, 1682, 1794

### Example 393

### 2-[7-Acetyl-2-oxo-5-[4-(trifluoromethoxy)phenyl]-1,3-benzoxazol-3(2H)-yl]-N-methyl-N-pyridin-3-ylacetamide

The title compound is obtained from the compound synthesized in Reference Example 392 in a similar manner to Example 29.

IR (cm⁻¹): 629, 1203, 1385, 1672, 1797

### Example 394

### 2-(2-Oxo-5-pyridin-3-yl-1,3-benzothiazol-3(2H)-yl)-N,N-diisopropylacetamide

The title compound is obtained from the compound synthesized in Reference Example 8 in a similar manner to Example 1 and Example 29.

IR (cm⁻¹): 717, 802, 1184, 1639, 1686

### Example 395

### N-Methyl-2-(2-oxo-5-pyridin-4-yl-1,3-benzothiazol-3(2H)-yl)-N-phenylacetamide

The title compound is obtained from the compound synthesized in Reference Example 8 in a similar manner to Example 1 and Example 29.

IR (cm⁻¹): 700, 756, 1493, 1662, 1695

### Example 396

### N-Methyl-2-(2-oxo-7-pyridin-3-yl-1,3-benzothiazol-3(2H)-yl)-N-phenylacetamide

The title compound is obtained from the compound synthesized in Reference Example 9 in a similar manner to Example 1 and Example 29.

IR (cm⁻¹): 702, 773, 1322, 1448, 1664

### Example 397

### N-Methyl-2-[2-oxo-5-(pyridin-3-ylamino)-1,3-benzothiazol-3(2H)-yl]-N-phenylacetamide

The title compound is obtained from the compound synthesized in Reference Example 8 in a similar manner to Example 1 and Example 47.

IR (cm⁻¹): 698, 1120, 1321, 1483, 1662

### Example 398

### tert-Butyl 5-bromo-3-{2-[methyl(phenyl)amino]-2-oxoethyl}-2-oxo-2,3-dihydro-1H-benzimidazole-1-carboxylate

In a similar manner to Example 139, the title compound is obtained from tert-butyl 5-bromo-2-oxo-2,3-dihydro-1H-benzimidazole-1-carboxylate, which is synthesized by the method disclosed in the literature (J. Org. Chem., (1995), 60, 1565-1582).

IR (cm⁻¹): 698, 810, 1145, 1682, 1768

### Example 399

### tert-Butyl 3-{2-[methyl(phenyl)amino]-2-oxoethyl}-2-oxo-5-phenyl-2,3-dihydro- 1H-benzimidazole-1-carboxylate

The title compound is obtained from the compound synthesized in Example 398 in a similar manner to Example 29.

IR (cm⁻¹): 698, 760, 1321, 1672, 1747

### Example 400

### N-Methyl-2-(2-oxo-6-phenyl-2,3-dihydro-1H-benzimidazol-1-yl)-N-phenylacetamide

To a solution of the compound synthesized in Example 399 (1.15 g, 2.52 mmol) in acetic acid (2.50 mL) is added a 4N hydrochloric acid/ 1,4-dioxane (2.50 mL, 10.0 mmol), and the mixture is stirred at 20-25°C for one hour. The reaction solution is concentrated under reduced pressure, and thereto is added toluene, and the mixture is further evaporated under reduced pressure to give N-methyl-2-(2-oxo-6-phenyl-2,3-dihydro-1H-benzimidazol-1-yl)-N-phenylacetamide (943 mg, 100 %).

IR (cm⁻¹): 696, 762, 1483, 1670, 1697

### Example 401

### 2-(3-Isopropyl-2-oxo-6-phenyl-2,3-dihydro-1H-benzimidazol-1-yl)-N-methyl-N-phenylacetamide

To a solution of the compound synthesized in Example 400 (143 mg, 0.400 mmol), 2-propanol (72.0 mg, 1.20 mmol), and triphenylphosphine (157 mg, 0.600 mmol) in tetrahydrofuran (2.5 mL) is added diethyl azodicarboxylate (40 % toluene solution, 261 mg, 0.600 mmol), and the mixture is stirred at 20-25°C for 7 hours. The reaction solution is concentrated under reduced pressure, and the residue is purified by silica gel column chromatography to give 2-(3-isopropyl-2-oxo-6-phenyl-2,3-dihydro-1H-benzimidazol-1-yl)-N-methyl-N-phenylacetamide (77.0 mg, 48 %).

IR (cm⁻¹): 624, 700, 756, 1660, 1707

### Example 402

### 2-(3-Butyl-2-oxo-6-phenyl-2,3-dihydro-1H-benzimidazol-1-yl)-N-methyl-N-phenylacetamide

To a solution of the compound synthesized in Example 400 (143 mg, 0.400 mmol), butanol (89.0 mg, 1.20 mmol), and triphenylphosphine (157 mg, 0.600 mmol) in tetrahydrofuran (2.5 mL) is added diethyl azodicarboxylate (40 % toluene solution, 261 mg, 0.600 mmol), and the mixture is stirred at 20-25°C for 7 hours. The reaction solution is concentrated under reduced pressure, and the residue is purified by silica gel column chromatography to give 2-(3-butyl-2-oxo-6-phenyl-2,3-dihydro-1H-benzimidazol-1-yl)-N-methyl-N-phenylacetamide (104 mg, 63%).

IR (cm⁻¹): 696, 760, 1495, 1659, 1716

### Example 403

### 2-(6-Bromo-2-oxo-2,3-dihydro-1H-indol-1-yl)-N-methyl-N-phenylacetamide

In a similar manner to Example 139, the title compound is obtained from 6-bromo-1,3-dihydro-2H-indol-2-one, which is synthesized by the method disclosed in the literature (Synthesis, (1993), 51-53).

IR (cm⁻¹): 698, 899, 1369, 1662, 1726

### Example 404

### N-Methyl-2-[2-oxo-5-(phenylethynyl)-1,3-benzoxazol-3(2H)-yl]-N-phenylacetamide

A solution of dichlorobisacetonitrile palladium (II) (1.00 mg, 3.00 µmol), 2-dicyclohexylphosphino-2,4,6-triisopropyl-1,1-biphenyl (3.90 mg, 8.30 µmol), cesium carbonate (234 mg, 720 µmol) in acetonitrile (1.0 mL) is prepared under argon atmosphere, and thereto is added the compound synthesized in Example 27 (100 mg, 277 µmol). The reaction mixture is stirred at 20-25°C for 30 minutes, and thereto is added dropwise phenylacetylene (40.0 µL, 360 µmol), and the mixture is stirred at 90°C for 2 hours. The mixture is cooled to room temperature, diluted with water, and extracted with ethyl acetate. The organic layer is washed with a saturated saline solution, and dried over sodium sulfate. The drying agent is removed by filtration, and the solvent is evaporated under reduced pressure. The residue is purified by silica gel column chromatography (hexane/ethyl acetate = 3/1) to give N-methyl-2-[2-oxo-5-(phenylethynyl)-1,3-benzoxazol-3(2H)-yl]-N-phenylacetamide (73.0 mg, 69 %).

IR (cm⁻¹): 694, 1016, 1238, 1670, 1782

### Example 405

### N-Methyl-2-[2-oxo-5-(pyridin-3-ylethynyl)-1,3-benzoxazol-3(2H)-yl]-N-phenylacetamide

The title compound is obtained from the compound synthesized in Example 27 in a similar manner to Example 404.

IR (cm⁻¹): 690, 746, 1497, 1670, 1772

### Example 406

### 2-[5-(3-Hydroxyprop-1-in-1-yl)-2-oxo-1,3-benzoxazol-3(2H)-yl]-N-methyl-N-phenylacetamide

The title compound is obtained from the compound synthesized in Example 27 in a similar manner to Example 404.

IR (cm⁻¹): 700, 1196, 1495, 1632, 3196

### Experiment 1: Benzodiazepine ω3 receptor and benzodiazepine ω1, ω2 receptors binding inhibition test

The benzodiazepine ω3 receptor binding assay was carried out according to the method disclosed in the literature (Mol. Phamacol., 34, 800-805, 1988), and the benzodiazepine ω1, ω2 receptor binding assays were carried out according to the method disclosed in the literature (Neurophamacol., 34, 1169-1175, 1995), respectively.

SD male rats (Japan Charles River) were decapitated, and the kidney and the cerebral cortex were collected. The kidney membrane fraction (ω3) was prepared as follows. The kidney was homogenized with ice-cold 50 mM Tris-HCl buffer (pH 7.6) of about 5-times volume of the wet tissue weight, and the homogenate was centrifuged at 4°C for 10 minutes at 20,000g. The pellets thus obtained were suspended again, and centrifuged at 4°C for 10 minutes at 20,000g. These resuspension and centrifugation procedures were repeated once more, and the obtained pellets were diluted in a 50 mM Tris-HCl buffer (pH 7.6) into a concentration of 0.1 mg of protein per one assay of the receptor binding test. The cerebral cortex membrane fraction (ω1 and ω2) was prepared as follows. The cerebral cortex was homogenized with ice-cold potassium phosphate buffer (200 mM KCl, 20 mM KOH, 20 mM KH₂PO₄, pH 7.4) of 15-times volume of the wet tissue weight, and the homogenate was centrifuged at 4°C for 15 minutes at 32,500g. The obtained pellets were suspended again and centrifuged at 4°C for 15 minutes at 32,500g. These resuspension and centrifugation procedures were repeated once more, and the obtained pellets were diluted in the potassium phosphate buffer into a concentration of 0.1 mg of protein per one assay of the receptor binding test. Both of these membrane fractions were frozen and stored at -80°C.

As the [³H]-labeled ligand, [³H]-PK-11195 (for ω3; PerkinElmer) or [³H]-Ro-15-1788 [Flumazenil] (for ω1 and ω2; PerkinElmer) was used. As a non-labeled ligand, PK-11195 (for ω3; Sigma-Aldrich Corporation) or Flumazenil (for ω1 and ω2; Sigma-Aldrich Corporation) was used. In order to obtain total binding in the binding inhibition assay, a 0.5 % DMSO, the [³H] labeled ligand (final concentration: 1 nM), and the membrane fraction were mixed (total volume: 1 mL), and the mixture was incubated at 4°C (for ω3) or at 25°C (for ω1 and ω2) for one hour. In order to obtain non-specific binding, the non-labeled ligand (final concentration: 10 µM) was added instead of 0.5 % DMSO, and in order to study the binding affinity of the present compounds, a solution of the present compound (final concentration: 100 nM for ω3, or 10 µM for ω1 and ω2) in DMSO was added. After the one-hour incubation, the labeled ligand bound to the receptor was collected by filtration with suction through a 0.3 % polyethyleneimine-treated GF/B filter using a cell harvester, and washed with ice-cold 50 mM Tris-HCl buffer (for ω3) or ice-cold potassium phosphate buffer (for ω1 and ω2) once. The radioactivity on the GF/B filter was measured with liquid scintillation counter (manufactured by Packard, Tri Carb 2700TR). The results were expressed by the inhibition rate (%) against the binding to the labeled ligand.

The experiment as described in Experiment 1 was performed on the compounds obtained in Examples. The results of the benzodiazepine ω3 receptor binding inhibition assay are shown in Table 1. With regard to all of the compounds of Examples as listed in Table 1, benzodiazepine ω1 and ω2 receptor binding inhibitory rate was not more than 60 %, when a 10 µM DMSO solution thereof was used.

**Table 1**

| Compound (Example No.) | [³H]-PK-11195 Binding inhibitory rate (%) |
|---|---|
| 5 | 98 |
| 18 | 71 |
| 29 | 79 |
| 55 | 98 |
| 56 | 99 |
| 60 | 100 |
| 61 | 100 |
| 67 | 53 |
| 96 | 95 |
| 113 | 93 |
| 135 | 86 |
| 136 | 95 |
| 137 | 83 |
| 142 | 83 |
| 147 | 100 |
| 150 | 97 |
| 161 | 96 |
| 185 | 97 |
| 195 | 91 |
| 213 | 71 |
| 327 | 88 |
| 350 | 80 |
| 369 | 85 |
| 373 | 89 |
| 389 | 97 |
| 395 | 95 |
| 401 | 88 |

### Experiment 2: Isoniazid-induced convulsion test (anti-convulsant effect)

The antagonistic effect of the present compounds on the Isoniazid-induced convulsion test was measured according to the method disclosed in the literature (J. Pharmacol. Exp. Ther., 26, 649-656, 1993).

Isoniazid inhibits glutamate decarboxylase that catalyzes GABA biosynthesis, decreases brain GABA levels, and induces systemic convulsion due to GABA depletion at the GABA neuron terminus. Therefore, drugs directly or indirectly enhancing GABA_{A} receptor function such as benzodiazepine receptor agonists, neurosteroids such as allopregnanolone, and benzodiazepine ω3 receptor agonists, which enhance the synthesis of neurosteroids, are known to exhibit antagonistic activity against this systemic convulsion.

In this experiment, male mice of ddY strain (5 weeks old, Japan SLC Inc.) were used in a group of 5. Twenty minutes after interperitoneal administration of the present compounds at a dose of 30 mg/kg in a suspension in 0.5 % methyl cellulose, mice were injected with isoniazid (manufactured by Sumitomo Pharmaceuticals Co., Ltd., 300 mg/kg) subcutaneously, and immediately thereafter, the mice were placed individually in plastic observation cages. The onset time of systemic clonic convulsion and tonic convulsion was measured and recorded after the Isoniazid-administration. The average onset time of the mice treated with each compound was calculated and expressed by a percentage (%) against to the onset time of the mice in the vehicle-treated group.

The experiment as described in Experiment 2 was performed on the compounds obtained in Examples. The results thereof are shown in Table 2.

**Table 2**

| Compound (Example No.) | Onset time of convulsion (%) |
|---|---|
| 18 | 194 |
| 96 | 152 |

### INDUSTRIAL APPLICABILITY

It is found that the compounds of the present invention have a selective and high affinity for benzodiazepine ω3 receptor. Accordingly, the present invention can provide a novel agent for treatment or prevention of central nervous diseases including the symptoms of depression or anxiety.

## Claims

1. An antianxiety or antidepressant agent comprising a compound of the formula (1): 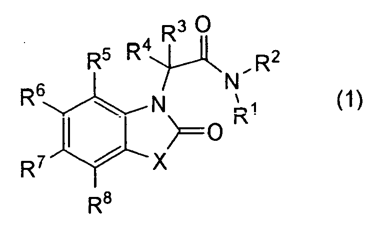 wherein R¹ and R² are independently a hydrogen atom, an optionally substituted alkyl group, an optionally substituted cycloalkyl group, an optionally substituted alkenyl group, an optionally substituted alkynyl group, an optionally substituted aryl group, an optionally substituted heteroaryl group, or an optionally substituted saturated heterocyclic group, or R¹ and R² combine together with the adjacent nitrogen atom to which they bond, and form an optionally substituted saturated heterocyclic group;
R³ and R⁴ are independently a hydrogen atom, a halogen atom, an optionally substituted alkyl group, an optionally substituted cycloalkyl group, an optionally substituted alkenyl group, or an optionally substituted alkynyl group;
R⁵, R⁶, R⁷ and R⁸ are independently a hydrogen atom, an optionally substituted alkyl group, an optionally substituted cycloalkyl group, an optionally substituted alkenyl group, an optionally substituted alkynyl group, a halogen atom, a cyano group, a nitro group, a hydroxy group, an optionally substituted amino group, an optionally substituted alkoxy group, an optionally substituted alkanoyl group, an optionally substituted alkoxycarbonyl group, an optionally substituted aryloxycarbonyl group, an optionally substituted heteroaryloxycarbonyl group, a carboxyl group, an optionally substituted carbamoyl group, an optionally substituted sulfamoyl group, an optionally substituted ureido group, an optionally substituted alkylthio group, an optionally substituted alkylsulfinyl group, an optionally substituted alkylsulfonyl group, or a group of the formula: -E-A (in which E is a single bond, an oxygen atom, a sulfur atom, -SO-, -SO₂-, -NR⁹- or -CO-, A is an optionally substituted aryl group or an optionally substituted heteroaryl group, and R⁹ is a hydrogen atom or an optionally substituted alkyl group);
X is an oxygen atom, a sulfur atom, NR¹⁰, or CR¹¹R¹² (in which R¹⁰ is a hydrogen atom, an optionally substituted alkyl group, an optionally substituted cycloalkyl group, an optionally substituted alkenyl group, an optionally substituted alkynyl group, an optionally substituted alkanoyl group, or an optionally substituted alkoxycarbonyl group, R¹¹ and R¹² are independently a hydrogen atom, an optionally substituted alkyl group, an optionally substituted cycloalkyl group, an optionally substituted alkenyl group, an optionally substituted alkynyl group, an optionally substituted aryl group, an optionally substituted heteroaryl group, a halogen atom, a cyano group, a hydroxy group, an optionally substituted amino group, an optionally substituted alkoxy group, an optionally substituted aryloxy group, an optionally substituted alkanoyl group, an optionally substituted aroyl group, an optionally substituted heteroarylcarbonyl group, an optionally substituted alkoxycarbonyl group, a carboxyl group, or an optionally substituted carbamoyl group, or R¹¹ and R¹² combine each other and form an oxo group or =NOH);
alternatively,
(i) when X is NR¹⁰, then by combining R⁸ and R¹⁰, the formula (1) may be expressed by the formula (2): 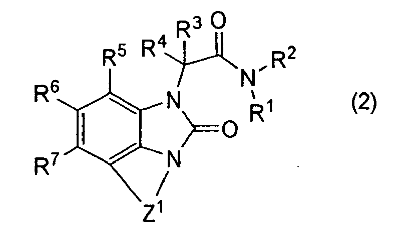 wherein R¹, R², R³, R⁴, R⁵, R⁶ and R⁷ are as defined above, and Z¹ is an optionally substituted alkylene group, and one of the carbon atoms of said alkylene group can be replaced by an oxygen atom, a sulfur atom or -NR¹³- (in which R¹³ is a hydrogen atom or an optionally substituted alkyl group), and a double bond may be formed between any adjacent atoms of said alkylene group;
(ii) by combining R⁴ and R⁵, the formula (1) may be expressed by the formula (3): 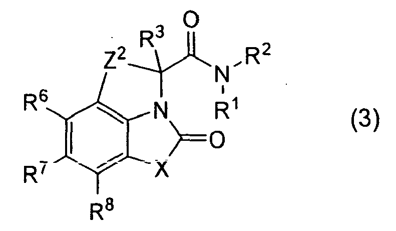
wherein R¹, R², R³, R⁶, R⁷, R⁸ and X are as defined above, Z² is an optionally substituted alkylene group, and one of the carbon atoms of said alkylene group can be replaced by an oxygen atom, a sulfur atom or -NR¹³- (in which R¹³ is a hydrogen atom or an optionally substituted alkyl group), and a double bond may be formed between any adjacent atoms of said alkylene group;
provided that
(1) when X is an oxygen atom or a sulfur atom under the following conditions (a) or (b), then R¹ and R² never form an optionally substituted saturated heterocyclic group by combining together with the adjacent nitrogen atom to which they bond;
(a) all of R⁵, R⁶, R⁷ and R⁸ are a hydrogen atom;
(b) one or two groups of R⁵, R⁶, R⁷ and R⁸ are independently a halogen atom, and the remaining groups are a hydrogen atom;
(2) when X is CR¹¹R¹², and R¹¹ and R¹² are independently an optionally substituted alkyl group, an optionally substituted cycloalkyl group, an optionally substituted alkenyl group, or an optionally substituted alkynyl group under the following conditions (a) or (b), then R¹ and R² are not a hydrogen atom nor an optionally substituted alkyl group, or R¹ and R² never form an optionally substituted saturated heterocyclic group by combining together with the adjacent nitrogen atom;
(a) all of R⁵, R⁶, R⁷ and R⁸ are a hydrogen atom;
(b) one or two groups of R⁵, R⁶, R⁷ and R⁸ are independently a halogen atom, an optionally substituted alkyl group or a nitro group, and the remaining groups are a hydrogen atom, or a prodrug thereof, or a pharmaceutically acceptable salt thereof.

2. A compound of the formula (1'): 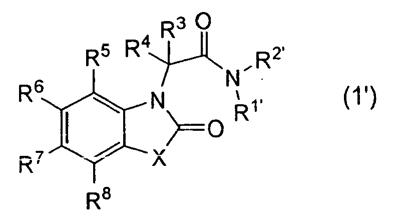 wherein R^{1'} and R^{2'} are independently a hydrogen atom, an optionally substituted alkyl group, an optionally substituted cycloalkyl group, an optionally substituted alkenyl group, an optionally substituted alkynyl group, an optionally substituted aryl group, an optionally substituted heteroaryl group, or an optionally substituted saturated heterocyclic group, or R^{1'} and R^{2'} combine together with the adjacent nitrogen atom to which they bond, and form a group of the formula (4): 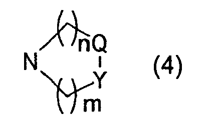 (in which n is 0 or 1, m is 1, 2 or 3, Y is a single bond, an oxygen atom or a sulfur atom, Q is methylene, ethylene, or an optionally substituted o-phenylene group);
R³ and R⁴ are independently a hydrogen atom, a halogen atom, an optionally substituted alkyl group, an optionally substituted cycloalkyl group, an optionally substituted alkenyl group, or an optionally substituted alkynyl group;
R⁵, R⁶, R⁷ and R⁸ are independently a hydrogen atom, an optionally substituted alkyl group, an optionally substituted cycloalkyl group, an optionally substituted alkenyl group, an optionally substituted alkynyl group, a halogen atom, a cyano group, a nitro group, a hydroxy group, an optionally substituted amino group, an optionally substituted alkoxy group, an optionally substituted alkanoyl group, an optionally substituted alkoxycarbonyl group, an optionally substituted aryloxycarbonyl group, an optionally substituted heteroaryloxycarbonyl group, a carboxyl group, an optionally substituted carbamoyl group, an optionally substituted sulfamoyl group, an optionally substituted ureido group, an optionally substituted alkylthio group, an optionally substituted alkylsulfinyl group, an optionally substituted alkylsulfonyl group, or a group of the formula: -E-A (in which E is a single bond, an oxygen atom, a sulfur atom, -SO-, -SO₂-, -NR⁹- or -CO-, A is an optionally substituted aryl group or an optionally substituted heteroaryl group, and R⁹ is a hydrogen atom or an optionally substituted alkyl group);
X is an oxygen atom, a sulfur atom, NR¹⁰, or CR¹¹R¹² (in which R¹⁰ is a hydrogen atom, an optionally substituted alkyl group, an optionally substituted cycloalkyl group, an optionally substituted alkenyl group, an optionally substituted alkynyl group, an optionally substituted alkanoyl group, or an optionally substituted alkoxycarbonyl group, R¹¹ and R¹² are independently a hydrogen atom, an optionally substituted alkyl group, an optionally substituted cycloalkyl group, an optionally substituted alkenyl group, an optionally substituted alkynyl group, an optionally substituted aryl group, an optionally substituted heteroaryl group, a halogen atom, a cyano group, a hydroxy group, an optionally substituted amino group, an optionally substituted alkoxy group, an optionally substituted aryloxy group, an optionally substituted alkanoyl group, an optionally substituted aroyl group, an optionally substituted heteroarylcarbonyl group, an optionally substituted alkoxycarbonyl group, a carboxyl group, or an optionally substituted carbamoyl group, or R¹¹ and R¹² combine and form an oxo group or =NOH);
Alternatively,
(i) when X is NR¹⁰, then by combining R⁸ and R¹⁰, the formula (1') may be expressed by the formula (2'): 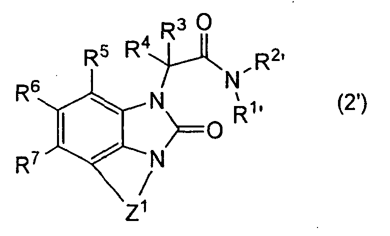 wherein R^{1'}, R^{2'}, R³, R⁴, R⁵, R⁶ and R⁷ are as defined above, Z¹ is an optionally substituted alkylene group, and one of the carbon atoms of said alkylene group can be replaced by an oxygen atom, a sulfur atom or -NR¹³- (in which R¹³ is a hydrogen atom or an optionally substituted alkyl group), and a double bond may be formed between any adjacent atoms of said alkylene group;
(ii) by combining R⁴ and R⁵, the formula (1') may be expressed by the formula (3'): 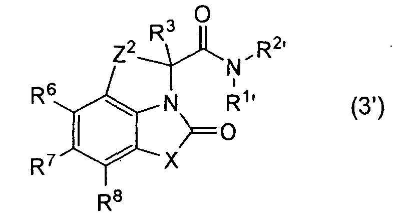
wherein R^{1'}, R^{2'}, R³, R⁶, R⁷, R⁸ and X are as defined above, Z² is an optionally substituted alkylene group, and one of the carbon atoms of said alkylene group can be replaced by an oxygen atom, a sulfur atom or -NR¹³- (in which R¹³ is a hydrogen atom or an optionally substituted alkyl group), and a double bond may be formed between any adjacent atoms of said alkylene group,
provided that in cases other than the above (i) or (ii),
(1) R^{1'} and R^{2'} are not simultaneously a hydrogen atom,
(2) R^{1'} or R^{2'} is not a saturated heterocyclic group,
(3) when R^{1'} and R^{2'} combine together with the adjacent nitrogen atom to which they bond and form a group of the formula (4), then Q is an optionally substituted o-phenylene group,
(4) R⁵, R⁶, R⁷ and R⁸ are not simultaneously a hydrogen atom,
(5) when one or two groups of R⁵, R⁶, R⁷ and R⁸ are independently a halogen atom or an optionally substituted alkyl group, then the remaining groups are not a hydrogen atom,
(6) when X is a sulfur atom, and one or two groups of R⁵, R⁶, R⁷ and R⁸ are independently a halogen atom, a nitro group, an alkyl group, a halogen-substituted alkyl group, an alkoxy group, or an optionally substituted amino group, then the remaining groups are not a hydrogen atom,
(7) when X is an oxygen atom, and one or two groups of R⁵, R⁶, R⁷ and R⁸ are independently a halogen atom, an alkoxy group, or an optionally substituted arylcarbonyl group, and the remaining groups are a hydrogen atom, then R^{1'} or R^{2'} is not a hydrogen atom,
(8) when X is an oxygen atom, R⁷ is a nitro group, and R⁵, R⁶ and R⁸ are a hydrogen atom, then R^{1'} and R^{2'} are not simultaneously an alkyl group,
(9) when X is NR¹⁰, and one or two groups of R⁵, R⁶, R⁷ and R⁸ are independently an optionally substituted alkyl group, an optionally substituted alkoxy group, a halogen atom, or a cyano group, then the remaining groups are not a hydrogen atom,
(10) when X is CR¹¹R¹², then R¹¹ and R¹² are independently a hydrogen atom, an alkyl group optionally substituted by a halogen atom, an optionally substituted cycloalkyl group, an optionally substituted alkenyl group, or an optionally substituted alkynyl group, or R¹¹ and R¹² combine each other and form an oxo group or =NOH, and R^{1'} or R^{2'} is not a hydrogen atom, or a prodrug thereof, or a pharmaceutically acceptable salt thereof.

3. The compound according to claim 2, wherein in cases where the formula (1') in claim 2 is not expressed by the formula (2') or the formula (3'), and further
(11) when one or two groups of R⁵, R⁶, R⁷ and R⁸ are independently a halogen atom, an optionally substituted alkyl group, an optionally substituted pyrimidylamino group or an optionally substituted thiazolyl group, then the remaining groups are not a hydrogen atom,
(12) when X is a sulfur atom, and one or two groups of R⁵, R⁶, R⁷ and R⁸ are independently a halogen atom, a nitro group, an alkyl group, a haloalkyl group, an optionally substituted alkoxy group, or an optionally substituted amino group, then the remaining groups are not a hydrogen atom,
(13) when X is an oxygen atom, one or two groups of R⁵, R⁶, R⁷ and R⁸ are independently a halogen atom, an optionally substituted alkoxy group, or an optionally substituted arylcarbonyl group, and the remaining groups are a hydrogen atom, then R^{1'} or R^{2'} is not a hydrogen atom,
(14) when X is NR¹⁰, one or two groups of R⁵, R⁶, R⁷ and R⁸ are independently an optionally substituted heteroaryl group, and the remaining groups are a hydrogen atom, then R^{1'} or R^{2'} is not a hydrogen atom,
or a prodrug thereof, or a pharmaceutically acceptable salt thereof.

4. The compound according to claim 2, wherein X is NR¹⁰, and R⁸ and R¹⁰ combine each other, and thereby said compound is expressed by the formula (2"): 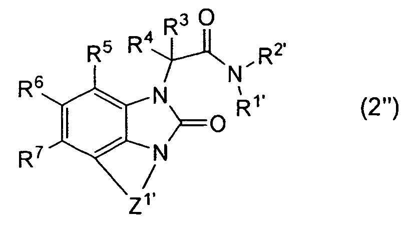 in which R^{1'}, R^{2'}, R³, R⁴, R⁵, R⁶ and R⁷ are as defined in claim 2, and Z^{1'} is an optionally substituted alkylene group, and one of the carbon atoms of said alkylene group can be replaced by an oxygen atom, a sulfur atom or -NR¹³- (in which R¹³ is a hydrogen atom or an optionally substituted alkyl group), or a prodrug thereof, or a pharmaceutically acceptable salt thereof.

5. The compound according to claim 4, wherein at least one of R⁵, R⁶ and R⁷ is -E-A (in which E and A are as defined in claim 2), or a prodrug thereof, or a pharmaceutically acceptable salt thereof.

6. The compound according to claim 4 or 5, wherein Z^{1'} is an optionally substituted trimethylene or tetramethylene, and one of the carbon atoms of said trimethylene and tetramethylene can be replaced by an oxygen atom, or a prodrug thereof, or a pharmaceutically acceptable salt thereof.

7. The compound according to claim 2, wherein R⁴ and R⁵ combine each other, and thereby said compound is expressed by the formula (3"): 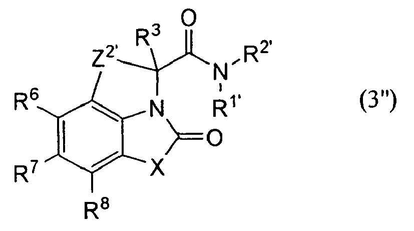 in which R^{1'}, R²', R³, R⁶, R⁷, R⁸ and X are as defined in claim 2, Z²' is an optionally substituted alkylene group, and one of the carbon atoms of said alkylene group can be replaced by an oxygen atom, or a prodrug thereof, or a pharmaceutically acceptable salt thereof.

8. The compound according to claim 7, wherein at least one of R⁶, R⁷ and R⁸ is -E-A (in which E and A are as defined in claim 2), or a prodrug thereof, or a pharmaceutically acceptable salt thereof.

9. The compound according to claim 7 or 8, wherein Z^{2'} is an optionally substituted ethylene group, or a prodrug thereof, or a pharmaceutically acceptable salt thereof.

10. The compound according to claim 2 or 3, wherein R^{1'} is a hydrogen atom, an optionally substituted alkyl group, an optionally substituted cycloalkyl group, an optionally substituted alkenyl group, or an optionally substituted alkynyl group, R^{2'} is an optionally substituted alkyl group, an optionally substituted aryl group, or an optionally substituted heteroaryl group, or R^{1'} and R^{2'} combine together with the nitrogen atom to which they bond, and form a group of the formula (4'): 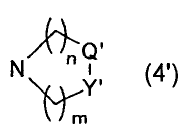 (in which n is 0 or 1, m is 1, 2 or 3, Y' is a single bond or an oxygen atom, and Q' is an optionally substituted o-phenylene group);
R³ and R⁴ are independently a hydrogen atom, a halogen atom, or an optionally substituted alkyl group;
at least one of R⁵, R⁶, R⁷ and R⁸ is a group of the formula: -E-A (in which E and A are as defined in claim 2), or a prodrug thereof, or a pharmaceutically acceptable salt thereof.

11. The compound according to claim 10, wherein X is an oxygen atom or a sulfur atom, or a prodrug thereof, or a pharmaceutically acceptable salt thereof.

12. The compound according to claim 10, wherein X is NR¹⁰, and R¹⁰ is a hydrogen atom or an optionally substituted alkyl group, or a prodrug thereof, or a pharmaceutically acceptable salt thereof.

13. The compound according to claim 10, wherein X is CR¹¹R¹², and R¹¹ and R¹² are independently a hydrogen atom, an alkyl group optionally substituted by a halogen atom, an optionally substituted cycloalkyl group, an optionally substituted alkenyl group, or an optionally substituted alkynyl group, or a prodrug thereof, or a pharmaceutically acceptable salt thereof.

14. The compound according to claim 2 or 3, wherein R^{1'} and R^{2'} are a hydrogen atom or an optionally substituted alkyl group, R⁵, R⁶, R⁷ and R⁸ are independently an alkyl group substituted by a hydroxy group, a nitro group, a cyano group, an alkoxy group, a cycloalkyl group, an optionally substituted amino group, an alkylsulfonyl group, an arylsulfonyl group, or an optionally substituted heteroaryl group; an optionally substituted cycloalkyl group; an optionally substituted alkenyl group; an optionally substituted alkynyl group; a hydroxy group; a substituted amino group; a substituted alkoxy group; an optionally substituted alkanoyl group; an optionally substituted alkoxycarbonyl group; an optionally substituted aryloxycarbonyl group; an optionally substituted heteroaryloxycarbonyl group; a carboxyl group; an optionally substituted carbamoyl group; an aryl-substituted sulfamoyl group; an optionally substituted ureido group; an optionally substituted alkylthio group; an optionally substituted alkylsulfinyl group; an optionally substituted alkylsulfonyl group; or a group of the formula: -E-A' (in which E is a single bond, an oxygen atom, a sulfur atom, -SO-, -SO₂-, -NR⁹- or -CO-, A' is a phenyl group substituted by a hydroxy- or amino-substituted alkyl group, a halogen-substituted alkoxy group, an alkoxycarbonyl group, a carboxyl group, an amino group (said amino group may optionally be substituted by one or two groups selected from an alkyl group, an alkanoyl group and an alkoxycarbonyl group), a carbamoyl group, an aryl group, an aryloxy group, an alkylsulfonyl group or an arylsulfonyl group; an optionally substituted naphthyl group; or an optionally substituted heteroaryl group, R⁹ is a hydrogen atom or an optionally substituted alkyl group), or a prodrug thereof, or a pharmaceutically acceptable salt thereof.

15. The compound according to claim 2 or 3, wherein at least one of R^{1'} and R^{2'} is an aryl group (said aryl group may optionally be substituted by a halogen atom, a hydroxy group, an alkoxy group, or an alkanoyl group), X is a sulfur atom, and R⁵, R⁶, R⁷ and R⁸ are independently a substituted alkyl group (the substituent thereof is selected from a hydroxy group, a nitro group, a cyano group, an alkoxy group, a cycloalkyl group, an amino group, an alkylamino group, a dialkylamino group, an alkanoylamino group, an alkoxycarbonylamino group, an alkylsulfonyl group, an arylsulfonyl group, an optionally substituted aryl group and an optionally substituted heteroaryl group); an optionally substituted cycloalkyl group; an optionally substituted alkenyl group; an optionally substituted alkynyl group; a halogen atom; a cyano group; a nitro group; a hydroxy group; an optionally substituted amino group; a substituted alkoxy group; an optionally substituted alkanoyl group; an optionally substituted alkoxycarbonyl group; an optionally substituted aryloxycarbonyl group; an optionally substituted heteroaryloxycarbonyl group; a carboxyl group; an optionally substituted carbamoyl group; an optionally substituted sulfamoyl group; an optionally substituted ureido group; an optionally substituted alkylthio group; an optionally substituted alkylsulfinyl group; an optionally substituted alkylsulfonyl group; or a group of the formula: -E-A (in which E is a single bond, an oxygen atom, a sulfur atom, -SO-, -SO₂-, -NR⁹- or -CO-, A is an optionally substituted aryl group or an optionally substituted heteroaryl group, R⁹ is a hydrogen atom or an optionally substituted alkyl group), or a prodrug thereof, or a pharmaceutically acceptable salt thereof.

16. The compound according to claim 2 or 3, wherein at least one of R^{1'} and R^{2'} is an aryl group (said aryl group may optionally be substituted by a halogen atom, a hydroxy group, an alkoxy group, or an alkanoyl group), and X is an oxygen atom, NR¹⁰, or CR¹¹R¹², or a prodrug thereof, or a pharmaceutically acceptable salt thereof.

17. A compound of the formula (5): 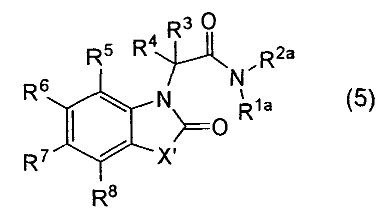 wherein R^{1a} is an optionally substituted alkyl group or an optionally substituted cycloalkyl group, R^{2a} is an optionally substituted aryl group or an optionally substituted heteroaryl group, or R^{1a} and R^{2a} combine together with the nitrogen atom to which they bond and form a group of the formula (4"): 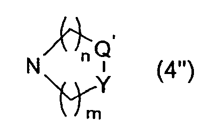 (in which n, m and Y are as defined in claim 2, and Q' is an optionally substituted o-phenylene group),
R³ and R⁴ are independently a hydrogen atom, a halogen atom, an optionally substituted alkyl group, an optionally substituted cycloalkyl group, an optionally substituted alkenyl group, or an optionally substituted alkynyl group,
R⁵, R⁶, R⁷ and R⁸ are independently a hydrogen atom, an optionally substituted alkyl group, an optionally substituted cycloalkyl group, an optionally substituted alkenyl group, an optionally substituted alkynyl group, a halogen atom, a cyano group, a nitro group, a hydroxy group, an optionally substituted amino group, an optionally substituted alkoxy group, an optionally substituted alkanoyl group, an optionally substituted alkoxycarbonyl group, an optionally substituted aryloxycarbonyl group, an optionally substituted heteroaryloxycarbonyl group, a carboxyl group, an optionally substituted carbamoyl group, an optionally substituted sulfamoyl group, an optionally substituted ureido group, an optionally substituted alkylthio group, an optionally substituted alkylsulfinyl group, an optionally substituted alkylsulfonyl group, or a group of the formula: -E-A (in which E is a single bond, an oxygen atom, a sulfur atom, -SO-, -SO₂-, -NR⁹- or -CO-, A is an optionally substituted aryl group or an optionally substituted heteroaryl group, and R⁹ is a hydrogen atom or an optionally substituted alkyl group), provided that R⁵, R⁶, R⁷ and R⁸ are not simultaneously a hydrogen atom,
X' is an oxygen atom, a sulfur atom, NR¹⁰, or CR^{11a}R^{12a} (in which R¹⁰ is as defined in claim 2, R^{11a} and R^{12a} are independently a hydrogen atom, an alkyl group optionally substituted by a halogen atom, an optionally substituted cycloalkyl group, an optionally substituted alkenyl group, or an optionally substituted alkynyl group, or R^{11a} and R^{12a} combine and form an oxo group or =NOH),
provided that
(1) when X is a sulfur atom or NR¹⁰, and one or two groups of R⁵, R⁶, R⁷ and R⁸ are independently a halogen atom, an alkyl group, a trihalomethyl group, or an optionally substituted alkoxy group, then the remaining groups are not a hydrogen atom,
(2) when X is an oxygen atom, then R⁷ is not a halogen atom,
or a prodrug thereof, or a pharmaceutically acceptable salt thereof.

18. The compound according to claim 17, wherein R^{1a} is an optionally substituted alkyl group or an optionally substituted cycloalkyl group, R^{2a} is an optionally substituted aryl group or an optionally substituted heteroaryl group, and at least one of R⁵, R⁶, R⁷ and R⁸ is a group of the formula: -E-A (in which E and A are as defined in claim 2), or a prodrug thereof, or a pharmaceutically acceptable salt thereof.

19. The compound according to claim 18, wherein E is a single bond, or a prodrug thereof, or a pharmaceutically acceptable salt thereof.

20. The compound according to claim 17, wherein R^{1a} is an optionally substituted alkyl group, R^{2a} is an optionally substituted aryl group or an optionally substituted heteroaryl group, and R⁶ and/or R⁸ are a halogen atom, or a prodrug thereof, or a pharmaceutically acceptable salt thereof.

21. A compound of the formula (6): 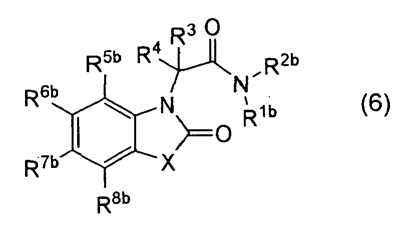 wherein R^{1b} and R^{2b} are independently a substituted alkyl group, an optionally substituted alkenyl group, or an optionally substituted alkynyl group,
R³ and R⁴ are as defined in claim 2,
R^{5b}, R^{6b}, R^{7b} and R^{8b} are independently a hydrogen atom, an optionally substituted alkyl group, an optionally substituted cycloalkyl group, an optionally substituted alkenyl group, an optionally substituted alkynyl group, a halogen atom, a cyano group, a nitro group, a hydroxy group, an optionally substituted amino group, an optionally substituted alkoxy group, an optionally substituted alkanoyl group, an optionally substituted alkoxycarbonyl group, an optionally substituted aryloxycarbonyl group, an optionally substituted heteroaryloxycarbonyl group, a carboxyl group, an optionally substituted carbamoyl group, an optionally substituted sulfamoyl group, an optionally substituted ureido group, an optionally substituted alkylthio group, an optionally substituted alkylsulfinyl group, an optionally substituted alkylsulfonyl group, or a group of the formula: -E-A^{b} (in which E is as defined in claim 2, A^{b} is a substituted phenyl group (the substituent thereof is selected from a halogen atom, an alkyl group substituted by a hydroxy group or an optionally substituted amino group, a halogen-substituted alkoxy group, an alkoxycarbonyl group, a carboxyl group, an amino group (said amino group being optionally substituted by one or two groups selected from an alkyl group, an alkanoyl group, an alkoxycarbonyl group, etc.), a carbamoyl group, an aryl group, an aryloxy group, an alkylsulfonyl group and an arylsulfonyl group); an optionally substituted naphthyl group; or an optionally substituted heteroaryl group), and at least one of R^{5b}, R^{6b}, R^{7b} and R^{8b} is a group of the formula: -E-A^{b},
X is an oxygen atom, a sulfur atom, NR¹⁰, or CR^{11b}R^{12b} (in which R¹⁰ is as defined in claim 2, R^{11b} and R^{12b} are independently a hydrogen atom, an alkyl group optionally substituted by a halogen atom, an optionally substituted cycloalkyl group, an optionally substituted alkenyl group, or an optionally substituted alkynyl group, or R^{11b} and R^{12b} combine to form an oxo group or =NOH), or a prodrug thereof, or a pharmaceutically acceptable salt thereof.

22. A drug comprising as the active ingredient the compound as set forth in any one of claims 2 to 21, or a prodrug thereof, or a pharmaceutically acceptable salt thereof.

23. An antianxiety or antidepressant agent comprising as the active ingredient the compound as set forth in any one of claims 2 to 21, or a prodrug thereof, or a pharmaceutically acceptable salt thereof.
